# EUROPEAN PATENT SPECIFICATION

(11) **EP 3 843 775 B1**
(45) Date of publication and mention of the grant of the patent: **03.04.2024**
(21) Application number: 19855681.3
(22) Date of filing: 29.08.2019
(51) Int. Cl.: A61K 38/19, A61K 35/66, A61K 38/20, A61P 29/00, A61P 37/06, C07K 14/52, C07K 14/525

(54) **BAFF POLYPEPTIDE FOR USE IN TREATING MULTIPLE SCLEROSIS**
BAFF-POLYPEPTID ZUR VERWENDUNG BEI DER BEHANDLUNG VON MULTIPLER SKLEROSE
POLYPEPTIDE BAFF POUR LE TRAITEMENT DE LA SCLÉROSE EN PLAQUES

(30) Priority: 30.08.2018 US 201862724714 P; 21.11.2018 US 201862770408 P
(43) Date of publication of application: 07.07.2021
(73) Proprietor: The Governing Council of the University of Toronto, Toronto, Ontario M5G 1L5 (CA)
(72) Inventor: GOMMERMAN, Jennifer, Toronto, Ontario M5R 3C5 (CA); ROJAS, Olga, Toronto, Ontario M6L 3G4 (CA)
(74) Representative: Appleyard Lees IP LLP
(86) International application number: PCT/CA2019/051200
(87) International publication number: WO 2020/041885

(56) References cited:
- EP-B1- 1 853 293
- WO-A1-2013/103966
- US-A1- 2017 081 387
- US-B2- 8 852 591
- DANIELLE T. AVERY ET AL: "BAFF selectively enhances the survival of plasmablasts generated from human memory B cells", JOURNAL OF CLINICAL INVESTIGATION, vol. 112, no. 2, 15 July 2003 (2003-07-15) , pages 286-297, XP055075578, ISSN: 0021-9738, DOI: 10.1172/JCI200318025
- KEPPLER SELINA J. ET AL: "The Wanderings of Gut-Derived IgA Plasma Cells: Impact on Systemic Immune Responses", FRONTIERS IN IMMUNOLOGY, vol. 12, 15 April 2021 (2021-04-15), XP055914452, DOI: 10.3389/fimmu.2021.670290
- Miyazaki Yusei; Niino Masaaki; Takahashi Eri; Suzuki Masako; Mizuno Masanori; Hisahara Shin; Fukazawa Toshiyuki; Amino Itaru; Naka: "Fingolimod induces BAFF and expands circulating transitional B cells without activating memory B cells and plasma cells in multiple sclerosis", Clinical Immunology, vol. 187, 25 October 2017 (2017-10-25), pages 95-101, XP085352013, ISSN: 1521-7035, DOI: 10.1016/j.clim.2017.10.009
- Jennifer L Gommerman , Olga L Rojas , Jorg H Fritz: "Re-thinking the functions of IgA + plasma cells", Gut Microbes, vol. 5, no. 5, 3 September 2014 (2014-09-03), pages 652-662, XP055696687, ISSN: 1949-0984, DOI: 10.4161/19490976.2014.969977
- Paul A Moore , Ornella Belvedre , Amy Orr , Krystyna Pieri , David W LaFleur , Ping Feng , Daniel Soppet , Meghan Charters , Reine: "BLyS: Member of the Tumor Necrosis Factor Family and B Lymphocyte Stimulator", Science, vol. 285, 9 July 1999 (1999-07-09), pages 260-263, XP002142252, ISSN: 1095-9203, DOI: 10.1126/science.285.5425.260
- Denga Chu , Mingchao Shi , Yue Lang , Donghui Shem , Tao Jin , Jie Zhu , Li Cui: "Gut Microbiota in Multiple Sclerosis and Experimental Autoimmune Encephalomyelitis: Current Applications and Future Perspectives", Mediators ofInflammation, vol. 2018, 8168717, 2 April 2018 (2018-04-02), pages 1-17, XP055696690, ISSN: 1466-1861, DOI: 10.1155/2018/8168717
- Karin Kannel , Kristi Alnek , Liina Vahter , Katrin Gross-Paju , Raivo Uibo , Kalle V Kisand: "Changes in Blood B Cell -Activating Factor (BAFF) Levels in Multiple Sclerosis: A Sign of Treatment Outcome", PLoS One, vol. 10, no. 11, 23 November 2015 (2015-11-23), pages 1-16, XP055696694, ISSN: 1932-6203, DOI: 10.1371/journal.pone.0143393
- Angela Wang; Olga L Rojas; Jennifer Gommerman: "P261 Excess IgA-Producing Plasma Cells in BAFF-Transgenic Mice Regulate Experimental Autoimmune Encephalomyelitis (EAE) at the T Cell Effector Stage", Multiple Sclerosis Journal; ActriMS 2019 Forum, vol. 25, no. S1, 1 April 2019 (2019-04-01) , pages 135-136, XP009526122, Dallas, Texas ISSN: 1352-4585, DOI: 10.1177/1352458519843086
- Rojas Olga L; Pröbstel Anne-Katrin; Porfilio Elisa A; Wang Angela A; Charabati Marc; Sun Tian; Lee Dennis S W; Galicia Georgina; R: "Recirculating Intestinal IgA-Producing Cells Regulate Neuroinflammation via IL -10", Cell, vol. 176, no. 3, 24 January 2019 (2019-01-24), pages 610-624, XP055781967, ISSN: 1097-4172, DOI: 10.1016/j.cell.2018.11.035

## Description

### FIELD OF THE DISCLOSURE

The present disclosure relates generally to the field of immunology. More particularly, the present disclosure relates to methods of treating an autoimmune disease.

### BACKGROUND OF THE DISCLOSURE

After initial encounter with antigen, B cells can differentiate into plasmablasts (PB), plasma cells (PC) and memory B cells. PB, which are rapidly produced upon antigen encounter, are short-lived effector cells whereas PC are long-lived mediators of lasting humoral immunity (Koch et al., 1981; Nutt et al., 2015). In humans, long-lived PC that have downregulated B220 and CD19 lineage markers produce specific antibodies (Ab) for years after encountering their cognate antigens (Manz et al., 1998).

Studies in mice indicate that long-lived PC reside in the bone marrow (BM) in niches that are rich in survival factors such as Interleukin-6 (IL-6), B-cell Activating Factor (BAFF) and A Proliferation Inducing Ligand (APRIL) (Chu and Berek, 2013). Analogous cytokine-rich niches exist in the gut lamina propria supporting mucosal IgA⁺ PC (Chu et al., 2014).

PC have been associated with disease pathogenesis, including autoimmune diseases such as Multiple Sclerosis (MS) where they have been found both in the central nervous system (CNS) parenchyma, cerebral spinal fluid (Ritchie et al., 2004) and in the meninges (Serafini et al., 2004). Treatment of relapsing-remitting MS (RRMS) with anti-CD20 antibodies that deplete B cells is followed by rapid and durable suppression of relapses, and prevention of newly formed inflammatory lesions in the CNS (Hauser et al., 2008; Kappos et al., 2011).

However, this therapy does not target CD20^{neg} PC. Accordingly, oligoclonal immunoglobulin bands in the cerebrospinal fluid (CSF) of MS patients, a diagnostic hallmark of the disease, are unchanged following anti-CD20 treatment (Piccio et al., 2010). In contrast to anti-CD20 therapy, treatment with atacicept (TACI-Ig), an agent that neutralizes both APRIL and BAFF, not only reduces circulating B cells but also decreases serum Ab titres, particularly IgM and IgA (Tak et al., 2008). Atacicept was tested in RRMS with the sensible hypothesis that depletion of a broader array of B lineage cells would have an even more beneficial effect on the disease than anti-CD20 therapy. Surprisingly however, treatment of RRMS patients with atacicept resulted in dose-dependent disease exacerbations (Kappos et al., 2014), and also promoted the development of MS in optic neuritis patients (Sergott et al., 2015). EP1853293B1 discloses a method of treatment for treating, inhibiting or reducing tissue deterioration, injury or damage due to neuro-, musucular- or neuro-muscular- degenerative disease, or for resoring tissue adversely affected by said disease in a subject, including administering an effective amount of a composition including a peptide agent including amino aid sequence LKKTET or LKKTNT, a conservative variant thereof, or a peptide agent that stimulates production of an LKKTET or LKKTNT peptide, or a conservative variant thereof, in a tissue.

There is a need to better understand how B cells regulate autoimmune diseases. There is also a need for improved methods of treating autoimmune diseases, such as MS.

### SUMMARY OF THE DISCLOSURE

The present invention is defined in the appended set of claims.

The inventors have invented methods of treating an autoimmune disease in a subject.

Disclosed herein is a method of treating an autoimmune disease in a subject is provided. The method comprises administering an effective amount of one or more of:
- a B-cell Activating Factor (BAFF) polypeptide;
- a BAFF polypeptide and an agent that promotes survival and/or migration of gut-derived commensal-reactive B cells to the central nervous system of the subject;
- a BAFF polypeptide and an agent that depletes B cells; or
- a BAFF polypeptide and a gut commensal that increases IgA levels
to the subject.

In a disclosure of the method of treating an autoimmune disease in a subject provided herein, the autoimmune disease is a non-systemic organ-specific autoimmune disease.

In a disclosure of the method of treating an autoimmune disease in a subject provided herein, the autoimmune disease is multiple sclerosis.

In a disclosure of the method of treating an autoimmune disease in a subject provided herein, the BAFF polypeptide is fused to the human Fc region of an immunoglobulin polypeptide.

In a disclosure of the method of treating an autoimmune disease in a subject provided herein, the commensal-reactive B cells are IgA+ plasmablasts and/or plasma cells.

In a disclosure of the method of treating an autoimmune disease in a subject provided herein, the commensal-reactive B cells express interleukin-10 (IL-10) and/or inducible nitric oxide synthase (iNOS).

In a disclosure of the method of treating an autoimmune disease in a subject provided herein, the agent that promotes survival and/or migration of gut-derived commensal-reactive B cells is a cytokine or a chemokine.

In a disclosure of the method of treating an autoimmune disease in a subject provided herein, the agent that promotes survival and/or migration of gut-derived commensal-reactive B cells is IL-10 and/or iNOS.

In a disclosure of the method of treating an autoimmune disease in a subject provided herein, the agent that depletes B cells comprises an antibody.

In a disclosure of the method of treating an autoimmune disease in a subject provided herein, the agent that depletes B cells comprises an antibody that binds to CD19 and/or CD20.

In a disclosure of the method of treating an autoimmune disease in a subject provided herein, the gut commensal is a commensal microbe.

In a disclosure of the method of treating an autoimmune disease in a subject provided herein, the administering an effective amount of a gut commensal comprises oral or rectal administration of a microbe or community of microbes.

In a disclosure of the method of treating an autoimmune disease in a subject provided herein, the gut commensal comprises a microbe/community of microbes that supports the generation of immunoregulatory IgA plasma cells.

In a disclosure of the method of treating an autoimmune disease in a subject provided herein, the gut commensal comprises *Tritrichomonas musculis* or a gut microbial community that has been modified by carriage of *Tritrichomonas musculis.*

Further disclosed herein is a method of reducing inflammation in a subject is provided. The method comprises administering an effective amount of one or more of:
- a B-cell Activating Factor (BAFF) polypeptide;
- a BAFF polypeptide and an agent that promotes survival and/or migration of gut-derived commensal-reactive B cells to the central nervous system of the subject;
- a BAFF polypeptide and an agent that depletes B cells; or
- a BAFF polypeptide and a gut commensal that increases IgA levels
to the subject.

In a disclosure of the method of reducing inflammation in a subject provided herein, the inflammation is reduced in the periphery of the subject.

In a disclosure of the method of reducing inflammation in a subject provided herein, the inflammation is reduced in the central nervous system.

In a disclosure of the method of reducing inflammation in a subject provided herein, the inflammation is neuroinflammation.

In a disclosure of the method of reducing inflammation in a subject provided herein, the neuroinflammation is caused by multiple sclerosis.

In a disclosure of the method of reducing inflammation in a subject provided herein, the BAFF polypeptide is fused to the human Fc region of an immunoglobulin polypeptide.

In a disclosure of the method of reducing inflammation in a subject provided herein, the commensal-reactive B cells are IgA+ plasmablasts and/or plasma cells.

In a disclosure of the method of reducing inflammation in a subject provided herein, the commensal-reactive B cells express IL-10 and/or iNOS.

In a disclosure of the method of reducing inflammation in a subject provided herein, the agent that promotes survival and/or migration of gut-derived commensal-reactive B cells is a cytokine or a chemokine.

In a disclosure of the method of reducing inflammation in a subject provided herein, the agent that promotes survival and/or migration of gut-derived commensal-reactive B cells is IL-10 and/or iNOS.

In a disclosure of the method of reducing inflammation in a subject provided herein, the agent that depletes B cells comprises an antibody.

In a disclosure of the method of reducing inflammation in a subject provided herein, the agent that depletes B cells comprises an antibody that binds to CD19 and/or CD20.

In a disclosure of the method of reducing inflammation in a subject provided herein, the gut commensal is a commensal microbe.

In a disclosure of the method of reducing inflammation in a subject provided herein, the administering an effective amount of a gut commensal comprises oral or rectal administration of a microbe or community of microbes.

In a disclosure of the method of reducing inflammation in a subject provided herein, the gut commensal comprises a microbe/community of microbes that supports the generation of immunoregulatory IgA plasma cells.

In a disclosure of the method of reducing inflammation in a subject provided herein, the gut commensal comprises *Tritrichomonas musculis* or a gut microbial community that has been modified by carriage of *Tritrichomonas musculis.*

Further disclosed herein is a method of enriching gut-derived commensal-reactive IgA+ plasmablasts and/or plasma cells in the central nervous system of a subject is provided. The method comprises administering an effective amount of one or more of:
- a B-cell Activating Factor (BAFF) polypeptide;
- a BAFF polypeptide and an agent that promotes survival and/or migration of gut-derived commensal-reactive B cells to the central nervous system of the subject;
- a BAFF polypeptide and an agent that depletes B cells; or
- a BAFF polypeptide and a gut commensal that increases IgA levels
to the subject.

In a disclosure of the method of enriching gut-derived commensal-reactive IgA+ plasmablasts and/or plasma cells in the central nervous system of a subject provided herein, the gut-derived commensal-reactive IgA+ plasmablasts and/or plasma cells express IL-10 and/or iNOS.

In a disclosure of the method of enriching gut-derived commensal-reactive IgA+ plasmablasts and/or plasma cells in the central nervous system of a subject provided herein, the subject has an autoimmune disease.

In a disclosure of the method of enriching gut-derived commensal-reactive IgA+ plasmablasts and/or plasma cells in the central nervous system of a subject provided herein, the subject has a non-systemic organ-specific autoimmune disease.

In a disclosure of the method of enriching gut-derived commensal-reactive IgA+ plasmablasts and/or plasma cells in the central nervous system of a subject provided herein, the subject has multiple sclerosis.

In a disclosure of the method of enriching gut-derived commensal-reactive IgA+ plasmablasts and/or plasma cells in the central nervous system of a subject provided herein, the BAFF polypeptide is fused to the human Fc region of an immunoglobulin polypeptide.

In a disclosure of the method of enriching gut-derived commensal-reactive IgA+ plasmablasts and/or plasma cells in the central nervous system of a subject provided herein, the agent that promotes survival and/or migration of gut-derived commensal-reactive B cells is a cytokine or a chemokine.

In a disclosure of the method of enriching gut-derived commensal-reactive IgA+ plasmablasts and/or plasma cells in the central nervous system of a subject provided herein, the agent that promotes survival and/or migration of gut-derived commensal-reactive B cells is IL-10 and/or iNOS.

In a disclosure of the method of enriching gut-derived commensal-reactive IgA+ plasmablasts and/or plasma cells in the central nervous system of a subject provided herein, the agent that depletes B cells comprises an antibody.

In a disclosure of the method of enriching gut-derived commensal-reactive IgA+ plasmablasts and/or plasma cells in the central nervous system of a subject provided herein, the agent that depletes B cells comprises an antibody that binds to CD19 and/or CD20.

In a disclosure of the method of enriching gut-derived commensal-reactive IgA+ plasmablasts and/or plasma cells in the central nervous system of a subject provided herein, the gut commensal is a commensal microbe.

In a disclosure of the method of enriching gut-derived commensal-reactive IgA+ plasmablasts and/or plasma cells in the central nervous system of a subject provided herein, the administering an effective amount of a gut commensal comprises oral or rectal administration of a microbe or community of microbes.

In a disclosure of the method of enriching gut-derived commensal-reactive IgA+ plasmablasts and/or plasma cells in the central nervous system of a subject provided herein, the gut commensal comprises a microbe/community of microbes that supports the generation of immunoregulatory IgA plasma cells.

In a disclosure of the method of enriching gut-derived commensal-reactive IgA+ plasmablasts and/or plasma cells in the central nervous system of a subject provided herein, the gut commensal comprises *Tritrichomonas musculis* or a gut microbial community that has been modified by carriage of *Tritrichomonas musculis.*

Further disclosed herein is a method of promoting survival of gut-derived commensal-reactive IgA+ plasmablasts and/or plasma cells in a subject to reduce inflammation in a tissue is provided. The method comprises administering an effective amount of one or more of:
- a B-cell Activating Factor (BAFF) polypeptide;
- a BAFF polypeptide and an agent that promotes survival and/or migration of gut-derived commensal-reactive B cells to the central nervous system of the subject;
- a BAFF polypeptide and an agent that depletes B cells; or
- a BAFF polypeptide and a gut commensal that increases IgA levels
to the subject.

In a disclosure of the method of promoting survival of gut-derived commensal-reactive IgA+ plasmablasts and/or plasma cells in a subject to reduce inflammation in a tissue provided herein, the gut-derived commensal-reactive IgA+ plasmablasts and/or plasma cells express IL-10 and/or iNOS.

In a disclosure of the method of promoting survival of gut-derived commensal-reactive IgA+ plasmablasts and/or plasma cells in a subject to reduce inflammation in a tissue provided herein, the subject has an autoimmune disease.

In a disclosure of the method of promoting survival of gut-derived commensal-reactive IgA+ plasmablasts and/or plasma cells in a subject to reduce inflammation in a tissue provided herein, the subject has a non-systemic organ-specific autoimmune disease.

In a disclosure of the method of promoting survival of gut-derived commensal-reactive IgA+ plasmablasts and/or plasma cells in a subject to reduce inflammation in a tissue provided herein, the subject has multiple sclerosis.

In a disclosure of the method of promoting survival of gut-derived commensal-reactive IgA+ plasmablasts and/or plasma cells in a subject to reduce inflammation in a tissue provided herein, the BAFF polypeptide is fused to the human Fc region of an immunoglobulin polypeptide.

In a disclosure of the method of promoting survival of gut-derived commensal-reactive IgA+ plasmablasts and/or plasma cells in a subject to reduce inflammation in a tissue provided herein, the agent that promotes survival and/or migration of gut-derived commensal-reactive B cells is a cytokine or a chemokine.

In a disclosure of the method of promoting survival of gut-derived commensal-reactive IgA+ plasmablasts and/or plasma cells in a subject to reduce inflammation in a tissue provided herein, the agent that promotes survival and/or migration of gut-derived commensal-reactive B cells is IL-10 and/or iNOS.

In a disclosure of the method of promoting survival of gut-derived commensal-reactive IgA+ plasmablasts and/or plasma cells in a subject to reduce inflammation in a tissue provided herein, the agent that depletes B cells comprises an antibody.

In a disclosure of the method of promoting survival of gut-derived commensal-reactive IgA+ plasmablasts and/or plasma cells in a subject to reduce inflammation in a tissue provided herein, the agent that depletes B cells comprises an antibody that binds to CD19 and/or CD20.

In a disclosure of the method of promoting survival of gut-derived commensal-reactive IgA+ plasmablasts and/or plasma cells in a subject to reduce inflammation in a tissue provided herein, the gut commensal is a commensal microbe.

In a disclosure of the method of promoting survival of gut-derived commensal-reactive IgA+ plasmablasts and/or plasma cells in a subject to reduce inflammation in a tissue provided herein, the administering an effective amount of a gut commensal comprises oral or rectal administration of a microbe or community of microbes.

In a disclosure of the method of promoting survival of gut-derived commensal-reactive IgA+ plasmablasts and/or plasma cells in a subject to reduce inflammation in a tissue provided herein, the gut commensal comprises a microbe/community of microbes that supports the generation of immunoregulatory IgA plasma cells.

In a disclosure of the method of promoting survival of gut-derived commensal-reactive IgA+ plasmablasts and/or plasma cells in a subject to reduce inflammation in a tissue provided herein, the gut commensal comprises *Tritrichomonas musculis* or a gut microbial community that has been modified by carriage of *Tritrichomonas musculis.*

Other features and advantages are described in the following detailed description.

### DESCRIPTION OF THE DRAWINGS

The patent or application file contains at least one drawing.

The disclosures are illustrated by way of examples in the accompanying drawings, wherein:
**Fig. 1a** shows histogram plots of the expression of B220, Ki67 and CD138 on *Prdm1-*YFP⁺ cells in the brain, spinal cord, bone marrow and draining lymph nodes (Br, Sc, BM and LN, respectively) during the chronic phase of Experimental Autoimmune Encephalomyelitis (EAE) of *Prdm1-YFP* mice; grey filled histograms represent the Fluorescence Minus One (FMO) control for each stain and empty histograms represent specific antibody (Ab) staining as determined by flow cytometry. The experiment was repeated 3 times with at least 5 mice per group.
**Fig. 1b** is a graph showing the absolute numbers of *Prdm1*-YFP⁺B220^{-/dim} cells in the Br and Sc at different time points of EAE determined by flow cytometry. The experiment was repeated 3 times with at least 5 mice per group; **p<0.01, (Mann Whitney Test) (Mean and SD).
**Fig. 1c** shows representative contour plots of intracellular IgA and IgG expression by *Prdm1-*YFP⁺B220^{-/dim} cells determined by flow cytometry. Gating of *Prdm1*-YFP⁺B220^{-/dim} cells is shown in the top panel, and gating of IgA and IgG expressing cells is shown in the bottom panel. The experiment was repeated 3 times with at least 5 mice per group.
**Fig. 1d** is a graph showing the frequency of *Prdm1*-YFP⁺ cells that express IgA or IgG in the Br and Sc, as determined by flow cytometry. The experiment was repeated 3 times with at least 5 mice per group; NS (Not Significant), (Mann Whitney Test) (Mean and SD).
**Fig. 1e** shows graphs of the number of IgA or IgG antibody secreting cells (ASC) in the Sc (left panel) and Br (right panel) determined by two-color ELISPOT of unimmunized (Ul) or EAE WT mice (chronic phase). The experiment was repeated 3 times with at least 5 mice per group; *p<0.05, NS (Not Significant), (Mann Whitney Test) (Mean and SD).
**Fig. 1f** is a graph showing the number of IgA or IgG ASC in the Br determined by two-color ELISPOT of UI mice or IgA^{-/-} chimeric EAE mice. The experiment was repeated twice with at least 6 mice per group; **p<0.01, (Mann Whitney Test) (Mean and SD).
**Fig. 2a** shows representative immunofluorescence images of Aicd-YFP small intestines stained with DAPI, anti-CD8α and anti-CD138 and visualized for YFP at the pre-onset (Ul) or chronic phase of EAE. The experiment was repeated 3 times with 3-8 mice per group.
**Fig. 2b** is a graph showing the number of CD138⁺ cells in the small intestines of UI and EAE mice, normalized to the number of CD8⁺ cells. The experiment was repeated 3 times with 3-8 mice per group. *p<0.05 (Two-way ANOVA Test or Mann Whitney Test, Mean and SD).
**Fig. 2c** is a graph showing the number of YFP⁺ cells in the small intestines of UI and EAE mice, normalized to the number of CD8⁺ cells. The experiment was repeated 3 times with 3-8 mice per group. **p<0.01 (Two-way ANOVA Test or Mann Whitney Test, Mean and SD).
**Fig. 2d** shows graphs depicting EAE clinical scores over time (left panel) and cumulative score (right panel) of PB/PC adoptive transfer in CD19^{cre}Prdm1^{fl/fl} recipients, with adoptive transfer time-points indicated by arrows. The experiment was repeated 5 times with 3-4 mice per group. **p<0.01 (Two-way ANOVA Test or Mann Whitney Test, Mean and SD).
**Fig. 2e** shows graphs depicting EAE clinical scores over time (left panel) and cumulative score (right panel) of PB/PC adoptive transfer in *Jht*^{*-*/*-*} recipients, with adoptive transfer time-points indicated by arrows. The experiment was repeated 5 times with 3-4 mice per group. **p<0.01 (Two-way ANOVA Test or Mann Whitney Test, Mean and SD).
**Fig. 2f** shows graphs depicting the flow cytometry gating strategy for detection of adoptively transferred *Prdm1*-YFP⁺ cells in the Br (left column), BM (middle column) and LN (right column) of PB/PC-less recipient mice.
**Fig. 3a** is a graph showing the frequency of rotavirus (RV)-specific IgA and IgG ASC in the small intestinal lamina propria (SILP) of uninfected (Ul) mice or mice infected with RV and/or Flu. The experiment was repeated 5 times, and the graph contains all pooled data (n = 7-15 mice per group). ND (Not Detectable) (Mann Whitney Test, Mean and SD).
**Fig. 3b** is a graph showing the frequency of RV-specific IgA and IgG ASC in the BM of uninfected (Ul) mice or mice infected with RV and/or Flu. The experiment was repeated 5 times, and the graph contains all pooled data (n = 7-15 mice per group). *p<0.05, **p<0.01 (Mann Whitney Test, Mean and SD)
**Fig. 3c** is a graph showing the frequency of RV-specific IgA and IgG ASC in the lungs of uninfected (Ul) mice or mice infected with RV and/or Flu. The experiment was repeated 5 times, and the graph contains all pooled data (n = 7-15 mice per group). *p<0.05, ND (Not Detectable) (Mann Whitney Test, Mean and SD).
**Fig. 3d** is a schematic representation of parabiosis experimental design. Parabiosis experiments were conducted over 60 days with two groups of mice: RV-only ("A") and UI ("B"), wherein A and B mice were subsequently paired together. The experiments were repeated 2 times.
**Fig. 3e** is a graph showing the amount of fecal RV antigen assessed using enzyme-linked immunosorbant assay (ELISA) in fecal samples from uninfected (Ul) mice and in mice 6 days (D6) and 12 days (D12) post-infection. Mice infected with RV cleared the virus by D12 and were subsequently paired with their parabiont partner on D15 post-infection. The graph contains all pooled data (n = 4 pairs of mice per group). ***p<0.001 (Mann Whitney Test, Mean and SD).
**Fig. 3f** shows sample images of ELISPOTs from each collected tissue obtained from the parabiosis experiments, wherein except for the first vertical panel, Parabiont 1 was RV infected and Parabiont 2 was Ul. Both pairs in the first vertical panel were uninfected.
**Fig. 3g** is a graph of total RV-specific IgA ASC/1×10⁶ cells in the SILP of parabionts. The graph contains all pooled data (n = 4 pairs of mice per group). *p<0.05 (Mann Whitney Test, Mean and SD).
**Fig. 3h** is a graph of total RV-specific IgA ASC/1×10⁶ cells in the BM of parabionts. The graph contains all pooled data (n = 4 pairs of mice per group). *p<0.05 (Mann Whitney Test, Mean and SD).
**Fig. 4a** is a schematic representation of the RV-EAE experiments using MOG₃₅₋₅₅-induced EAE. The experiment was repeated 3 times.
**Fig. 4b** is a graph showing the frequency of RV-specific IgA ASC in the Br of unimmunized (UI) mice or mice infected with RV and/or immunized with MOG₃₅₋₅₅. The number of mice depicted is Ul (n=7), RV (n=6), EAE (n=6), RV EAE Peak (n=4), RV EAE Chronic (n=5). **p<0.01, ***p<0.001, ****p<0.0001 (Mann Whitney Test, Mean and SD).
**Fig. 4c** is a graph showing the frequency of RV-specific IgA ASC in the Sc of unimmunized (UI) mice or mice infected with RV and/or immunized with MOG₃₅₋₅₅. The number of mice depicted is Ul (n=7), RV (n=6), EAE (n=6), RV EAE Peak (n=4), RV EAE Chronic (n=5). *p<0.05 (Mann Whitney Test, Mean and SD).
**Fig. 4d** is a graph showing the frequency of RV-specific IgA ASC in the SILP of unimmunized (Ul) mice or mice infected with RV and/or immunized with MOG₃₅₋₅₅. The number of mice depicted is Ul (n=7), RV (n=6), EAE (n=6), RV EAE Peak (n=4), RV EAE Chronic (n=5). **p<0.01, ***p<0.001 (Mann Whitney Test, Mean and SD).
**Fig. 4e** is a graph showing the frequency of RV-specific IgA ASC in the BM of unimmunized (Ul) mice or mice infected with RV and/or immunized with MOG₃₅₋₅₅. The number of mice depicted is Ul (n=7), RV (n=6), EAE (n=6), RV EAE Peak (n=4), RV EAE Chronic (n=5). *p<0.05, **p<0.01 (Mann Whitney Test, Mean and SD).
**Fig. 4f** shows representative images of RV-specific IgA ASC ELISPOT from Br, Sc, SILP and BM of RV-EAE mice at the chronic stage of the disease.
**Fig. 4g** shows representative images of commensal-reactive IgA ASC derived from the SILP of naive WT, Germ Free and *Cd19^{cre}Prdm1*^{fl/fl} mice. PBS coated wells were used as a negative control.
**Fig. 4h** is a graph showing the frequency of commensal-reactive IgA ASC derived from the SILP of naive WT, Germ Free and *Cd19*^{cre}*Prdm1*^{fl/fl} mice. The experiment was repeated 3 times with 4-7 mice for each group. *p<0.05 (Mann Whitney Test, Mean and SD).
**Fig. 4i** is a graph showing the frequency of commensal-reactive IgA ASC derived from sorted *Prdm1*-YFP⁺ B220 or *Prdm1*-YFP⁻ B220⁺ cells from the SILP of *Prdm1*^{YFP} mice. The experiment was repeated 3 times with 4-7 mice for each group.
**Fig. 4j** shows representative images of commensal-reactive IgA ASC derived from sorted *Prdm1*-YFP⁺ B220⁻ or *Prdm1*-YFP⁻ B220⁺ cells from the SILP of *Prdm1*^{YFP} mice.
**Fig. 4k** is a graph showing the frequency of commensal-reactive IgA ASC in the BM (top panel) and Brain (bottom panel) of UI and EAE (chronic) WT mice. The experiment was repeated 3 times with 4-7 mice for each group. **p<0.01 (Mann Whitney Test, Mean and SD).
**Fig. 4l** shows representative images of commensal-reactive IgA ASC in the BM (top row) and Brain (bottom row) of UI and EAE (chronic) WT mice.
**Fig. 5a** shows representative immunofluorescence images of small intestines from WT, *IL10*^{*-*/*-*} and *CD19^{cre}Prdm1*^{*fl*/*fl*} mice stained with DAPI, anti-Ig_{A} and anti-IL10. The experiment was repeated 2 times with at least 5 mice per group, with IL10^{-/-} staining control being performed once.
**Fig. 5b** shows graphs depicting clinical scores over time (left panel) and cumulative score (right panel) of mixed BM chimeras, wherein PB/PC-deficient mice reconstituted with an 80/20 mixture of PB/PC-deficient + IL10^{-/-} BM, and control chimeras reconstituted with an 80/20 mixture of WT + IL10^{-/-} BM were subjected to EAE. The experiment was performed 3 times. ***p<0.001, NS (Not Significant) (Two-way ANOVA Test).
**Fig. 5c** shows graphs of a quantitative analysis of Hematoxylin & Eosin (H&E, left panel) and Luxol Fast Blue (LFB, right panel) staining of thoracic spinal cord sections derived from mixed BM chimeras. The experiment was performed 3 times with 4-8 mice per group. *p<0.05, **p<0.01 (Mann Whitney Test).
**Fig. 5d** shows representative images of H&E stain of thoracic spinal cord sections derived from mixed BM chimeras acquired at 4X magnification using a light microscope to visualize inflammation and demyelination. The experiment was performed 3 times with 4-8 mice per group.
**Fig. 5e** shows representative images of LFB stain of thoracic spinal cord sections derived from mixed BM chimeras acquired at 4X magnification using a light microscope to visualize inflammation and demyelination. The experiment was performed 3 times with 4-8 mice per group.
**Fig. 5f** shows representative images of commensal-reactive IgA ASC derived from live, pre-sorted Dump- (CD4, CD8, F4/80 negative) and subsequently sorted as Thy1.1⁺ (top row) or Thy1.1⁻ (bottom row) cells from the brain (7500 cells/well) or BM (150 cells/well) during the chronic phase of EAE.
**Fig. 5g** shows graphs of the frequency of commensal-reactive IgA ASC derived from live, pre-sorted Dump- (CD4, CD8, F4/80 negative) and subsequently sorted as Thy1.1⁺ or Thy1.1⁻ cells from the brain (left panel) or BM (right panel) during the chronic phase of EAE. The experiment was performed 3 times with 5 mice per group (Mann Whitney Test).
**Fig. 6a** shows representative immunofluorescence images of the SILP of BAFF-Tg^{+/+} mice before (Ul) and during the chronic phase of MOG₃₅₋₅₅-induced EAE stained with DAPI, anti-CD138 and anti-lgA.
**Fig. 6b** is a graph showing the number of CD138⁺ cells in the SILP of BAFF-Tg^{+/+} mice before (Ul) and during the chronic phase of MOG₃₅₋₅₅-induced EAE, normalized to the number of CD8⁺ cells. The experiment was repeated 3 times with at least 4 mice per group. *p<0.05 (Mann Whitney Test).
**Fig. 6c** is a graph showing the frequency of IgA⁺ cells in the SILP of BAFF-Tg^{+/+} mice before (Ul) and during the chronic phase of MOG₃₅₋₅₅-induced EAE, normalized to the number of CD8⁺ cells. The experiment was repeated 3 times with at least 4 mice per group. *p<0.05 (Mann Whitney Test).
**Fig. 6d** is a graph showing the number of IgA or IgG ASC enumerated by ELISPOT analysis in the Br of BAFF^{+/+}-Tg mice during MOG₃₅₋₅₅-induced EAE (chronic phase) compared with Ul BAFF^{+/+}-Tg mice. The experiment was repeated 3 times with at least 4 mice per group. **p<0.01, NS (Not Significant) (Mann Whitney Test).
**Fig. 6e** is a graph showing the number of IgA or IgG ASC enumerated by ELISPOT analysis in the Sc of BAFF^{+/+}-Tg mice during MOG₃₅₋₅₅-induced EAE (chronic phase) compared with Ul BAFF^{+/+}-Tg mice. The experiment was repeated 3 times with at least 4 mice per group. NS (Not Significant) (Mann Whitney Test).
**Fig. 6f** is a graph showing clinical scores overtime in BAFF^{+/+}-Tg, BAFF^{+/-}-Tg and WT littermates after immunization with MOG₃₅₋₅₅. The experiment was repeated 3 times with at least 4 mice per group. ***p<0.001 (Two-way ANOVA Test).
**Fig. 6g** is a graph showing clinical scores over time in BAFF^{+/+} and WT mice after immunization with hrMOG. The experiment was repeated 2 times with at least 4-5 mice per group. ***p<0.001 (Two-way ANOVA Test).
**Fig. 6h** shows graphs of the frequency of IFNγ⁺ CD4⁺ T cells (left panel) or IL17⁺ CD4⁺ T cells (right panel) in the spleen (Sp), inguinal lymph nodes (iLN), or axillary lymph nodes (AxLN) from WT or BAFF^{+/+}-Tg littermates with chronic EAE. ***p<0.001, NS (Not Significant) (Mann Whitney Test).
**Fig. 6i** is a graph showing EAE clinical scores over time of adoptive transfer EAE whereby pre-primed T cells were transferred into WT or BAFF^{+/-}-Tg recipient mice. ***p<0.001 (Two-way ANOVA Test).
**Fig. 6j** shows representative immunofluorescence images of small intestines stained with DAPI, anti-lgA and anti-IL10 from BAFF^{+/+}-Tg and BAFF^{+/-}-Tg × IL10^{-/-} mice.
**Fig. 6k** is a graph showing clinical scores over time in BAFF^{+/-}-Tg, BAFF^{+/-}-Tg × IL10^{-/-} and WT littermates after immunization with MOG₃₅₋₅₅. ***p<0.001 (Two-way ANOVA Test).
**Fig. 6l** is a graph showing EAE clinical scores over time in Baff^{+/-}IL10^{-/-} recipients after transfer of *Prdm1^{YFP}*-IL10^{*-*/*-*} gut PC or *Prdm1^{YFP}* gut PC. ***p<0.001 (Two-way ANOVA Test).
**Fig. 7a** is a graph showing EAE clinical scores over time in *Prdm1^{cre}*-YFP^{fl/fl} mice. The experiment was repeated 3 times with at least 5 mice per group.
**Fig. 7b** is a graph showing EAE clinical scores over time in *Aicd^{cre}*-YFP^{fl/fl} mice. The experiment was repeated 3 times with at least 5 mice per group.
**Fig. 7c** shows graphs depicting the flow cytometry gating strategy used to analyze PB/PC. SSC-H (side scatter height), SSC-W (side scatter width), SSC-A (side scatter area), FSC-A (forward scatter area), LID (live/dead). The "DUMP" gate eliminates cells staining positive for CD4, CD8 and F4/80, PB/PC gate based on YFP expression and low/negative expression of B220, PB/PC gate to confirm PB/PC phenotype with IgA and Ki67.
**Fig. 7d** is a graph showing the median fluorescence intensity (MFI) of CD138 after gating on YFP⁺B220^{int/-} PB/PC cells in the BM (blue) and LN (green) of *Aicd^{cre}*-YFP^{fl/fl} mice at different time points during EAE (clinical score depicted in red). The experiment was repeated 3 times with at least 5 mice per group.
**Fig. 7e** is a graph showing the absolute numbers of YFP⁺B220^{int/-} PB/PC in the Br and Sc of *Aicd^{cre}*-YFP^{fl/fl} mice at different time points during EAE. The experiment was performed 5 times with 4-5 mice per group. **p<0.01 (Mann Whitney Test, Mean and SEM).
**Fig. 7f** is a graph showing the frequency of cells expressing IgA or IgG (intracellular), expressed as a percentage of total of YFP⁺ cells in the Br and Sc of *Aicd^{cre}*-YFP^{fl/fl} mice. The experiment was performed 5 times with 4-5 mice per group.
**Fig. 7g** is a graph showing the number of CD138⁺ (left panel) or IgA⁺ (right panel) PC in the SILP of *Cd19^{cre}Prdm1*^{fl/fl} mice and littermate controls, as determined by immunofluorescence microscopy. The number of CD8α⁺ cells was used as a normalizing denominator. The experiment was repeated 2 times with 5-8 mice per group. *p<0.05, **p<0.01, NS (Not Significant), (Mann Whitney Test, Mean and SEM).
**Fig. 7h** is a graph showing the levels of IgA (left panel) or IgG (right panel) in the serum of *Cd19^{cre}Prdm1*^{fl/fl} mice and littermate controls, as measured by ELISA. The experiment was repeated 2 times with 5-8 mice per group. ***p<0.001, NS (Not Significant), (Mann Whitney Test, Mean and SEM).
**Fig. 7i** shows graphs depicting EAE clinical scores over time (left panel) and cumulative score (right panel) of *Cd19*^{-cre} *Prdm1*^{*fl*/*fl*} and *Cd19*^{-cre}*Prdm1*^{fl/+} littermates. The experiment was performed with 5-6 mice per group. *p<0.05, ***p<0.001 (Two-way ANOVA Test and Mann Whitney Test, Mean and SEM).
**Fig. 7j** shows graphs depicting EAE clinical scores over time (left panel) and cumulative score (right panel) of *Aicd*^{cre}*Prdm1*^{fl/fl} and WT mice. The experiment was performed with 5-6 mice per group. **p<0.01, ***p<0.001 (Two-way ANOVA Test and Mann Whitney Test, Mean and SEM).
**Fig. 8** shows graphs depicting a flow cytometry gating strategy for sorting gut PC versus gut B cells, wherein post-sort analysis demonstrated 96-98% purity with the majority of transferred cells expressing intracellular IgA and staining negative for Ki67 and B220. FSC-H (forward scatter height), FSC-W (forward scatter width), FSC-A (forward scatter area), SSC-A (side scatter area). The "DUMP" gate eliminates cells staining positive for CD19, CD4, CD8 and F4/80.
**Fig. 9a** is a schematic representation of the dual-infection (RV and Flu) model used for tracking gut-derived PB/PC.
**Fig. 9b** is a graph showing a representative infection course, wherein RV antigen (Ag) and RV-specific IgA were measured by ELISA at the indicated time points.
**Fig. 9c** shows representative images of RV-specific IgA (top panel) and IgG (bottom panel) ASC detected after Flu infection by ELISPOT. Each spot is counted as one ASC.
**Fig. 9d** is a graph showing the percentage of weight remaining in mice from each of the groups depicted in Fig. 10a over a representative disease course during flu infection. Day 6 was considered to be the end point as few mice survived beyond day 6. All harvests were performed on or before this time point.

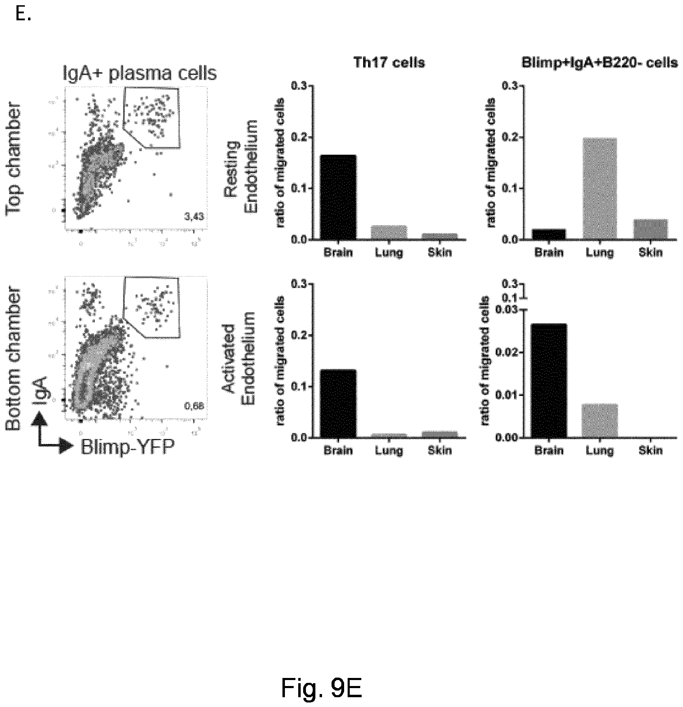

**Fig. 9e** shows graphs depicting an experiment assessing *in vitro* migration of SILP-derived Prdm1^{YFP}+IgA+ PB/PC through resting endothelial cell layers (top panel) or activated endothelial cell layers (bottom panel). Representative flow cytometry plots show the gating strategy and frequencies of IgA⁺ plasma cells from the non-migrated cells (top chamber) and after migration through resting lung endothelium layer (bottom chamber). IgA+ PB/PC were analyzed as CD4⁻CD8⁻F4/80⁻B220⁻IgA⁺Blimp YFP⁺ live singlets. The ratio of migrated cells was calculated as the number of cells in the bottom chamber / the number of cells in both the bottom + top chamber. The experiment was repeated 2 times with similar results. One representative experiment is shown.

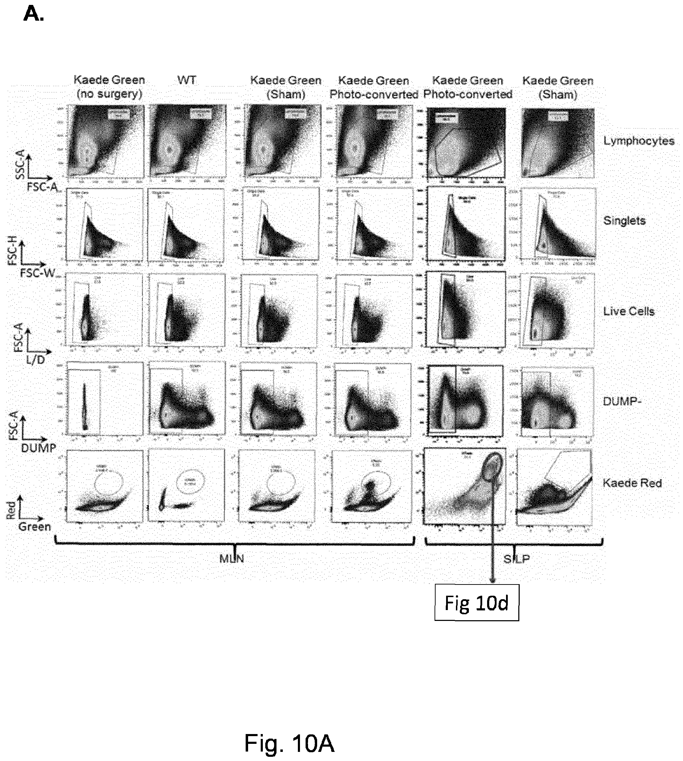

**Fig. 10a** shows graphs depicting the flow cytometry gating strategy for negative controls (Kaede-green (non-photoconverted), WT and Sham-surgery mice) and photoconverted Kaede-transgenic mice for quantification of Kaede-Red (K-Red) populations in various tissues (mesenteric lymph nodes (MLN) and SILP are shown), and also the analysis of IgA versus B220 derived from K-Red cells in the gut and BM. Gates were established for each individual tissue (due to tissue-specific autofluorescent properties) based on sham surgery controls for K-Red assessment. Gates were established for each individual tissue based on FMO controls for IgA/B220 assessment.
**Fig. 10b** is a graph showing the frequency of photoconverted (Kaede-Red+) cells in the MLN and SILP. The experiment was performed 3 times with at least 5 mice per group. **p<0.01 (Mean and SEM).
**Fig. 10c** is a flow cytometry plot (left panel) and a summary graph (right panel) showing the absolute numbers of photoconverted IgA+ PB/PC cells in the BM of WT, non-photoconverted KGreen and photoconverted Kaede mice. The experiment was performed 3 times with at least 5 mice per group. Depicted is a single experiment with 2 WT, 2 non-photoconverted KGreen and 6 photoconverted Kaede mice. **p<0.01 (Mean and SEM).
**Fig. 10d** is a flow cytometry plot (left panel) and a graph (right panel) showing the absolute numbers of photoconverted IgA+ PB/PC cells in the SILP of WT, non-photoconverted KGreen and photoconverted Kaede mice. The experiment was performed 3 times with at least 5 mice per group. Depicted is a single experiment with 2 WT, 2 non-photoconverted KGreen and 6 photoconverted Kaede mice. **p<0.01 (Mean and SEM).
**Fig. 10e** is a graph showing the amount of RV-Ag in stool samples collected at the indicated time points post-RV infection, as assessed by ELISA. The experiment was performed 3 times with at least 5 mice per group.
**Fig. 10f** is a graph showing representative clinical scores over time in mice with MOG₃₅₋₅₅-induced EAE. The experiment was performed 3 times with at least 5 mice per group. UI (uninfected).
**Fig. 11a** is a graph showing EAE clinical scores over time in WT mice colonized or not colonized with *Tritrichomonas musculis (T.mu).* Incidence of disease was 9/11 *for T.mu-* mice and 7/11 for *T.mu*+ mice. The experiment was performed once with 10 mice per group. All mice are depicted. *p<0.05, (Two-way ANOVA Test).
**Fig. 11b** is a graph showing EAE clinical scores over time in WT mice colonized or not colonized with *T.mu.* The experiment was performed once with 10 mice per group. Only mice that developed disease are depicted. In these mice, the average onset of disease was day 12.4 +/-1.0 for *T.mu-* mice and day 14 +/- 2.4 for *T. Mu*+ mice. *p<0.05, (Two-way ANOVA Test).
**Fig. 11c** shows representative images of H&E staining of Sc from *T.mu-* (left panel) and *T.mu*+ (right panel) mice harvested at the chronic phase of the disease.
**Fig. 11d** shows representative images of LFB staining of Sc from *T.mu-* (left panel) and *T.mu*+ (right panel) mice harvested at the chronic phase of the disease.
**Fig. 11e** shows graphs depicting the quantity of H&E (left panel) and LFB (right panel) staining from the experiments shown in Fig. 11c and Fig. 11d, respectively. The experiment was performed once with 5 mice per group. *p<0.05 (Mann Whitney Test, Mean and SEM).
**Fig. 11f** shows graphs depicting the quantity of IgA detected in fecal (left panel) and serum (right panel) samples collected from the experiment shown in Fig. 11a. The experiment was performed once with 10 mice per group. All mice are depicted. **p<0.01, ***p<0.001, ****p<0.0001, NS (Not Significant) (Mann Whitney Test, Mean and SEM).
**Fig. 11g** shows graphs depicting the frequency of total IgA-ASC in the SILP (left panel), BM (middle panel) and Br (right panel) samples collected during the chronic phase of EAE from the experiment shown in Fig. 11a. Experiment was performed once with 5 mice per group. *p<0.05, ***p<0.001 (Mann Whitney Test, Mean and SEM).
**Fig. 11h** shows graphs depicting the frequency (left panel) and absolute number (right panel) of cytokine producing CD4⁺ T cells from the Sc of *T.mu-* and *T*.mu+ mice at the chronic stage of EAE evaluated by flow cytometry. *p<0.05, NS (Not Significant) (Mann Whitney Test, Mean and SEM).
**Fig. 11i** shows graphs depicting clinical score over time (left panel) and cumulative score (right panel) of BM chimeras in which B cell deficient *Jht*^{*-*/-} mice were reconstituted with WT or IgA^{-/-} BM and subjected to EAE. **p<0.01, (Mann Whitney Test, Mean and SEM).
**Fig. 11j** shows representative flow cytometry plots of SILP-resident cells from 10BiT mice. Expression of Thy1.1 (which reports on the expression of *Il10* mRNA) is represented by a histogram (right panel) derived from pre-gating on single cell live lymphocytes from the SILP of 10BiT mice.
**Fig. 12a** shows graphs depicting EAE clinical scores over time (left panel) and cumulative score (right panel) in *Nos2*^{*-*/*-*} + *Jht*^{*-*/}*⁻ -> Jht*^{*-*/*-*} and *Nos2*^{*-*/*-*} + WT -> WT mixed-BM chimeras. The experiment was performed 4 times with 6-10 mice per group. *p<0.05, NS (Not Significant) (Two-way ANOVA and Mann Whitney Test, Mean and SEM).
**Fig. 12b** shows graphs depicting EAE clinical scores over time (left panel) and cumulative score (right panel) in *Nos2*^{*-*/*-*} + *Cd19*^{cre}*Prdm1*^{*-*/}*⁻ -> Jht*^{*-*/*-*} and *Nos2*^{*-*/*-*} + WT -> *Jht*^{-/-} mixed-BM chimeras. The experiment was performed once with 7 mice per group. ***p<0.001 (Two-way ANOVA and Mann Whitney Test, Mean and SEM).
**Fig. 12c** shows representative images of H&E staining in the Sc of *Nos2*^{*-*/*-*} + WT → *Jht*^{-/-} (left panel) and *Nos2*^{*-*/*-*} + *Cd19*^{cre}*Prdm1*^{*-*/*-*} → *Jht*^{*-*/*-*} (right panel) mixed-BM chimeras.
**Fig. 12d** shows representative images of LFB staining in the Sc of *Nos2*^{*-*/*-*} + WT → *Jht*^{*-*/-} (left panel) and *Nos2*^{*-*/*-*} + *Cd19*^{cre}*Prdm1*^{*-*/*-*} → *Jht*^{*-*/*-*} (right panel) mixed-BM chimeras.
**Fig. 12e** shows graphs depicting the quantity of H&E (top panel) and LFB (bottom panel) staining from the experiments shown in Fig. 12c and Fig. 12d, respectively. **p<0.01 (Mann Whitney Test, Mean and SEM).
**Fig. 13a** shows graphs depicting the frequency (left panel) and absolute numbers (right panel) of cytokine Interferon γ (IFNy), Interleukin 17 (IL-17) and Granulocyte-macrophage colony-stimulating factor (GM-CSF) producing CD4+ T cells from the Sc of WT and BAFF-Tg^{+/-}mice during adoptive transfer EAE as evaluated by flow cytometry. The experiment was performed once with 5-6 mice per group. *p<0.05, NS (Not Significant) (Mann Whitney Test, Mean and SEM).
**Fig. 13b** shows a graph (left panel) and representative images (right panel) of a quantification of commensal-reactive IgA ASC in the Br of WT and Baff-Tg mice after adoptive transfer EAE as evaluated by ELISPOT. Experiment was performed once with 5-6 mice per group. NS (Not Significant) (Mann Whitney Test, Mean and SEM).
**Fig. 13c** shows representative images of H&E staining of the Sc from WT (left panel) and BAFF-Tg^{+/-} (right panel) mice during adoptive transfer EAE.
**Fig. 13d** shows representative images of LFB staining of the Sc from WT (left panel) and BAFF-Tg^{+/-} (right panel) mice during adoptive transfer EAE.
**Fig. 13e** shows graphs depicting the quantity of H&E (left panel) and LFB (right panel) staining from the experiments shown in Fig. 13c and Fig. 13d, respectively. The experiment was performed once with 5-6 mice per group. **p<0.01 (Mann Whitney Test, Mean and SEM).
**Fig. 13f** is a graph showing EAE clinical scores over time in WT and *Tnfrsf13b*^{*-*/*-*} mice that lack TACI. The WT mice are littermate controls derived from back-crosses to C57BI/6 animals. The experiment was performed twice with 5-9 mice per group. **p<0.01 (Two-way ANOVA Test).
**Fig. 13g** shows representative immunofluorescence images of DAPI and CD138 staining, or a merged image of DAPI, IgA, IL10 and CD138 staining, in small intestinal samples from BAFF-Tg^{+/+} (top panel) and BAFF-Tg^{+/-} × IL10^{-/-} (bottom panel) mice. Arrows in the zoomed image of BAFF-Tg^{+/+} SILP represent cells that are positive for both IgA and IL10 and arrows in the zoomed image of BAFF-Tg^{+/-} × IL10^{-/-} SILP represent cells that are positive for IgA only.
**Fig. 13h** is a graph showing the number of IgA⁺ and IgA⁺IL10⁺ cells per 100 µm² from WT or BAFF-Tg^{+/+} mice. The dotted line represents the number of IL10⁺ cells counted in an IL10^{-/-} mouse to show the specificity of the assay. The experiment was performed once with 5 mice per group. **p<0.01, ***p<0.001, ****p<0.0001 (Mann Whitney Test, Mean and SEM).
**Fig. 13i** shows representative flow cytometry dot plot (left panel) and histogram plot (right panel) of intracellular detection of IL10 in IgA⁺B220⁻PB/PC in Baff-Tg mice. The experiment was performed 2 times with 6 mice per group.
**Fig. 14** is a graph showing EAE clinical scores over time in WT C57BL6 mice immunized with MOG₃₅₋₅₅ and intraperitoneally injected with PBS (control) or a BAFF polypeptide fused to a Fragment crystallizable region (BAFF-Fc) at day 8 and day 12 (indicated by arrows) after immunization. The 16-point clinical score scale was used to evaluate symptoms. The experiment was performed once with 4 mice per group. ***p<0.005 (Two-way ANOVA Test, Mean and SEM).
**Fig. 15a** is a graph showing the viability of total lymphocytes derived from mouse bone marrow, evaluated by FACS using Aqua viability dye after D2 and D5 of culture, using different concentrations of BAFF-Fc. The experiment was performed twice.
**Fig. 15b** is a graph showing the viability of IgA+PB/PC specifically derived from mouse bone marrow, evaluated by FACS using Aqua viability dye after D2 and D5 of culture, using different concentrations of BAFF-Fc. The experiment was performed twice
**Fig. 16a** is a graph depicting the frequency of commensal-reactive IgA ASC per million cells derived from different tissues in BAFF-Tg mice using an ELISPOT assay. The experiment was performed twice with 2 mice per group (Mean and SEM).
**Fig. 16b** shows representative images of commensal-reactive IgA ELISPOTS in different tissues from BAFF-Tg mice.
**Fig. 17a** is a graph showing the that a variable number of commensal-reactive IgA ASC can be detected by ELISPOT in the peripheral blood of healthy volunteers. (Mean and SEM, * p=0.05, Mann-Whithney Test).
**Fig. 17b** is a graph showing that a dynamic number of commensal-reactive IgA ASC are detected in PBMC from the same volunteer over time.
**Fig. 17c** shows representative images of the commensal-lgA ASC obtained by ELISPOT using thawed PBMC from healthy volunteers after a B/PC enrichment with negative selection beads.
**Fig. 18a** is a graph showing the frequency of cell viability in total PBMC or derived from B cells/PC only, evaluated by FACS using the Aqua viability marker after D3 of culture with or without BAFF-Fc.
**Fig. 18b** depicts representative FACS dot plot images showing an enrichment of IL-10 producing IgA PB/PC after in vitro culture with BAFF-Fc.
**Fig. 18c** is a graph showing the effect of addition of BAFF-Fc (10ng/ml) during the Elispot assay on the number of commensal-reactive IgA ASC.
**Fig. 18d** is a graph showing the effect of BAFF-Fc on the size of commensal-reactive IgA ASC spots in the ELISPOT assay. (Mean and SEM, * p=0.05, Mann-Whithney Test).

### DETAILED DESCRIPTION OF THE DISCLOSURE

Methods for treating an autoimmune disease in a subject; for reducing inflammation in a subject; for enriching gut-derived commensal-reactive IgA+ plasmablasts and/or plasma cells in the central nervous system of a subject; and for promoting survival of gut-derived commensal-reactive IgA+ plasmablasts and/or plasma cells in a subject to reduce inflammation in a tissue are disclosed.

### Definitions

Unless defined otherwise, all technical and scientific terms used herein generally have the same meaning as commonly understood by one of ordinary skill in the art to which this disclosure belongs.

As used herein, the singular forms "a," "an," and "the," are intended to include the plural forms as well, unless the context clearly indicates otherwise.

The phrase "and/or" should be understood to mean "either or both" of the elements so conjoined, i.e., elements that are conjunctively present in some cases and disjunctively present in other cases. Thus, as a non-limiting example, a reference to "A and/or B", when used in conjunction with open-ended language such as "comprising" can refer, in one disclosure, to A only (optionally including elements other than B); in another disclosure, to B only (optionally including elements other than A); in yet another disclosure, to both A and B (optionally including other elements); etc.

As used herein, the phrase "one or more," in reference to a list of one or more elements, should be understood to mean at least one element selected from any one or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "one or more" refers, whether related or unrelated to those elements specifically identified. Thus, as a non-limiting example, "one or more of A and B" (or, equivalently, "one or more of A or B," or, equivalently "one or more of A and/or B") can refer, in one disclosure, to at least one, optionally including more than one, A, with no B present (and optionally including elements other than B); in another disclosure, to at least one, optionally including more than one, B, with no A present (and optionally including elements other than A); in yet another disclosure, to at least one, optionally including more than one, A, and at least one, optionally including more than one, B (and optionally including other elements); etc.

When the term "about" is used in conjunction with a numerical range, it modifies that range by extending the boundaries above and below those numerical values. In general, the term "about" is used herein to modify a numerical value above and below the stated value by a variance of 20%, 10%, 5%, or 1%. The term "about" is used to modify a numerical value above and below the stated value by a variance of 10%. In certain disclosures, the term "about" is used to modify a numerical value above and below the stated value by a variance of 5%. The term "about" is used to modify a numerical value above and below the stated value by a variance of 1%.

When a range of values is listed herein, it is intended to encompass each value and sub-range within that range. For example, "1-5 ng" is intended to encompass 1 ng, 2 ng, 3 ng, 4 ng, 5 ng, 1-2 ng, 1-3 ng, 1-4 ng, 1-5 ng, 2-3 ng, 2-4 ng, 2-5 ng, 3-4 ng, 3-5 ng, and 4-5 ng.

A "subject" is a vertebrate, preferably a mammal (e.g., a non-human mammal), more preferably a primate and still more preferably a human. Mammals include, but are not limited to, primates, humans, farm animals, sport animals, and pets.

It will be further understood that the terms "comprises," "comprising," "includes," and/or "including," when used herein, specify the presence of stated features, integers, steps, operations, elements, and/or components, but do not preclude the presence or addition of one or more other features, integers, steps, operations, elements, components, and/or groups thereof.

The term "treatment", "treat" or "treating" or "amelioration" as used herein is an approach for obtaining beneficial or desired clinical results. For purposes of this disclosure, beneficial or desired clinical results include, but are not limited to, one or more of the following: reduction in inflammation, decreased extent of damage from a disease, condition, or disorder, decreased duration of a disease, condition, or disorder, and/or reduction in the number, extent, or duration of symptoms related to a disease, condition, or disorder. The term includes the administration of the compounds, agents, drugs or pharmaceutical compositions of the present disclosure to prevent or delay the onset of one or more symptoms, complications, or biochemical indicia of a disease or condition; lessening or improving one or more symptoms; shortening or reduction in duration of a symptom; or arresting or inhibiting further development of a disease, condition, or disorder. Treatment may be prophylactic (to prevent or delay the onset of a disease, condition, or disorder, or to prevent the manifestation of clinical or subclinical symptoms thereof) or therapeutic suppression or alleviation of symptoms after the manifestation of a disease, condition, or disorder.

The term "autoimmune disease" as used herein refers to a disease, disorder or condition where a host's immune cells attack its own cells and/or tissues, resulting in damage to these cells and/or tissues. Examples of autoimmune disease include, but are not limited, to multiple sclerosis, autoimmune arthritis and type I diabetes. The autoimmune disease may be relapsing-remitting multiple sclerosis, primary progressive multiple sclerosis or secondary progressive multiple sclerosis.

The term "non-systemic organ-specific autoimmune disease" as used herein refers to a disease in which an immune response is directed toward antigens in a specific organ or specific organ system of a subject. The term "non-systemic organ-specific autoimmune disease" is used in contrast to the term "systemic autoimmune disease", which refers to a disease in which an immune response is directed against self-antigens present in many organs and tissues of the body resulting in widespread tissue damage to the host. Examples of systems in which a non-systemic organ-specific autoimmune disease include, but are not limited to, the central nervous system. Examples of a non-systemic organ-specific autoimmune disease include, but are not limited to, multiple sclerosis, autoimmune arthritis and type I diabetes. The non-systemic organ-specific autoimmune disease may be relapsing-remitting multiple sclerosis, primary progressive multiple sclerosis or secondary progressive multiple sclerosis.

The term "administering," refers to the placement of an agent, a drug, a compound, or a pharmaceutical composition as disclosed herein into a subject by a method or route which results in at least partial delivery of the composition at a desired site. The agent, drug, compound, or pharmaceutical composition disclosed herein can be administered by any appropriate route which results in an effective treatment in the subject.

The term "effective amount" as used herein is an amount sufficient to affect any one or more beneficial or desired results. An effective amount may prevent, alleviate or ameliorate symptoms of a disease, condition, or disorder. For prophylactic use, beneficial or desired results may include eliminating or reducing the risk, lessening the severity, or delaying the onset of a disease, condition, or disorder, including biochemical, histological and/or behavioral symptoms of the disease, condition, or disorder, its complications and intermediate pathological phenotypes presenting during development of the disease, condition, or disorder. For therapeutic use, beneficial or desired results may include clinical results such as reducing one or more symptoms of an autoimmune disease, decreasing the dose of other medications required to treat the disease, enhancing the effect of another medication, and/or delaying the progression of the disease in a subject. Beneficial or desired results may also include reducing inflammation, enriching gut-derived commensal-reactive IgA+ plasmablasts and/or plasma cells in a tissue, organ or organ system of interest and/or promoting survival of gut-derived commensal-reactive IgA+ plasmablasts and/or plasma cells. An effective dosage can be administered in one or more administrations.

For purposes of this disclosure, an effective dosage of an agent, a drug, a compound, or a pharmaceutical composition is an amount sufficient to accomplish prophylactic or therapeutic treatment either directly or indirectly. As is understood in the clinical context, an effective dosage of an agent, a drug, a compound, or a pharmaceutical composition may or may not be achieved in conjunction with another agent, drug, compound, or pharmaceutical composition. Thus, an "effective dosage" may be considered in the context of administering one or more therapeutic agents, and a single agent may be considered to be given in an effective amount if, in conjunction with one or more other agents, a desirable result may be or is achieved. The amount may vary from one subject to another and may depend upon one or more factors, such as, for example, subject gender, age, body weight, subject's health history, and/or the underlying cause of the disease, condition, or disorder to be prevented, inhibited and/or treated.

The term "B-cell Activating Factor polypeptide" or "BAFF polypeptide" refers to any naturally occurring polypeptide of BAFF, which may be derived from any suitable organism. As used herein, "BAFF" refers to a mammalian BAFF, such as human, rat or mouse, as well as non-human primate, bovine, ovine, or porcine BAFF. The BAFF polypeptide may be human (see, e.g., Accession Number NP_006564.1) and is an amino acid molecule comprising the amino acid sequence shown in SEQ ID NO: 1, or is encoded by a nucleic acid molecule comprising the polynucleotide sequence shown in SEQ ID NO: 2 (see APPENDIX). The BAFF polypeptide is murine (see, e.g., Accession Number NP_296371.1) and is an amino acid molecule comprising the amino acid sequence shown in SEQ ID NO: 3, or is encoded by a nucleic acid molecule comprising the polynucleotide sequence shown in SEQ ID NO: 4 (see APPENDIX). The term "BAFF polypeptide" also encompasses portions, variants, isoforms, and other homologs of such BAFF molecules. A variant of BAFF includes those sequences wherein one or more nucleotides of the polynucleotide or polypeptides of the polypeptide sequence have been substituted, deleted, and/or inserted that encode a functional BAFF polypeptide. Variants of BAFF will preferably have at least 80%, more preferably, at least 90%, and even more preferably, at least 95% amino acid sequence identity with the native sequence BAFF polypeptide. Variant BAFF molecules will generally be characterized by having the same type of activity as naturally occurring BAFF, such as the ability to induce class switching of B cells from IgM to IgA *in vitro.* Methods for detecting class switching of B cells from IgM to IgA *in vitro* are known to those skilled in the art. BAFF polypeptides can be isolated from nature or can be produced by recombinant and/or synthetic means. BAFF polypeptides may be modified to self-assemble into a higher order oligomer. Methods for oligomerizing polypeptides are known to those skilled in the art.

The term "reducing inflammation" as used herein refers to decreasing pain, redness, swelling, heat, and/or loss of function in a subject as compared to the pain, redness, swelling, heat, and/or loss of function in a subject not administered one or more of: a BAFF polypeptide; a BAFF polypeptide and an agent that promotes survival and/or migration of gut-derived commensal-reactive B cells to the central nervous system of the subject; a BAFF polypeptide and an agent that depletes B cells; or a BAFF polypeptide and a gut commensal that increases IgA levels. The reduced inflammation may be reduced neuroinflammation. In a patient with MS, "reducing inflammation" may refer to a reduction of lymphocytes in the central nervous system. The reduction in inflammation may be about 5%, 10%, 15%, 20%, 25%, 30%, 35%, 40%, 45%, 50%, 55%, 60%, 65%, 70%, 75%, 80%, 85%, 90%, or 95% relative to the inflammation in a subject not administered one or more of: a BAFF polypeptide; a BAFF polypeptide and an agent that promotes survival and/or migration of gut-derived commensal-reactive B cells to the central nervous system of the subject; a BAFF polypeptide and an agent that depletes B cells; or a BAFF polypeptide and a gut commensal that increases IgA levels. Methods for assessing reduced inflammation in subjects with MS are known in the art and include, for example, Expanded Disability Status Scale (EDSS), or tracking gadolinium-enhanced brain lesions by Magnetic Resonance Imaging (MRI).

The term "commensal-reactive IgA+ plasmablasts and/or plasma cell" as used herein refers to a plasmablasts and/or plasma cell that produces at least IgA antibodies which react with a gut commensal. Methods for detecting the presence of commensal-reactive IgA+ plasmablasts and/or plasma cells in a tissue (e.g., in the blood) may include, for example, a commensal-reactive ELISPOT assay as disclosed herein. A commensal-reactive IgA+ plasmablast and/or plasma cell may also produce immunoregulatory molecules. Such immunoregulatory molecules may include cytokines and/or chemokines. Such immunoregulatory molecules may include, for example, interleukin-10 (IL-10), interleukin-35 (IL-35), inducible nitric oxide synthase (iNOS), programmed death-ligand 1 (PD-L1), and/or lymphocyte-activation gene 3 (LAG3). Methods for detecting such immunoregulatory molecules are known to those skilled in the art.

The term "enriching cells in the central nervous system" as used herein means increasing the number and/or survival of cells in the central nervous system, and/or promoting migration of cells to the central nervous system in a subject as compared to a subject not administered one or more of: a BAFF polypeptide; a BAFF polypeptide and an agent that promotes survival and/or migration of gut-derived commensal-reactive B cells to the central nervous system of the subject; a BAFF polypeptide and an agent that depletes B cells; or a BAFF polypeptide and a gut commensal that increases IgA levels. The cells enriched in the central nervous system may be plasmablasts and/or plasma cells (PB/PC). The cells enriched in the central nervous system may be derived from the gut. The cells enriched in the central nervous system may be gut-derived commensal-reactive IgA+ PB/PC. Methods for assessing enrichment of cells in the central nervous system are known to those skilled in the art. Enrichment of cells, including enrichment of IgA+ PB/PC, in the central nervous system may be assessed, for example, by determining IgA levels in cerebrospinal fluid using flow cytometry. The cells in the central nervous system may be enriched by about 5%, 10%, 15%, 20%, 25%, 30%, 35%, 40%, 45%, 50%, 55%, 60%, 65%, 70%, 75%, 80%, 85%, 90%, or 95% relative to the enrichment in the central nervous system in a subject not administered one or more of: a BAFF polypeptide; a BAFF polypeptide and an agent that promotes survival and/or migration of gut-derived commensal-reactive B cells to the central nervous system of the subject; a BAFF polypeptide and an agent that depletes B cells; or a BAFF polypeptide and a gut commensal that increases IgA levels. The enrichment may be any order of magnitude greater, for example, about 1.5 times, 2 times, 5 times or 10 times greater than the enrichment in a subject not administered one or more of: a BAFF polypeptide; a BAFF polypeptide and an agent that promotes survival and/or migration of gut-derived commensal-reactive B cells to the central nervous system of the subject; a BAFF polypeptide and an agent that depletes B cells; or a BAFF polypeptide and a gut commensal that increases IgA levels.

The term "promoting survival" as used herein refers to increasing the survival of cells in a subject as compared to the survival in a subject not administered one or more of: a BAFF polypeptide; a BAFF polypeptide and an agent that promotes survival and/or migration of gut-derived commensal-reactive B cells to the central nervous system of the subject; a BAFF polypeptide and an agent that depletes B cells; or a BAFF polypeptide and a gut commensal that increases IgA levels. Methods for assessing cell survival are known to those skilled in the art. The cells with increased survival may be plasmablasts and/or plasma cells. The plasmablasts and/or plasma cells with increased survival may be derived from the gut. The cells with increased survival may be gut-derived commensal-reactive IgA+ plasmablasts and/or plasma cells. The increase in survival may be about 5%, 10%, 15%, 20%, 25%, 30%, 35%, 40%, 45%, 50%, 55%, 60%, 65%, 70%, 75%, 80%, 85%, 90%, or 95% relative to the to the survival in a subject not administered one or more of: a BAFF polypeptide; a BAFF polypeptide and an agent that promotes survival and/or migration of gut-derived commensal-reactive B cells to the central nervous system of the subject; a BAFF polypeptide and an agent that depletes B cells; or a BAFF polypeptide and a gut commensal that increases IgA levels. The increase in survival may be any increase or order of magnitude greater, for example, about 1.5 times, 2 times, 5 times or 10 times greater than the survival in a subject not administered one or more of: a BAFF polypeptide; a BAFF polypeptide and an agent that promotes survival and/or migration of gut-derived commensal-reactive B cells to the central nervous system of the subject; a BAFF polypeptide and an agent that depletes B cells; or a BAFF polypeptide and a gut commensal that increases IgA levels.

The term "promoting migration" as used herein refers to increasing the number of cells that move to a desired location in a subject as compared to the number of cells in a subject not administered one or more of: a BAFF polypeptide; a BAFF polypeptide and an agent that promotes survival and/or migration of gut-derived commensal-reactive B cells to the central nervous system of the subject; a BAFF polypeptide and an agent that depletes B cells; or a BAFF polypeptide and a gut commensal that increases IgA levels. The desired location may be a site of inflammation in the subject such as an inflamed tissue or an organ or system of organs associated with an autoimmune disease. The desired location may be the central nervous system. The cells migrating to the central nervous system may be IgA+ PB/PC. Methods for assessing migration of IgA+ PB/PC to the central nervous system may include, for example, determining IgA levels in cerebrospinal fluid using flow cytometry. The migration may be increased about 5%, 10%, 15%, 20%, 25%, 30%, 35%, 40%, 45%, 50%, 55%, 60%, 65%, 70%, 75%, 80%, 85%, 90%, or 95% relative to the migration in a subject not administered one or more of: a BAFF polypeptide; a BAFF polypeptide and an agent that promotes survival and/or migration of gut-derived commensal-reactive B cells to the central nervous system of the subject; a BAFF polypeptide and an agent that depletes B cells; or a BAFF polypeptide and a gut commensal that increases IgA levels. The increase in migration may be any increase or order of magnitude greater, for example, about 1.5 times, 2 times, 5 times or 10 times greater than the migration in a subject not administered one or more of: a BAFF polypeptide; a BAFF polypeptide and an agent that promotes survival and/or migration of gut-derived commensal-reactive B cells to the central nervous system of the subject; a BAFF polypeptide and an agent that depletes B cells; or a BAFF polypeptide and a gut commensal that increases IgA levels.

The term "agent that depletes B cells" as used herein refers to an agent, a drug, a compound, or a pharmaceutical composition that decreases the number of B cells, such as memory B cells that promote an autoimmune response and/or inflammation in a tissue, an organ, and/or an organ system. The agent may deplete commensal non-reactive B cells (e.g., B cells that do not react with a gut commensal). The agent may deplete pathogenic B cells (B cells, the activities of which contribute to the pathogenesis of a disease, for example, an autoimmune disease). Methods for determining the number of B cells are known to those skilled in the art. The agent that depletes B cells may be a drug, a compound, or a pharmaceutical composition. The agent that depletes B cells may be, for example, an antibody or antigen-binding fragment thereof, an antibody-drug conjugate, or a pharmaceutical composition comprising the antibody or antibody-drug conjugate. The agent that depletes B cells may be an antibody or antigen-binding fragment thereof that specifically binds to Cluster of Differentiation 19 (CD19) and/or CD20 proteins on the surface of B cells. The agent that depletes B cells may be an inhibitor of the Bruton's Tyrosine Kinase (BTK) signaling pathway. The B cells may be depleted about 5%, 10%, 15%, 20%, 25%, 30%, 35%, 40%, 45%, 50%, 55%, 60%, 65%, 70%, 75%, 80%, 85%, 90%, or 95% relative to the depletion in a subject not administered the agent that depletes B cells.

The term "gut commensal" as used herein refers to an organism or a group of organisms that are in a commensal symbiotic relationship with a host, and reside in or are derived from the gut of the host. The gut commensal may be a microbe, for example, a bacterium or a protist. The gut commensal may be a commensal microbe that is isolated from other commensal microbes, or it may be a combination or mixture thereof, such as a commensal microbial community, also referred to as a microbiome or a microbiota. The commensal microbial community may be a fecal microbial community. The gut commensal may comprise *Tritrichomonas musculis (T.mu)* or a gut microbial community that has been modified by the carriage, or by the introduction, of *T.mu.* The gut commensal may comprise isolated *T.mu* alone or *T.mu* in combination with one or more other commensal microbes.

The term "gut-derived" or "derived from the gut" as used herein refers to a cell that originates in the gut, for example, in the gut-associated lymphoid tissue (GALT) and/or the small intestinal Lamina Propria (SILP). The cell derived from the gut may be a plasmablast and/or plasma cell, for example, an IgA producing (IgA+) plasmablast and/or plasma cell. Cells derived from the gut may be detected, for example, the blood can be evaluated for cells that express the gut homing molecule alpha4beta7 and/or the chemokine receptors CCR9 and CCR10. Alternatively, the cells can be evaluated for the ability to produce IgA2 or characterized as producing the secretory form of IgA, which is produced only in the mucosa (whereas lgA1 is produced in the mucosa and in the periphery, e.g., in the bone marrow).

The term "pharmaceutically acceptable carrier" or "pharmaceutical acceptable excipient" as used herein includes any material which, when combined with an active ingredient, allows the ingredient to retain biological activity and is non-reactive with the subject's immune system. Examples include, but are not limited to, any of the standard pharmaceutical carriers such as a phosphate buffered saline solution, water, emulsions such as oil/water emulsion, and various types of wetting agents. Diluents for aerosol or parenteral administration may be phosphate buffered saline (PBS) or normal (0.9%) saline. Compositions comprising such carriers are formulated by well-known conventional methods (see, for example, Remington's Pharmaceutical Sciences, 18th edition, A. Gennaro, ed., Mack Publishing Co., Easton, PA, 1990; and Remington, The Science and Practice of Pharmacy 20th Ed. Mack Publishing, 2000).

### General techniques

Unless otherwise defined herein, scientific and technical terms used in connection with the present disclosure shall have the meanings that are commonly understood by those of ordinary skill in the art. Generally, nomenclatures used in connection with, and techniques of, cell and tissue culture, molecular biology, immunology, microbiology, genetics and protein and nucleic acid chemistry and hybridization described herein are those well-known and commonly used in the art.

The practice of the present disclosure will employ, unless otherwise indicated, conventional techniques of molecular biology (including recombinant techniques), microbiology, cell biology, biochemistry and immunology, which are within the skill of the art. Such techniques are explained fully in the literature, such as, Molecular Cloning: A Laboratory Manual, second edition (Sambrook et al., 1989) Cold Spring Harbor Press; Oligonucleotide Synthesis (M.J. Gait, ed., 1984); Methods in Molecular Biology, Humana Press; Cell Biology: A Laboratory Notebook (J.E. Cellis, ed., 1998) Academic Press; Animal Cell Culture (R.I. Freshney, ed., 1987); Introduction to Cell and Tissue Culture (J.P. Mather and P.E. Roberts, 1998) Plenum Press; Cell and Tissue Culture: Laboratory Procedures (A. Doyle, J.B. Griffiths, and D.G. Newell, eds., 1993-1998) J. Wiley and Sons; Methods in Enzymology (Academic Press, Inc.); Handbook of Experimental Immunology (D.M. Weir and C.C. Blackwell, eds.); Gene Transfer Vectors for Mammalian Cells (J.M. Miller and M.P. Calos, eds., 1987); Current Protocols in Molecular Biology (F.M. Ausubel et al., eds., 1987); PCR: The Polymerase Chain Reaction, (Mullis et al., eds., 1994); Current Protocols in Immunology (J.E. Coligan et al., eds., 1991); Sambrook and Russell, Molecular Cloning: A Laboratory Manual, 3rd. ed., Cold Spring Harbor Laboratory Press, Cold Spring Harbor, NY (2001); Ausubel et al., Current Protocols in Molecular Biology, John Wiley & Sons, NY (2002); Harlow and Lane Using Antibodies: A Laboratory Manual, Cold Spring Harbor Laboratory Press, Cold Spring Harbor, NY (1998); Coligan et al., Short Protocols in Protein Science, John Wiley & Sons, NY (2003); Short Protocols in Molecular Biology (Wiley and Sons, 1999); and Immunobiology (C.A. Janeway and P. Travers, 1997).

The inventors have shown that a "gut/brain" axis exists in the context of an animal model of MS. While IgA-producing PC are ideally situated to produce large quantities of anti-commensal antibodies in the gut during homeostasis, the inventors disclose that intestinal IgA⁺ B cells (one of the biggest reservoirs of lymphocytes in the body) represent a population of regulatory cells that can be recruited to inflamed tissues independent of their B cell receptor specificity. Accordingly, the inventors provide methods for treating an autoimmune disease in a subject; for reducing inflammation in a subject; for enriching gut-derived commensal-reactive IgA+ plasmablasts and/or plasma cells in the central nervous system of a subject; and for promoting survival of gut-derived commensal-reactive IgA+ plasmablasts and/or plasma cells in a subject to reduce inflammation in a tissue. Such method comprise administering to the subject an effective amount of one or more of: a B-cell Activating Factor (BAFF) polypeptide; a BAFF polypeptide and an agent that promotes survival and/or migration of gut-derived commensal-reactive B cells to the central nervous system of the subject; a BAFF polypeptide and an agent that depletes B cells; or a BAFF polypeptide and a gut commensal that increases IgA levels in the subject.

### BAFF polypeptide

The BAFF polypeptide may be a fragment or variant of BAFF that has the activity of BAFF. The BAFF may be mammalian BAFF. The BAFF may be human BAFF, such as shown in SEQ ID NO: 1 or such as encoded by a polynucleotide shown in SEQ ID NO: 2. The BAFF may be mouse BAFF, such as shown in SEQ ID NO: 3 or such as encoded by a polynucleotide shown in SEQ ID NO: 4.

Polynucleotides may comprise a native sequence (i.e., an endogenous sequence that encodes a BAFF polypeptide or a portion thereof) or may comprise a variant of such a sequence. Polynucleotide variants contain one or more substitutions, additions, deletions and/or insertions such that the biological activity of the encoded polypeptide is not diminished, relative to the native polypeptide. The variants may exhibit at least about 70% identity, at least about 80% identity, at least about 90% identity, at least about 95%, 96%, 97%, 98% or 99% identity to a polynucleotide sequence that encodes a native BAFF polypeptide or a portion thereof. These amounts are not meant to be limiting, and increments between the recited percentages are specifically envisioned as part of the disclosure.

Two polynucleotide or polypeptide sequences are said to be "identical" if the sequence of nucleotides or amino acids in the two sequences is the same when aligned for maximum correspondence as described below. Comparisons between two sequences are typically performed by comparing the sequences over a comparison window to identify and compare local regions of sequence similarity. A "comparison window" as used herein, refers to a segment of at least about 20 contiguous positions, usually 30 to about 75, or 40 to about 50, in which a sequence may be compared to a reference sequence of the same number of contiguous positions after the two sequences are optimally aligned.

Optimal alignment of sequences for comparison may be conducted using the MegAlign^{®} program in the Lasergene^{®} suite of bioinformatics software (DNASTAR^{®}, Inc., Madison, WI), using default parameters.

Variants may also, or alternatively, be substantially homologous to a native gene, or a portion or complement thereof. Such polynucleotide variants are capable of hybridizing under moderately stringent conditions to a naturally occurring DNA sequence encoding a native antibody (or a complementary sequence).

Suitable "moderately stringent conditions" include prewashing in a solution of 5X SSC, 0.5% SDS, 1.0 mM EDTA (pH 8.0); hybridizing at 50 °C-65 °C, 5X SSC, overnight; followed by washing twice at 65°C for 20 minutes with each of 2X, 0.5X and 0.2X SSC containing 0.1% SDS.

As used herein, "highly stringent conditions" or "high stringency conditions" are those that: (1) employ low ionic strength and high temperature for washing, for example 0.015 M sodium chloride/0.0015 M sodium citrate/0.1% sodium dodecyl sulfate at 50 °C; (2) employ during hybridization a denaturing agent, such as formamide, for example, 50% (v/v) formamide with 0.1% bovine serum albumin/0.1% Ficoll/0.1% polyvinylpyrrolidone/50 mM sodium phosphate buffer at pH 6.5 with 750 mM sodium chloride, 75 mM sodium citrate at 42 °C; or (3) employ 50% formamide, 5X SSC (0.75 M NaCl, 0.075 M sodium citrate), 50 mM sodium phosphate (pH 6.8), 0.1% sodium pyrophosphate, 5X Denhardt's solution, sonicated salmon sperm DNA (50 µg/mL), 0.1% SDS, and 10% dextran sulfate at 42 °C, with washes at 42 °C in 0.2X SSC (sodium chloride/sodium citrate) and 50% formamide at 55 °C, followed by a high-stringency wash consisting of 0.1X SSC containing EDTA at 55 °C. The skilled artisan will recognize how to adjust the temperature, ionic strength, etc. as necessary to accommodate factors such as probe length and the like.

A BAFF polypeptide of the disclosure may be made by any method known in the art. General techniques for production of recombinant polynucleotides and polypeptides are known in the art and/or are described herein. For example, DNA encoding a BAFF polypeptide is readily isolated and sequenced using conventional procedures (e.g., by using oligonucleotide probes that are capable of binding specifically to genes encoding the heavy and light chains of the monoclonal antibodies). Once isolated, the DNA can be inserted into a suitable vector, and the vector in turn can be introduced into a suitable host cell for replication and amplification, as further discussed herein. Polynucleotides may be inserted into host cells by any means known in the art. Cells are transformed by introducing an exogenous polynucleotide by direct uptake, endocytosis, transfection, F-mating or electroporation. Once introduced, the exogenous polynucleotide can be maintained within the cell as a non-integrated vector (such as a plasmid) or integrated into the host cell genome. The polynucleotide so amplified can be isolated from the host cell by methods well known within the art. See, e.g., Sambrook et al., 1989.

The polynucleotide may be placed into expression vectors known to those skilled in the art, which are then transfected into host cells such as *E. coli* cells, simian COS cells, Chinese hamster ovary (CHO) cells, or any other cell type known to those skilled in the art to obtain the synthesis of the BAFF polypeptide in the recombinant host cells. The BAFF polypeptide can then be isolated from the host cell using standard techniques known in the art.

A BAFF polypeptide may also be made by chemical synthesis. Methods of chemical synthesis are known in the art and are commercially available.

A BAFF polypeptide may be fused or conjugated with a moiety or a protein domain that targets the BAFF polypeptide to specific organs, tissues and/or cells within the body. A BAFF polypeptide may be fused or conjugated with a moiety or a protein domain to enhance solubility, stability, and/or receptor binding of the BAFF polypeptide. Techniques for recombinantly fusing and chemically conjugating polypeptides to a moiety or protein domain of interest are known to those skilled in the art. For example, a BAFF polypeptide may be fused to a Fragment crystallizable (Fc) immunoglobulin domain, for example a human Fc polypeptide. This allows the BAFF to be soluble in circulation in order to bind to any of its three receptors TNFRSF13b (TACI), TNFRSF13c (BAFF-R), and TNFRSF17 (BCMA).

### BAFF polypeptide in combination with an agent that promotes survival and/or migration of gut-derived commensal-reactive B cells to the central nervous system

A BAFF polypeptide may be used in combination with an agent that promotes survival and/or migration of gut-derived commensal-reactive B cells to the central nervous system. The agent that promotes survival and/or migration of gut-derived commensal-reactive B cells to the central nervous system may be a drug, a compound, or a pharmaceutical composition. The agent that promotes survival and/or migration of gut-derived commensal-reactive B cells to the central nervous system may be a cytokine or a chemokine. The cytokine or chemokine may be isolated from nature or can be produced by recombinant and/or synthetic means, as known to those skilled in the art.

The agent that promotes survival and/or migration of gut-derived commensal-reactive B cells to the central nervous system is a cytokine or a chemokine. The agent that promotes survival and/or migration of gut-derived commensal-reactive B cells to the central nervous system may be, for example, IL-10, IL-35, iNOS, PD-L1, LAG3, or a combination thereof. The agent that promotes survival and/or migration of gut-derived commensal-reactive B cells to the central nervous system may be a survival factor such as IL-6, APRIL, or a combination thereof. The IL-10, IL-35, iNOS, PD-L1, LAG3, IL-6 and APRIL may be isolated from nature or can be produced by recombinant and/or synthetic means, as known to those skilled in the art. A number of agents that promote survival and/or migration of gut-derived commensal-reactive B cells to the central nervous system are commercially available.

The BAFF polypeptide and the agent that promotes survival and/or migration of gut-derived commensal-reactive B cells to the central nervous system can be administered concurrently or sequentially in any order. The therapies may be combined in a single pharmaceutical composition, or formulated in separate pharmaceutical formulations.

### BAFF polypeptide in combination with an agent that depletes B cells

A BAFF polypeptide may be used in combination with an agent that depletes B cells. The agent that depletes B cells may be a drug, a compound, or a pharmaceutical composition. The agent that depletes B cells may deplete pathogenic and/or commensal-nonreactive B cells. The agent that depletes B cells may comprise an antibody. The antibody may bind to B cell markers. A number of B cell markers are known to those skilled in the art and may be suitable for use in combination with BAFF polypeptide. The agent that depletes B cells may comprise an antibody that binds to CD19 and/or CD20 proteins on the cell surface, and/or an antibody that binds to BTK protein. The antibody that binds to CD19, CD20 and/or BTK may be a monoclonal antibody, a chimeric antibody, a single chain antibody, a tetrameric antibody, a tetravalent antibody, a multispecific antibody, a domain-specific antibody, a domain-deleted antibody, a conjugated antibody, a fusion protein, an ScFc fusion protein, an Fab fragment, an Fab' fragment, an F(ab')2 fragment, an Fv fragment, an ScFv fragment, an Fd fragment, a single domain antibody, a nanobody, a dAb fragment, or a small modular immunopharmaceutical (SMIP). The agent that depletes B cells may be an inhibitor of CD19 and/or CD20, or an inhibitor of the BTK signaling pathway. Methods for making an agent that depletes B cells are known in the art. For example, anti-CD19 and anti-CD20 drugs are commercially available.

The BAFF polypeptide and the agent that depletes B cells can be administered concurrently or sequentially in any order. The therapies may be combined in a single pharmaceutical composition, or formulated in separate pharmaceutical formulations.

### BAFF polypeptide in combination with a gut commensal that increases IgA levels

A BAFF polypeptide may be used in combination with a gut commensal that increases IgA levels. The gut commensal may be a microbe, for example, a bacterium or a protist. The gut commensal may be a commensal microbe that is isolated from other commensal microbes, or it may be a combination or mixture thereof, such as a commensal microbial community, also referred to as a microbiome or a microbiota. The commensal microbial community may be a fecal microbial community. The gut commensal may comprise *Tritrichomonas musculis (T.mu)* or a gut microbial community that has been modified by the carriage, or by the introduction, of *T.mu.* The gut commensal may comprise isolated *T.mu* alone or *T.mu* in combination with one or more other commensal microbes. The gut commensal may comprise a microbe/community of microbes that supports the generation of immunoregulatory IgA plasma cells.

The BAFF polypeptide and the gut commensal that increases IgA levels can be administered concurrently or sequentially in any order. The therapies may be combined in a single pharmaceutical composition, or formulated in separate pharmaceutical formulations.

Pharmaceutical compositions comprising a BAFF polypeptide; and/or a BAFF polypeptide and an agent that promotes survival and/or migration of gut-derived commensal-reactive B cells to the central nervous system of the subject; and/or a BAFF polypeptide and an agent that depletes B cells; and/or a BAFF polypeptide and a gut commensal that increases IgA levels in the subject may be formulated in a conventional manner using one or more pharmaceutically acceptable carriers or excipients or stabilizers (Remington: The Science and practice of Pharmacy 20th Ed., 2000, Lippincott Williams and Wilkins, Ed. K. E. Hoover) and may be, for example, in the form of lyophilized formulations or aqueous solutions. Acceptable carriers, excipients, or stabilizers are nontoxic to recipients at the dosages and concentrations.

### Therapeutic methods

The methods disclosed herein comprise administering one or more of a BAFF polypeptide; and/or a BAFF polypeptide and an agent that promotes survival and/or migration of gut-derived commensal-reactive B cells to the central nervous system of the subject; and/or a BAFF polypeptide and an agent that depletes B cells; and/or a BAFF polypeptide and a gut commensal that increases IgA levels in the subject. The method may comprise administering two of the above therapies to the subject. The method may comprise administering three of the above therapies to the subject. The method may comprise administering all four of the above therapies to the subject. When used in combination the therapies can be administered concurrently or sequentially in any order. The therapies may be combined in a single pharmaceutical composition, or formulated in separate pharmaceutical formulations.

Therapeutic methods involve administering to a subject in need of treatment a therapeutically effective amount, or effective amount, of a BAFF polypeptide; and/or a BAFF polypeptide and an agent that promotes survival and/or migration of gut-derived commensal-reactive B cells to the central nervous system of the subject; and/or a BAFF polypeptide and an agent that depletes B cells; and/or a BAFF polypeptide and a gut commensal that increases IgA levels in the subject are contemplated by the present disclosure. One of ordinary skill in the art would be able to determine such amounts based on such factors as the subject's size, the nature of the disease to be treated, the severity of the subject's symptoms, and the particular composition or route of administration selected. The subject may be a human or non-human animal (e.g., rabbit, rat, mouse, monkey or other lower-order primate).

A BAFF polypeptide; and/or a BAFF polypeptide and an agent that promotes survival and/or migration of gut-derived commensal-reactive B cells to the central nervous system of the subject; and/or a BAFF polypeptide and an agent that depletes B cells; and/or a BAFF polypeptide and a gut commensal that increases IgA levels in the subject are contemplated by the present disclosure may be co-administered with known medicaments, and in some instances the therapy itself, for example, the BAFF polypeptide itself might itself be modified. A BAFF polypeptide may be modified to increase solubility, stability and/or receptor binding. For example, a BAFF polypeptide may be multimerized using a scaffolding compound in order to increase its binding avidity to the receptors TNFRSF13b (TACI) and/or TNFRSF17 (BCMA). Regarding coadministration with additional therapeutic agents, such agents can include a cytotoxic agent, a radiotoxic agent or an immunosuppressive agent. A BAFF polypeptide can be linked to the agent or can be administered separately from the agent. In the latter case (separate administration), a BAFF polypeptide can be administered before, after or concurrently with the agent or can be co-administered with other known therapies.

A BAFF polypeptide; and/or a BAFF polypeptide and an agent that promotes survival and/or migration of gut-derived commensal-reactive B cells to the central nervous system of the subject; and/or a BAFF polypeptide and an agent that depletes B cells; and/or a BAFF polypeptide and a gut commensal that increases IgA levels in the subject may be used as a therapeutic in a variety of situations, such as inflammatory conditions such as an autoimmune disease, non-autoimmune disorders such as atherosclerosis or situations where immunosuppression is desired. The autoimmune disease may be a non-systemic organ-specific autoimmune disease. Examples of a non-systemic organ-specific autoimmune disease include, but are not limited to, multiple sclerosis, autoimmune arthritis and type I diabetes. The non-systemic organ-specific autoimmune disease may be multiple sclerosis. The non-systemic organ-specific autoimmune disease may be relapsing-remitting multiple sclerosis, primary progressive multiple sclerosis or secondary progressive multiple sclerosis.

The studies described herein use Experimental Autoimmune Encephalomyelitis (EAE), an animal model of MS. EAE is a disease that requires autoimmune T cell activation. The skilled person would appreciate that other diseases that are characterized by autoimmune T cells such as multiple sclerosis, autoimmune arthritis and type I diabetes would also benefit from the therapeutic methods disclosed herein.

The subject who may benefit from the therapeutic methods disclosed herein may have an autoimmune disease and further have a non-autoimmune disorder. The non-autoimmune disorder may be atherosclerosis. It has been shown that patients with autoimmune diseases, such as systemic lupus erythematosus and rheumatoid arthritis, have a markedly increased risk of atherosclerosis and atherosclerotic cardiovascular disease, and that BAFF overexpression reduced hypercholesterolemia and atherosclerosis in hyperlipidemic mice. the subject who may benefit from the therapeutic methods disclosed herein may have multiple sclerosis and also has atherosclerosis.

Routes of administration of the BAFF polypeptide; and/or a BAFF polypeptide and an agent that promotes survival and/or migration of gut-derived commensal-reactive B cells to the central nervous system of the subject; and/or a BAFF polypeptide and an agent that depletes B cells; and/or a BAFF polypeptide and a gut commensal that increases IgA levels in the subject include but are not limited to topical, transdermal, parenteral, gastrointestinal, transbronchial and transalveolar. Topical administration is accomplished via a topically applied cream, gel, rinse, etc. containing an oligonucleotide conjugate. Transdermal administration is accomplished by application of a cream, rinse, gel, etc. capable of allowing the BAFF polypeptide; and/or a BAFF polypeptide and an agent that promotes survival and/or migration of gut-derived commensal-reactive B cells to the central nervous system of the subject; and/or a BAFF polypeptide and an agent that depletes B cells; and/or a BAFF polypeptide and a gut commensal that increases IgA levels in the subject to penetrate the skin and enter the blood stream. Parenteral routes of administration include but are not limited to electrical or direct injection such as direct injection into a central venous line, intravenous, intramuscular, intraperitoneal, intrathecal or subcutaneous injection. Gastrointestinal routes of administration include but are not limited to ingestion and rectal administration. Transbronchial and transalveolar routes of administration include but are not limited to inhalation, either via the mouth or intranasally. Administration may also be done directly to the site of the autoimmune disease or inflammation. For example, administration to the CNS may be done by an intra-cerebral ventricular injection directly into the cerebrospinal fluid.

A BAFF polypeptide may be co-administered with a gut commensal that increases IgA levels. The gut commensal that increases IgA levels may be administered rectally, for example, by fecal transplantation. Methods for performing fecal transplantation are known to those skilled in the art, for example, by colonoscopy, enema, orogastric tube. The gut commensal that increases IgA levels may also be administered orally in the form of a capsule containing freeze-dried fecal matter or material derived and/or isolated from fecal matter.

### EXAMPLES

The disclosure is further described in detail by reference to the following experimental examples. These examples are provided for purposes of illustration only, and are not intended to be limiting unless otherwise specified. Thus, the disclosure should in no way be construed as being limited to the following examples, but rather, should be construed to encompass any and all variations which become evident as a result of the teaching provided herein.

### EXAMPLE 1: Experimental models and subject details

A list of reagents and resources used to carry out the experiments disclosed herein is provided in Table I. The source and the corresponding identifier of each reagent or resource is also provided.

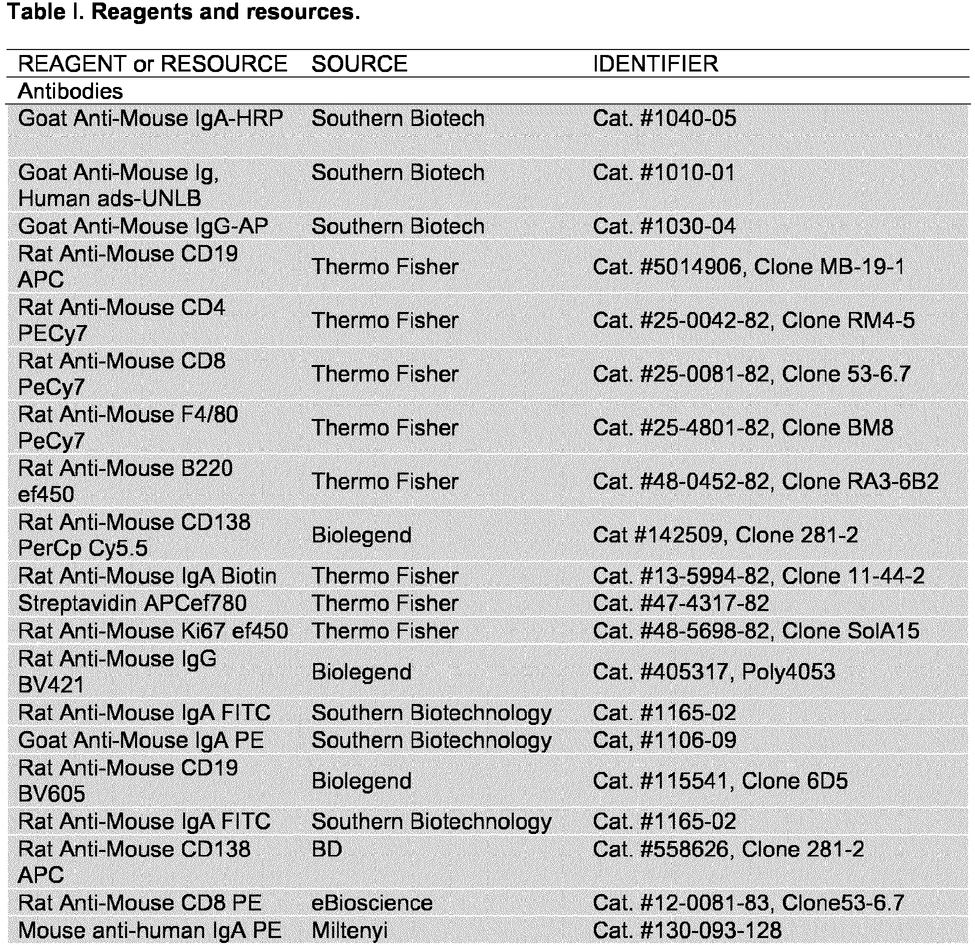

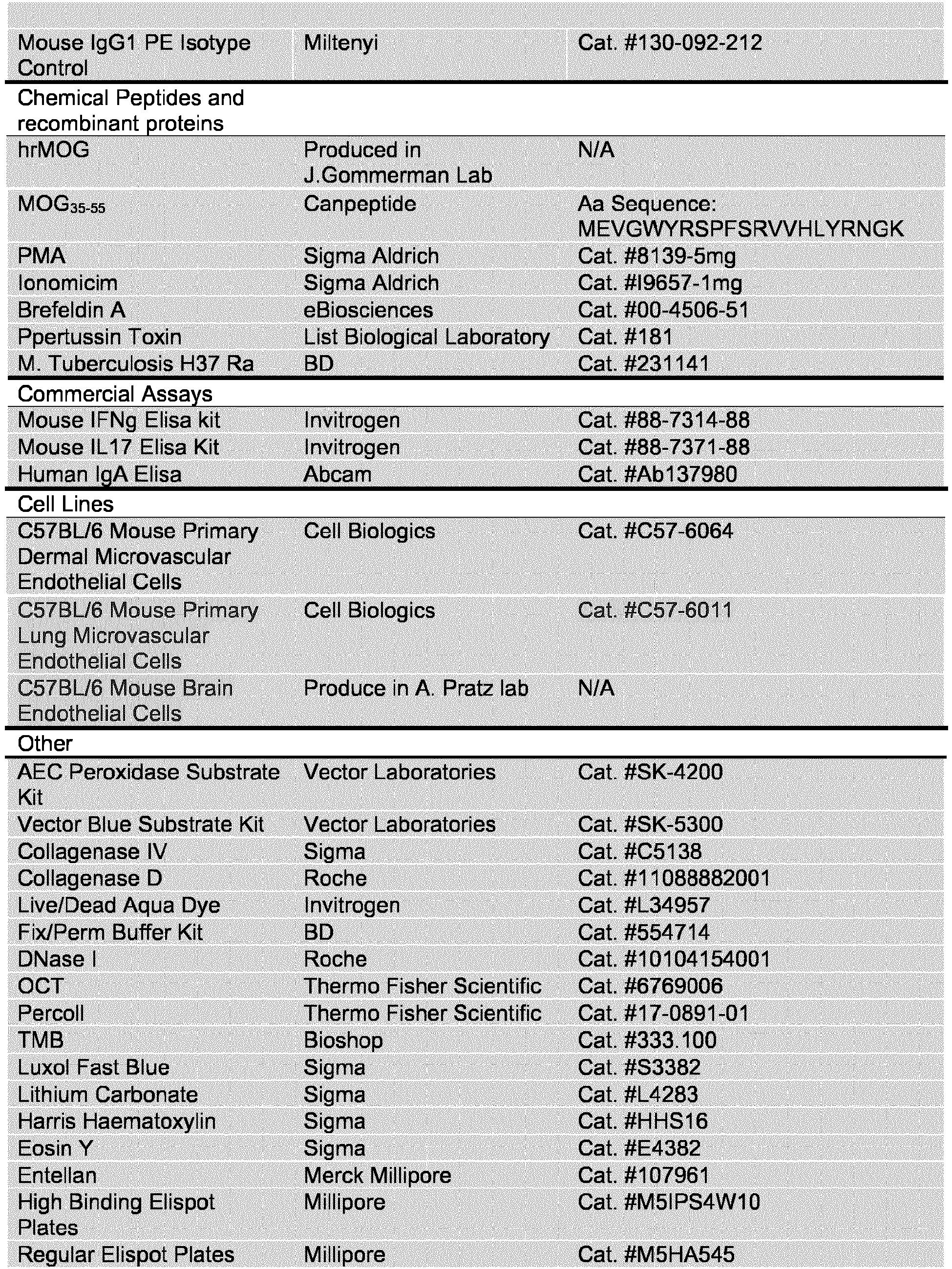

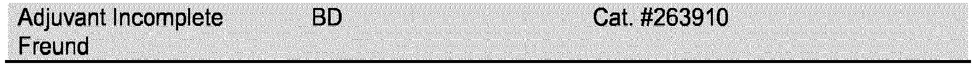

### Mice

*Prdm1*^{fl/fl} mice (Jackson Labs) were back-crossed with C57BL/6 mice (Charles River), *Aicd*^{Cre}xYFP^{fl/fl} mice (courtesy of Rafael Casellas) (Crouch et al., 2007) or *Cd*19Cre^{+/-} mice (Jackson Labs) to generate PC conditional knockout mice. *Prdm1*^{yfp} mice were purchased from Jackson Labs and subsequently interbred to maintain the line. BAFF-Transgenic (BAFF-Tg) mice (obtained from Drs. Ann Ranger and Jeff Browning, Biogen Inc) (McCarthy et al., 2011) were backcrossed with WT mice (Charles River) and subsequently interbred as BAFF-Tg^{+/+} or BAFF-Tg^{+l-} mice. BAFF-Tg^{+/+} were crossed with *Il10*^{*-*/*-*} to generate littermates for experiments. *Igh-J^{tm1Cgn}* (*Jht*^{*-*/*-*}), IgA^{-/-} and *Nos2*^{-/-}mice were used in some cases to generate BM chimeras. *Tnfsf13b*^{*-*/*-*} and corresponding littermate controls were housed at the University of Melbourne. 1110-Thy1.1 transcriptional reporter (10BiT) mice were provided by Dr. David Brooks at the University Health Network with permission from Casey Weaver. Kaede mice were generated as described (Tomura et al., 2008) and provided by Dr. Andrew Luster's lab at Harvard University and subsequently interbred to maintain the line. The genotypes of the above models were confirmed by PCR or qPCR. All animals were housed in a specific pathogen free, closed caging system and provided with a standard irradiated chow diet (Envigo Teklad (2918)), acidified water (reverse-osmosis and UV-sterilized) and housed under a 12-hour light cycle. All animal experiments were conducted with ethical approval from the University of Toronto, Faculty of Medicine animal care committee and the University of Melbourne animal care committee. The majority of the experiments were performed first in separately caged mice and then were repeated with co-caged and/or littermate mice to confirm the results.

### Bone marrow chimeras

In order to develop single or mixed-BM chimeras, BM cells from *Il10*^{*-*/*-*}, *Nos2*^{*-*/*-*} or IgA^{-/-}mice were used as donors alone or in combination with B-cell deficient *Jht*^{*-*/*-*}, WT, or ***Cd19^{cre}Prdm1*^{fl/fl}** BM cells, to reconstitute B-cell deficient *Jht*^{-/-} irradiated recipient mice thus creating BM chimeras in which B cells or PC are unable to produce IL10, iNOS or IgA.

### Induction of EAE

To induce EAE, female mice, 7 weeks of age, were immunized subcutaneously on day 0 with 100 µg of MOG₃₅₋₅₅ peptide or 100 µg of recombinant human MOG₁₋₁₂₀ (rhMOG) and 500 µg of H37Ra (DIFCO Laboratories) emulsified in incomplete Freund's adjuvant (BD, Biosciences). Mice were subcutaneously injected with 100µl of emulsion in 3 previously disinfected locations on the back for a total of 300µl of injected material per mouse. Pertussis toxin (PTX - List Biological Laboratories) was subsequently injected intraperitonally twice in 500µL PBS, on days 0 and 2, 500ng per injection. Due to high mortality in some strains, daily nursing was increased to improve survival by placing cages on heat pads with Pure-o'Cel paperless bedding, diet was supplemented with breeder mash (Envigo Teklad (2919)) to help mice gain or maintain body weight, and subcutaneous injections of 1ml-1.5ml of lactated Ringer's solution, as well as 5% dextrose for caloric supplementation were provided 1-3 times daily depending on symptoms and signs of dehydration. Disease incidence was typically 100%. This mouse model does not relapse, but rather displays a chronic form of EAE. Clinical signs of EAE were assessed daily (blinded) with a modified 0-16 clinical scoring system to better evaluate paralysis on each limb individually (Giuliani et al., 2005; Pikor et al., 2015). This composite scale provides superior sensitivity compared to the typical 5-point scale system, taking into account paralysis of each of the 4 limbs separately (Emerson et al., 2009; Fleming et al., 2005). Onset of disease was defined as a score ≥1, for at least two consecutive days. Peak phase was defined as the time point with the maximum score during disease. Chronic phase was defined as a consistent decrease in the peak score, persisting for at least two consecutive days. A cumulative clinical score was calculated for each mouse by adding the daily scores from the day of onset until the end of the experiment.

For some experiments, the conventional 6-score scale was also employed. In those cases, scores were assigned as follows: 0=asymptomatic, 1=loss of tail tone, 2=hind limb weakness, 3=partial hind limb paralysis, 4=complete hind limb paralysis, and 6=moribund.

### Influenza infection

Influenza strain A/Puerto Rico/8/1934(H1N1), also known as "PR8", was provided by Dr. Tania Watts, University of Toronto. Stock vials were diluted (sterile) between 5×10³ to 10⁶ TCID₅₀/mouse depending on the experiment in no more than 30µl/vial. Diluted stocks were kept sterile and on ice for the duration of the infection procedure. Mice were anesthetized with 5% isofluorane at an induction volume of 3L/min. Mice were removed from the isofluorane mask and using a P20 pipette and sterile tips, mice were given 15µl of diluted virus per nostril. Mice were administered more isofluorane between each nostril dose to ensure mice did not wake up mid-infection. Mice were placed back into their cage to revive. Weight was monitored daily. Mash food was given when mice lost approximately 10% of their body weight, and fluids were given on approximately day 4.

### Rotavirus infection

Mice received 100µl of 1.33% of Sodium Bicarbonate (Na₂CO₃) prior to infection with Rotavirus (RV) to neutralize stomach acid. Mice were then inoculated by oral gavage with 100µl 10⁴ ID₅₀ of EC virus (1:100 of the virus stock 10⁷ ID₅₀/ml in Medium199 (Sigma)), and then monitored for RV clearance by measuring RV antigen in stool. To detect RV antigen with an enzyme linked immunosorbent assay (ELISA), NUNC maxi-sorp 96-well plates were coated with rabbit-α-RV (ABD Serotech, CAT #AHP1360) diluted 1:2000 in TNC (10mM Tris, 100mM NaCl, 1mM CaCl₂, Ph 7.4) overnight at 4°C. Plates were then blocked with 5% BLOTTO (5% skim milk powder in TNC) for 2 hours at 37°C. Plates were dumped and patted dry. Fecal samples were added in duplicate at a dilution of 1/2 in 1% BLOTTO, and incubated for 2 hours at 37°C. Plates were washed 5 times with ~200µl 0.1% TWEEN20/TNC. 50µl/well of mouse-α-RV monoclonal antibody was added at 1:200 and incubated for 1 hour at 37°C. Plates were washed 3 times as before. 50µl/well of goat-α-mouse-IgG2b-HRP (Southern Biotech, CAT #1090-05) was added at 1:1000 and incubated for 30 min at 37°C. Plates were washed 3 times as before and developed with 50µl of 3,3',5,5'-Tetramethylbenzidine (Bioshop) and stopped with 50µl H₂SO₄. Plates were read on a photo-spectrometer at 450nm. An internal control for the RV ELISA was generated using fecal samples combined from several mice and tested for abundance of the RV Ag.

### Tritrichomonas musculis colonization

Purification of *T.mu* was performed as described (Chudnovskiy et al., 2016). Briefly, the cecal content of *T.mu* containing mice was harvested into 20ml of sterile PBS at 4°C and filtered through a 70µm cell strainer. Filtered cecal content was then spun at 1400rpm for 7min at 4°C. The supernatant was discarded and the pellet was washed twice with 40ml of sterile PBS. The pellet was then resuspended in 5ml of 40% percoll and overlaid on 5ml of 80% percoll. The 40/80% percoll (1X sterile PBS) was made from a 90% percoll solution diluted with 10X sterile PBS. The 40/80% percoll gradient centrifugation step was performed at 2500rpm for 20min at 20°C. The percoll interface containing *T.mu* was collected and spun down at 1400rpm for 7min at 4°C. The pellet was then resuspended in 5ml of sterile PBS and filtered again through a 70µm cell strainer. *T.mu* were then sorted into sterile PBS on a BD Influx using the 100µm nozzle at 27psi at 4°C. Purity was >99%. Sorted *T.mu* were then spun down at 1400rpm for 7min at 4°C and the pellet was resuspended in sterile PBS. Two million sorted *T.mu* were orally gavaged into C57BL/6 mice (Charles River) immediately after the sort. Mice were subjected to EAE three weeks post-infection. To quantify efficiency of infection, cecums were harvested and cut longitudinally. Cecal content was resuspended in 10ml of sterile PBS. Trophozoites were counted using a hemocytometer.

### EXAMPLE 2: Experimental methods

### Tissue harvesting

In all cases, mice were euthanized using a CO₂ tank attached to an animal cage with a flowmeter to regulate CO₂ pressure to 2-3 litres per minute as per the animal facility's standard operating procedures. Subsequently, mice were perfused with 30ml of cold PBS (Sigma) via the left ventricle whilst cutting the right atrium to allow fluid escape. To measure RV-specific IgA ASC in the lungs, perfusion protocol was adapted to achieve better perfusion of the lungs by pushing the needle up through the left ventricle to the left atrium to preferentially target the pulmonary circulation. Following perfusion, the lungs were removed and placed in cold PBS in a 6-well plate. Using a 70µm cell strainer and a 5ml syringe insert, lungs were mashed through the cell strainer mesh to make a single cell suspension. Cells were spun down at 1200rpm for 5 min and resuspended in 2ml of 80% Percoll (GE Healthcare) and 2ml of 40% Percoll was laid gently on top. Tubes were centrifuged at 1600 rpm (585 RCFxg) for 25 min to separate the fat on the top layer, lymphocytes at the midpoint between the 80% and 40% solutions, and debris in the bottom pellet. Approximately 1ml was collected from the midline to collect the lymphocytes and placed in a new tube with PBS and centrifuged at 1200rpm for 5 min. Cells were washed once more and then used for downstream applications.

For BM isolation, following euthanasia and perfusion, the skin of the mouse's leg was removed and the leg was detached at the hip joint. Using scissors and gauze, the muscle tissue was cleaned off the femur and tibia. The ends of each bone that forms the joint were removed and both the femur and tibia were placed in a punctured 0.6ml PCR tube placed at the opening of a 1.5ml microtube with 200µl of PBS (Sigma). Samples were pulse spun such that the marrow from each bone was collected at the bottom of the microtube. The bones were discarded and the BM pellets were resuspended in RBC lysis buffer (155mM NH₄Cl, 12mM NaHCO₃, 0.1mM EDTA) for 5 min on ice. Lysis buffer containing the cells was transferred to a 50mL tube containing 10ml of PBS to stop the lysis reaction and the cells were spun down at 1200rpm for 5 min. Samples were washed once more using the same method. Cells were kept on ice until ready for further use.

Blood was collected from the saphenous leg vein into capillary tubes. Samples were spun down at 10,000 rpm (9,000 RCFxg) for 5-7 minutes and serum was collected and transferred into autoclaved 0.6ml microtubes. All samples were frozen at -20°C until further use. For fecal matter preparation, 2-3 pellets of fecal matter were collected in 1.5ml microtubes. All fecal matter was frozen at -20°C until further use then subsequently thawed and weighed prior to downstream applications.

Axillary and inguinal Lymph Nodes (AxLN or iLN) as well as spleen (Sp) were collected and placed into cold PBS in a 6-well plate. Using a 70µm cell strainer and the back of an insert of a 5ml syringe, LNs were mashed through the mesh of the cell strainer to make a single cell suspension. Red blood cell lysis was performed at this point for spleen only. Cells were washed once in cold PBS and centrifuged at 1200rpm (329 RCFxg) for 5 min. Cells were kept on ice until ready for further use.

Brain (Br) and spinal cord (Sc) were homogenized first using a 70 µm strainer and a 10ml syringe, then cell suspensions were incubated with the addition of 60 µg/ml DNasel for 45 min at 37°C. The use of Collagenase D for Br digestion was avoided because it resulted in decreased PC recovery/viability. Lymphocytes were purified using a 30% Percoll solution and resuspended in PBS for counting and flow cytometry staining or in complete RPMI media (10% FBS, L-glutamine, sodium pyruvate, penicillin G, streptomycin sulfate, and β-mercaptoethanol) for ELISPOT assay.

SILP preparations were performed as previously described (Fritz et al., 2011). Briefly, small intestines were dissected and cleaned *in situ* of mesenteric fat and Peyer's patches were removed. Small pieces of the intestine were then thoroughly washed and EDTA solution was used to remove IELs. The remaining LP fraction was then digested with collagenase IV (Sigma-Aldrich, USA) and lymphocytes were enriched by Percoll gradient (GE Healthcare, Sweden). Given the inherent variability in gut preparations, cellular compartments were enumerated based on frequency rather than absolute numbers and complemented these findings with immunofluorescence.

For endothelial cell preparations, primary cultures of C57BL/6 murine brain parenchymal capillary endothelial cells were prepared as previously published (Lecuyer et al., 2017). Primary cultures of C57BL/6 murine lung and dermal endothelial cells were thawed and prepared as per manufacturer's instructions (Cell Biologics).

### Flow Cytometry

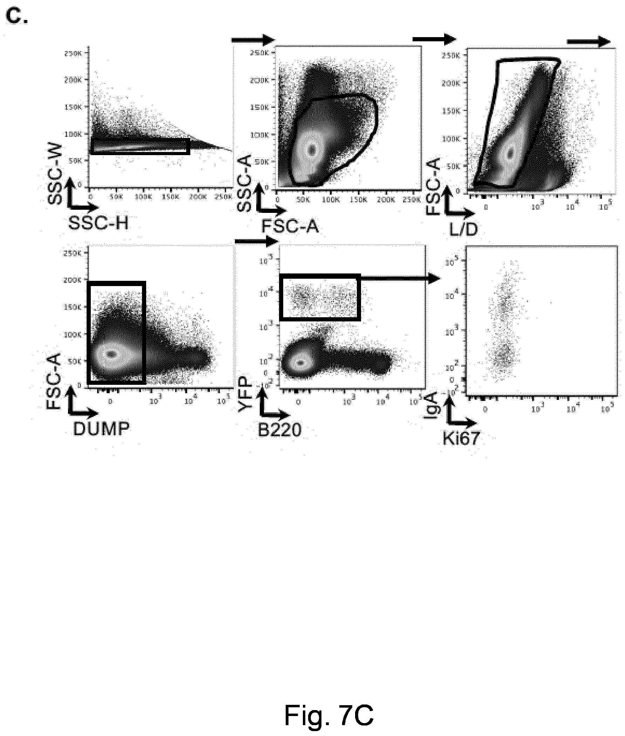

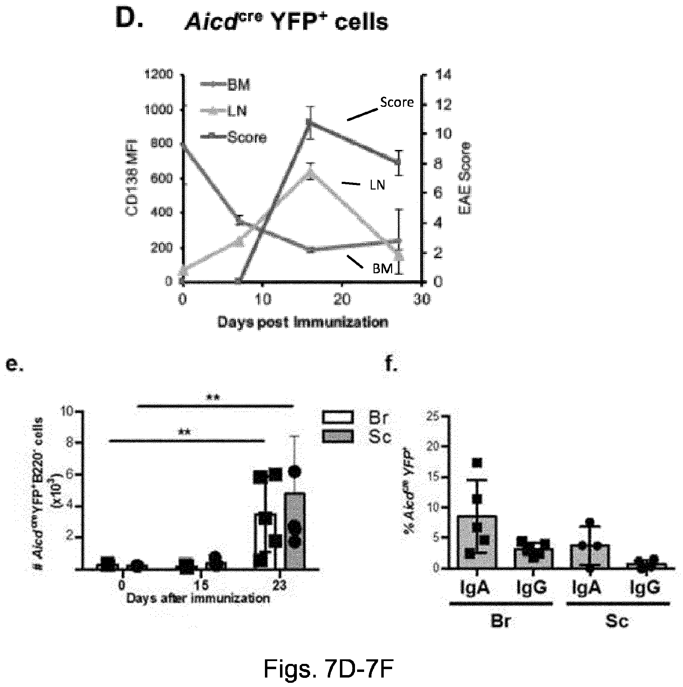

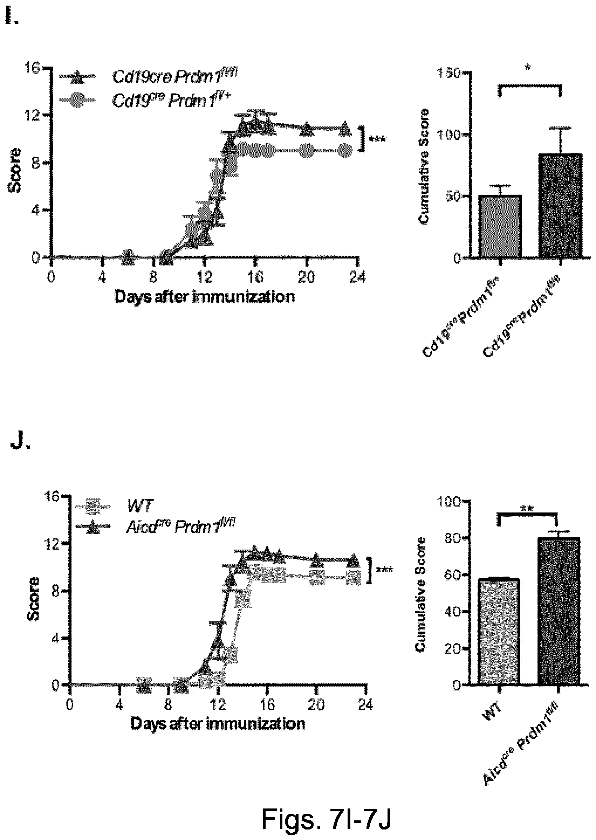

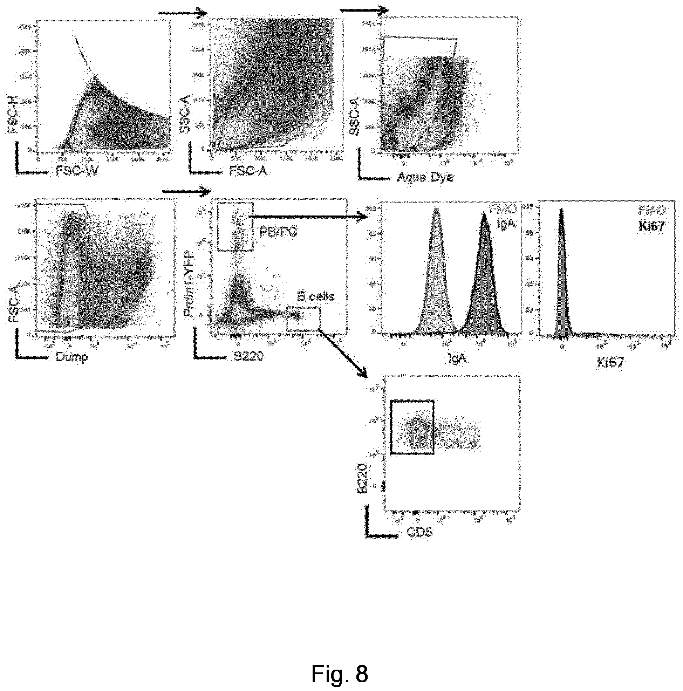

Cells were washed with ice-cold PBS containing 2% FBS (Wisent Inc, Canada) and prior to antibody staining a live/dead stain was applied using a fixable aqua dead cell stain kit (Molecular Probes). Subsequently, cells were incubated with 1 mg/ml of a rat anti-mouse CD16/CD32 antibody ("Fc-block", clone: 2.4G2 made in house) to block non-specific staining for 15 minutes at 4°C. Pre-determined concentrations of fluorochrome labeled antibodies were then added in a total volume of 100µl, thoroughly mixed with the cells and incubated for 15 minutes at 4°C. The following antibodies were used in different combinations among 3 panels, all of which were purchased from Ebiosciences unless otherwise noted: rat anti-mouse CD19-APC (1D3), rat anti-mouse CD4-PECy7 (GK1.5), CD8-PECy7, F4/80-PECy7 (collectively used as a "dump" gate, see Fig. 7c and Fig. 8), rat anti-mouse CD45R (B220)-eFluor450 (RA3-6B2), and rat anti-mouse CD138-PercpCy5.5 (281-2, BD Biosciences). After washing with FACS buffer, cells were fixed and permeabilized using a cytofix/cytoperm kit from BD Biosciences according to the manufacturer's protocol. Intracellular staining was then performed for 30 min at 4°C using the following antibodies: rat anti-mouse IgA-biotin (11-44-2) followed by Streptavidin-APC-eF780, Ki67-eFluor450, IgG-BV450 or IgA-FITC. In the case of T cell cytokine detection, the following antibodies were used: anti-mouse IFNγ PE, anti-mouse IL17 PerCPCy5.5 and anti-mouse GM-CSF FITC. Cells were then washed twice with Perm/Wash buffer and resuspended in FACS buffer prior to flow cytometric acquisition using either a Fortessa or an LSR-II instrument (BD Biosciences). Acquired data was analyzed and processed using FlowJo (Tree Star Inc.).

### Immunofluorescence microscopy

Small intestinal tissues were obtained from mouse dissection before EAE induction (D0), during the peak of the disease (D15), and during the chronic phase (D21 or D23). Briefly, after euthanasia and perfusion, small intestines were removed and placed on plastic wrap to clean out the fecal content. At the midpoint, curved forceps were used to clear the feces from the intestine and a 1cm piece was cut out, avoiding the Peyer's patches. The piece was placed in a histology tray and covered in OCT (Sakura Finetek) and was frozen in 2-methyl butane cooled on dry ice. Trays were wrapped in aluminum foil and stored at -80°C until further use. 5µm frozen sections were subsequently cut with a Leica CM3050 cryostat in preparation for acetone fixation and staining. For a more comprehensive examination of the tissues, five slides were prepared from each sample with two sections on each slide. Defrosted and PBS-re-hydrated tissues were subjected to Fc receptor blocking with Superblock solution in TBS (Thermo Scientific, USA) followed by primary antibody staining with rat-anti-mouse antibodies. Data depicted were obtained by staining with IgA-FITC, CD138-PE, and CD8α-APC, although some experiments used CD138-APC with CD8α-PE, or IL10 PE. After nuclear staining with DAPI, the mounted slides were visualized by microscopy using a LeicaDMRA2 microscope with OpenLab software where each fluorochrome was assigned a colour (DAPI = yellow, YFP/FITC = green, APC = blue, PE = red). Single RGB images were saved as TIFF files. In Photoshop (version CC), the original images were cropped using a 1000px × 1000px marquis and zoomed images were derived from these cropped images using a 250px × 250px marquis. Subsequently, brightness was adjusted for each fluorochrome uniformly across samples. TIFFS were then merged as overlays on imaged 1.15s (National Institute of Health, USA) and using the cell counter function, the number of CD138⁺ or IgA⁺ cells per every 10 CD8α⁺ cells was quantified. Immunofluorescence was used to confirm that numbers of CD8α⁺ cells in the SILP did not change during EAE. In the case of IL10/IgA counts, the number of cell counts were normalized according to the area. In order to account for the variability among sections within a tissue, a minimum of two to six images (technical replicates) on each biological replicate were acquired from each sample and an average cell count was taken for the final analysis.

### Hematoxylin & Eosin and Luxol Fast Blue staining

Mice whose spinal columns were harvested for histology were euthanized with CO₂ and intracardially perfused with PBS. The spinal columns were excised and post-fixed in 10% buffered formalin for 1 week prior to processed into paraffin. Seven micron paraffin coronal sections of mouse spinal cord were mounted on Superfrost Plus glass slides (Knittel Glass) and dried overnight at 37°C. Paraffin sections were deparaffinated in xylene and rehydrated through a series of ethanol. Histology was performed using standard Hematoxylin & Eosin (H&E) to visualize immune cell infiltration, and Luxol Fast Blue (LFB), which stains myelin and allows for visualization of areas of demyelination. RGB images of H&E and LFB stains from thoracic spinal cord sections were acquired at 4X magnification using a light microscope (Zeiss Axioscope). Quantitative analysis of stains was performed using imaged 1.15s in a blinded manner. The RGB images were separated into single color channels using the color deconvolution plugin. Single color channel for hematoxylin and LFB were subjected to thresholding followed by the Area Fraction measurement on the region of interest (total white matter area). Staining is expressed as percentage stained area of total white matter area.

### ELISPOT analysis

Membrane plates (Milipore HA clear plates, sterile 0.45um surfactant-free mixed cellulose ester membrane MSHAS4510) were coated (sterile) with goat-α-mouse -Ig diluted 1:1000 for the total Ig ELISPOT (Southern Biotechnologies #1010-01, 1mg/ml) diluted 1:1000, or coated with rabbit-anti-RV polyclonal antibody diluted 1:1000 for the RV-specific Ig ELISPOT (ABD Serotech, CAT #AHP1360) diluted 1:1000, and placed at 4°C overnight. For the commensal-reactive ELISPOT assay, high binding ELISPOT plates (Multiscreen HTS) were coated with autologous heat killed inactivated Fecal matter (1mg/ml). Plates were blocked the next morning with 10% FBS/RPMI (Sigma) for at least 2 hours while tissues were being prepared. Starting with 1 million cells, single cell suspensions were loaded onto the plate at serial 2-fold dilutions in FBS/RPMI, and left overnight. Cells were removed the next morning and plates were washed with 0.1% TWEEN-20/PBS 3x, leaving the third wash in the plate while rotating on an orbital shaker for 15 min and washed twice more. HRP conjugated IgA or IgG detection antibodies were subsequently added. In some cases, two-color ELISPOTs were done with the simultaneous addition of IgA-HRP and IgG-AP. Plates were washed again as before and developed while covered with aluminum foil for at least 9 minutes or until spots were visible using ImmPACT AEC Peroxidase (for HRP-conjugated Ab) (Vector Laboratories, CAT #SK-4205) or Vector Blue (for AP-conjugated Ab) (Vector Laboratories, CAT #SK-4300) substrates. For two-color ELISPOTS, plates were first developed with AEC, washed with distilled water, rinse once with Tris-HCl pH8.2-8.5 buffer, and developed again with Vector Blue. Plates were dried overnight and spots were counted using a light microscope taking into account the original cell dilution.

### Parabiosis: surgical attachment of two female mice

Sterile technique was used and animals were kept warm with a heating pad throughout the surgery. The entire experiment was conducted according to a published methodology (Rauch et al., 2012). Mice were weight-matched and caged for 1 week together prior to surgery to acclimate. On surgery day, analgesia was administered (meloxicam, 1mg/kg). Mice were anaesthetized with isofluorane (2-3% mixed with oxygen). After shaving the corresponding lateral aspects of each mouse, matching skin incisions were made from the olecranon to the knee joint of each mouse, and the subcutaneous fascia was bluntly dissected to create approximately 1/2 cm of free skin. The latissimus dorsi and abdominal external oblique muscles on each mouse were split. The peritoneal cavities were not penetrated. The olecranon was attached by a single monofilament suture and tie, and the dorsal and ventral skins were approximated by continuous suture. Because the union joins the bones, there was no need to use a flexible cohesive bandage post-surgery. This method gave firm support to both animals, which prevented the strain on the sutures of the skin and abdominal walls. To alleviate pain, buprenorphine (0.1mg/kg, s.c.) was administered every 12 hours for the first 72 hours post procedure. Wounds generally healed within a few days. The parabionts were placed in a cage (1 parabiont pair/cage) for the remainder of the experiment (1 month).

### Abdominal surgery for intestinal photoconversion in Kaede mice

In order to induce photoconversion of cells in the small intestine in Kaede mice, abdominal surgery to expose the intestines was necessary. At 6-8 weeks of age, survival surgery was performed where an incision was made on the midline of the abdomen to carefully expose the small intestines of Kaede or control mice (non-Kaede C57BI/6 mice). Hair was removed from the abdomen with an electric shaver one day prior to surgery. Mice were anesthetized with 2-3% isofluorane and their skin was subsequently sterilized with iodine/alcohol twice each and alternating using sterile gauze. Sterile technique was used throughout the surgery and mice were kept on a heat pad throughout. A drape with a hole in the middle was placed over the mouse and an incision was made into the abdomen, through the hole. A piece of sterile aluminum foil was placed on top of the sterile drape and used to cover the abdomen, in order to protect the surrounding tissue from exposure to the violet light. The small intestine was carefully pulled through the incision on top of the foil and using a handheld LED light, the intestines were exposed to violet light for 2 × 90 seconds for a total of 3 minutes, with the light source approximately 15cm away. These conditions were selected based on pilot experiments. It was noted that if the light source was too close or maintained for too long next to tissue, significant tissue necrosis occurred. Conversely, having the light source too far away did not allow for efficient photoconversion. During photoconversion, warmed, sterile PBS was continuously applied to the intestine with a syringe to keep the tissue hydrated until it was replaced back into the abdomen. The muscle layer and skin layer was closed with 4-0 absorbable interrupted sutures. The mouse was given 5 mg/kg Ketoprofen subcutaneously and 0.1mg/kg Buprenorphine subcutaneously before surgery and a second dose of Buprenorphine at the end of the day. The mice received subsequent equivalent doses of Ketoprofen once per day and Buprenorpine twice per day for one additional day after surgery. An LSR Fortessa flow cytometry machine from BD Bioscience at the University of Toronto Immunology Flow Cytometry Facility was used to analyse tissues from Kaede mice. The machine contained a laser and filter that was able to visualize photoconverted Kaede-Red, which has a similar wavelength to mCherry at 561nm. Unconverted Kaede-Green was visualized using the FITC channel with a wavelength of 488nm. Since the Kaede fluorescent protein is under transcriptional control of actin, Kaede-Red⁺ populations were Kaede-Red⁺ and Kaede-Green^{int}.

### PB/PC transfer

*Prdm1-YFP* SILP cells were sorted based on the expression of YFP separating two populations based on singlets (FSC-W versus FSC-H) followed by a generous lymphocyte gate (FSC-A versus SSC-A), followed by live cell gating (Aqua⁻) followed by elimination of irrelevant cells (Dump⁻: The "dump" gate are cells that stained positive for CD19-APC (1D3), rat anti-mouse CD4-PECy7 (GK1.5), CD8-PECy7, F4/80-PECy7) then sorted as YFP⁺B220⁻ (PB/PC) using a FACS ARIA Sorter machine. Purity of samples was confirmed by post-sort analysis as well as the fact that most of the sorted cells were IgA⁺, Ki67⁻ (see Fig. 7c and Fig. 8). *Prdm1*^{fl/fl} × *Cd*19^{cre} or *Jht*^{*-*/*-*} mice received IV tail injections of B cells or PB/PC at the onset of EAE and 2 more injections spaced 3-4 days apart thereafter and compared with PBS only controls. Depending on the experiment, mice received intravenous injections of 4000-10000 YFP⁺B220⁻ PB/PC at the onset of the disease and 2 more injections every 3-4 days thereafter, until they reached disease peak. More B cells than PB/PC were injected in order to prove the stringency of PB/PC efficacy compared to a separate cell subset from the same anatomical compartment. Mice were scored daily for evidence of clinical disease until the chronic phase of the disease, and then tissues were harvested to confirm presence of YFP⁺ cells by FACS.

### Adoptive transfer EAE in C57BL6 mice

Donor C57BL/6 mice were actively immunized with 100 µg MOG₃₅₋₅₅ peptide emulsified in CFA (Difco). At day 9 after immunization, lymphocytes from the spleen, axillary, inguinal, and cervical lymph nodes were collected and cultured *in vitro* for 72-84 hours in complete T cell media (RPMI1640, 10% fetal bovine serum (Gibco), 100 U/ml penicillin, 100 µg/ml streptomycin, 1X Glutamax (Gibco), 1X MEM non-essential amino acid (Gibco); 1mM sodium pyruvate (Sigma), 10mM HEPES (Lonza), and 50 µM β-mercaptoethanol). For T cell skewing, MOG₃₅₋₅₅ (20 µg/ml), rmlL-6 (20 ng/ml, Peprotech), rmlL-12p70 (3 ng/ml, Peprotech), rmlL-23 (20 ng/ml, R&D), and human TGFb (4 ng/ml, Peprotech) were added to the media. 40 million cells from cultured lymphocytes were transferred intraperitoneally to C57BL/6 or BAFF-Tg recipient mice. At day 2 post-transfer, recipients also received 200ng of pertussis toxin.

### Transmigration assay

The transmigration assays were performed using a modified Boyden chamber system adapted from previously published manuscripts (Cayrol et al., 2008; Ifergan et al., 2006; Lecuyer et al., 2017). Briefly, primary cultures of endothelial cells were detached and plated on Boyden chambers inserts in appropriate media as previously described (Lecuyer et al., 2017) and per manufacturer's instructions (Cell Biologics). When monolayers were formed after 72 hours in culture, culture media was changed for DMEM media supplemented with 20% FBS, and 1.10⁶ total mononuclear cells isolated from the gut of *Prdm1^{YFP}* mice or T_{H}17 lymphocytes were added to the upper compartment of the Boyden chambers. T_{H}17 lymphocytes served as positive control and were polarized in presence of anti-CD3 (10ug/mL), anti-CD28 (2ug/mL), anti-IFNy (20ug/mL), rmlL-6 (20ng/mL), TGFβ (4ng/mL) and rmlL-23 (20ng/mL), from ex vivo CD4⁺ T lymphocytes sorted (MACS - Miltenyi Biotec) from splenocytes of 8-10-week-old C57BL/6 mice. Transmigration was assessed by conducting flow cytometry analysis on cells harvested from upper and lower compartments of the Boyden chambers at 18-20hr post-migration. Activation of endothelial cells was performed as previously described (Lecuyer et al., 2017).

### Statistical analysis

Statistical analyses were performed using Graphpad Prism software 7 (GraphPad Software Inc.). The variability of distribution was assessed by Shapiro-Wilk normality test. Student's T-test was used for data with a normal distribution. The Mann-Whitney U test was used for non-Gaussian distributed data and 2-way ANOVA test was used to evaluate differences in clinical disease over time between groups. All tests were performed using 2-tailed analysis. Log-rank (Mantel-Cox test) was performed for evaluating differences in survival over time. Significance cutoff was set at p<0.05 at a 95% confidence level. Most graphs provide mean values with SD unless stated differently.

### EXAMPLE 3: IgA-producing cells are detected in the CNS during EAE

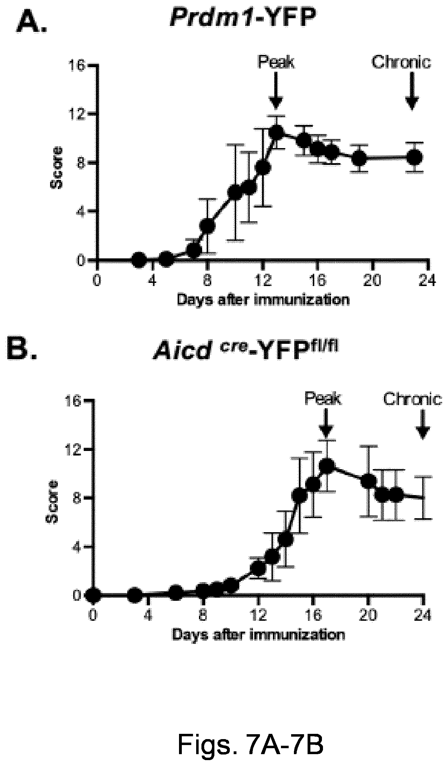

Anti-CD20 treatment of MS patients revealed that B cells play a role in MS that is independent of their production of antibodies (Piccio et al., 2010). In contrast, TACI-Ig, which impacts not only B cells but also serum antibody levels, worsened RRMS (Kappos et al., 2014) and also promoted the development of MS in optic neuritis patients (Sergott et al., 2015). The sharp contrast of these clinical trial results prompted a reassessment of the role of antibody-secreting cells during neuroinflammation. To do this, the MOG₃₅₋₅₅ experimental autoimmune encephalomyelitis (EAE) animal model was utilized for kinetic, phenotypic and functional studies (unless indicated otherwise, this is the EAE model used throughout). A kinetic analysis of PB/PC accumulation in the CNS during EAE has not been fully assessed using a reporter mouse system, a crucial tool given that PB/PC downregulate B cell lineage markers (Pracht et al., 2017). Since B cell differentiation into PB/PC is driven by the *Prdm1* gene and the subsequent up-regulation of Blimp1 protein (Minnich et al., 2016), EAE was induced in *Prdm1*^{yfp} mice to unambiguously monitor the presence of PB/PC in the brain (Br) and spinal cord (Sc) at different stages of disease (Fig. 7a). To corroborate these findings, EAE was also induced in a fate-map mouse that allows tracking of B cells that have been activated by antigen and thus have expressed activation induced cytidine deaminase (*Aicd*^{yfp} mice), an enzyme that is required for Ab class switching and hypermutation (Muramatsu et al., 2000) (Fig. 7b). After gating on YFP⁺ cells, expression of CD138 (a PB/PC marker), B220 (B220 is relatively down-regulated on PC compared to PB where it is expressed variably), and Ki67 (PC are typically non-proliferative Ki67⁻ whereas PB actively proliferate) was measured. A representative gating strategy for PB/PC derived from the BM from the *Prdm1*^{yfp} reporter mouse is shown in Fig. 7c. Of note, although CD138 is expressed to varying degrees on PB and PC, it was found that CD138 was dynamically modulated on BM-resident PB/PC during the course of disease (Fig. 7d). Due to the variable nature of B220/CD138 expression on Ab-producing B lineage cells, the PB/PC designator is used to describe Blimp⁺ B cells throughout this disclosure.

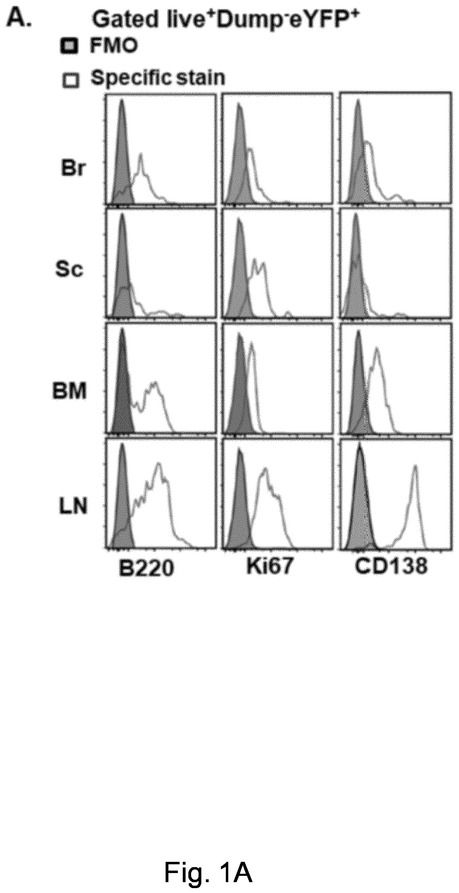

Focusing first on the chronic stage of disease, it was observed that *Prdm1*-YFP⁺ cells in the Br and Sc exhibited low expression of B220, were Ki67^{dim}, and were CD138^{low}. In comparison, *Prdm1*-YFP⁺ cells in the BM expressed variable levels of B220, were also Ki67^{dim}, and were CD138⁺. *Prdm1*-YFP⁺ cells in the draining axillary lymph nodes expressed the highest levels of B220, were Ki67⁺ and CD138^{high} (Fig. 1a), similar to what has been previously described in the spleen (Pracht et al., 2017).

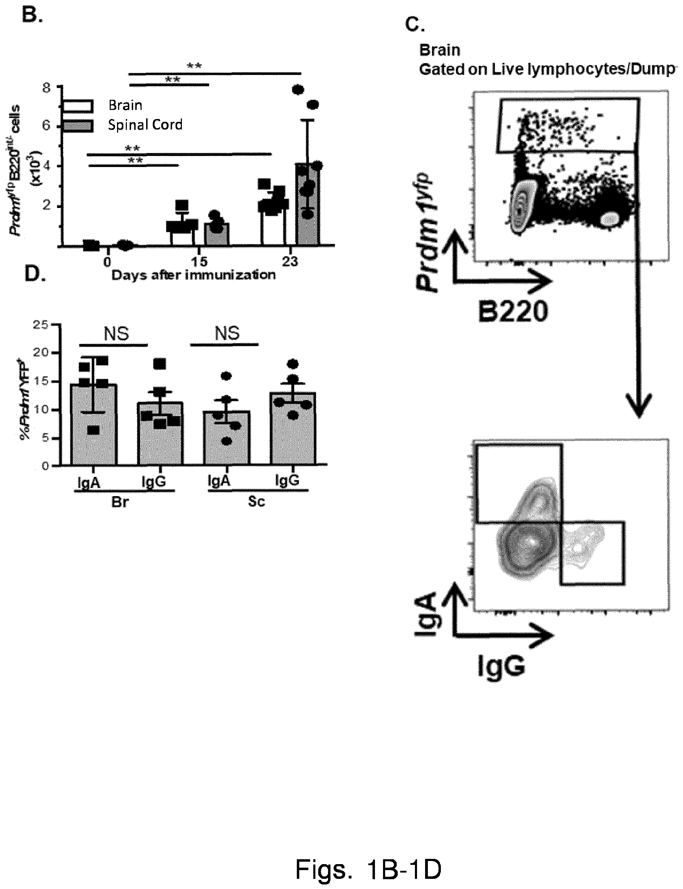

Next, PB/PC were enumerated in the Br and Sc at different time points of EAE. A significant increase in absolute numbers of *Prdm1*-YFP⁺B220^{int/-} cells was observed in both the Br and Sc at the peak of disease (approximately D15 after immunization) and an even greater increase was observed during the chronic phase of the disease (approximately D23 after immunization) compared with D0 where PB/PC could not be detected (Fig. 1b). Since immunization with MOG results in T cell-dependent production of class-switched antibodies (Ichikawa et al., 1999; Mitsdoerffer et al., 2010), to characterize the immunoglobulin isotype of CNS-resident *Prdm1*-YFP⁻B220^{int/-} cells, intracellular dual flow cytometry staining for IgG and IgA was performed at the chronic stage of EAE, where the highest number of *Prdm1*-YFP⁺B220^{int/-}cells in the CNS was observed (representative example in Fig. 1c). While the majority of *Prdm1-*YFP⁺B220^{int/-} cells were not class switched (IgG⁻IgA⁻double negative cells), IgG class switched cells could be detected in the CNS. Surprisingly, a significant portion of *Prdm1*-YFP⁺B220^{int/-} cells stained positive for intracellular IgA (Fig. 1d) and confirmatory results were obtained using *Aicd*^{cre}YFP^{fl/fl} mice (Fig. 7e-f).

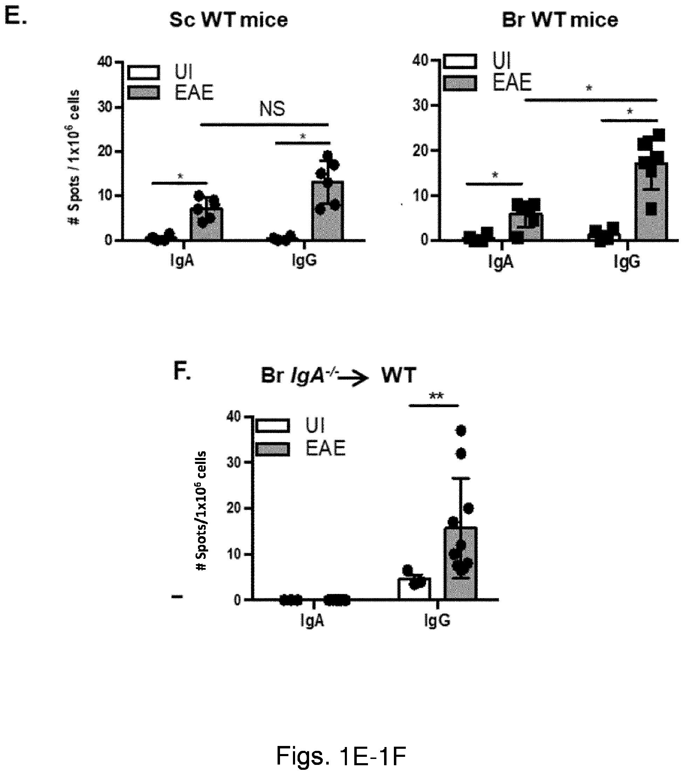

To validate the presence of class-switched PB/PC in the CNS during EAE, separate experiments to measure the frequency of IgG and IgA ASC in the CNS were performed by ELISPOT. Consistent with the flow cytometric findings, significant increases in frequencies of both IgG and IgA ASC in the CNS were observed during the chronic phase of EAE compared with unimmunized mice. Unlike the FACS results, it was found that there were higher numbers of IgG ASC in the Br (but not the Sc) compared to IgA using this readout (Fig. 1e). However, it is important to note that in contrast to the *ex vivo* flow cytometric approach for detecting intracellular production of IgG and IgA, the ELISPOT approach incorporates a 24 hour *in vitro* culture period prior to detection, which may skew results towards PB/PC that have the best survival capacity *in vitro.* Nevertheless, both the flow cytometry and ELISPOT assays show evidence of IgA producing cells in the CNS.

Since IgA is predominantly produced at mucosal surfaces, the presence of IgA⁺ PB/PC in the CNS was surprising. To confirm this result, potential artefactual detection of IgA⁺ ASC in the ELISPOT assay was ruled out by examining IgA ASC in the CNS of Jht^{-/-} chimeric mice that received a transplant of IgA^{-/-} BM. Accordingly, while IgG⁺ ASC were detected in the brain of chimeric IgA^{-/-} EAE mice during EAE, IgA⁺ ASC were undetectable (Fig. 1f), thus confirming the specificity of the assay.

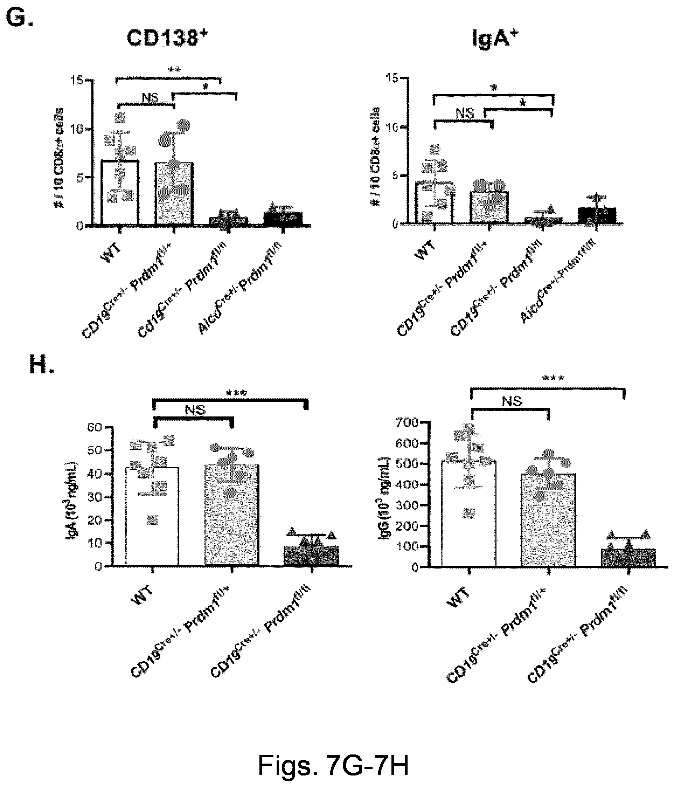

Given that PB/PC are detected in the CNS during EAE, it was next determined whether these cells impact the clinical course of the disease. To do this, PB/PC-less mice were generated by crossing *Cd19*^{cre} or *Aicd*^{cre} lines with *Prdm1*^{fl/fl} mice. The *Cd19*^{cre} line deletes *Prdm1* expression in all B cells, whereas the *Aicd*^{cre} line deletes *Prdm1* only in B cells that have encountered antigen. A reduction of PB/PC in *Cd19^{cre}Prdm1*^{fl/fl} and *Aicd*^{cre}*Prdm1*^{fl/fl} mice was confirmed by quantifying PC by immunofluorescence (Fig. 7g) and by measuring serum Ig levels by ELISA (Fig. 7h). It was found that PB/PC-less mice exhibited exacerbated EAE compared with littermate controls, irrespective of whether the *Cd19*^{cre} or *Aicd*^{cre} was used to delete *Prdm1* (Fig. 7i-j). Moreover, PB/PC-less mice exhibited higher mortality than WT mice (Table II). Thus, the absence of PB/PC results in an increase in the severity of EAE.

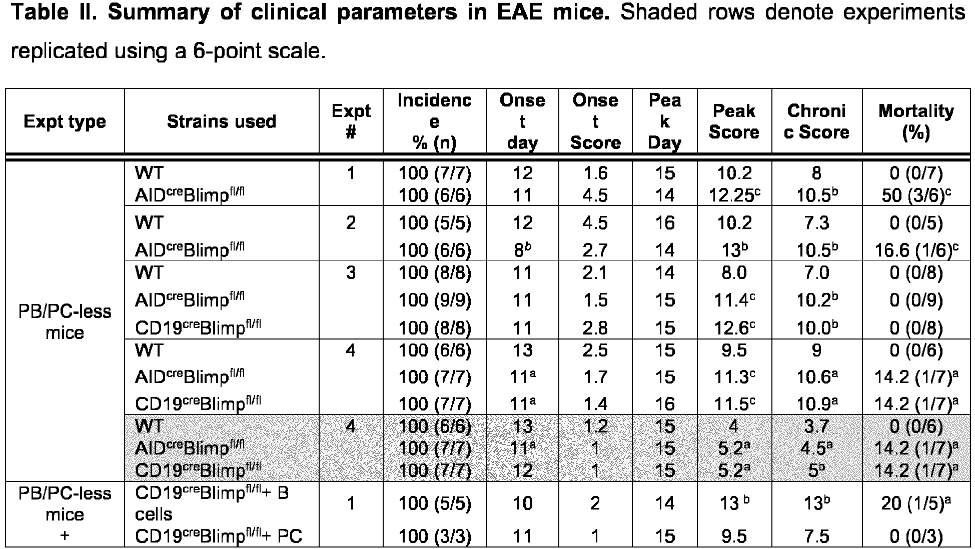

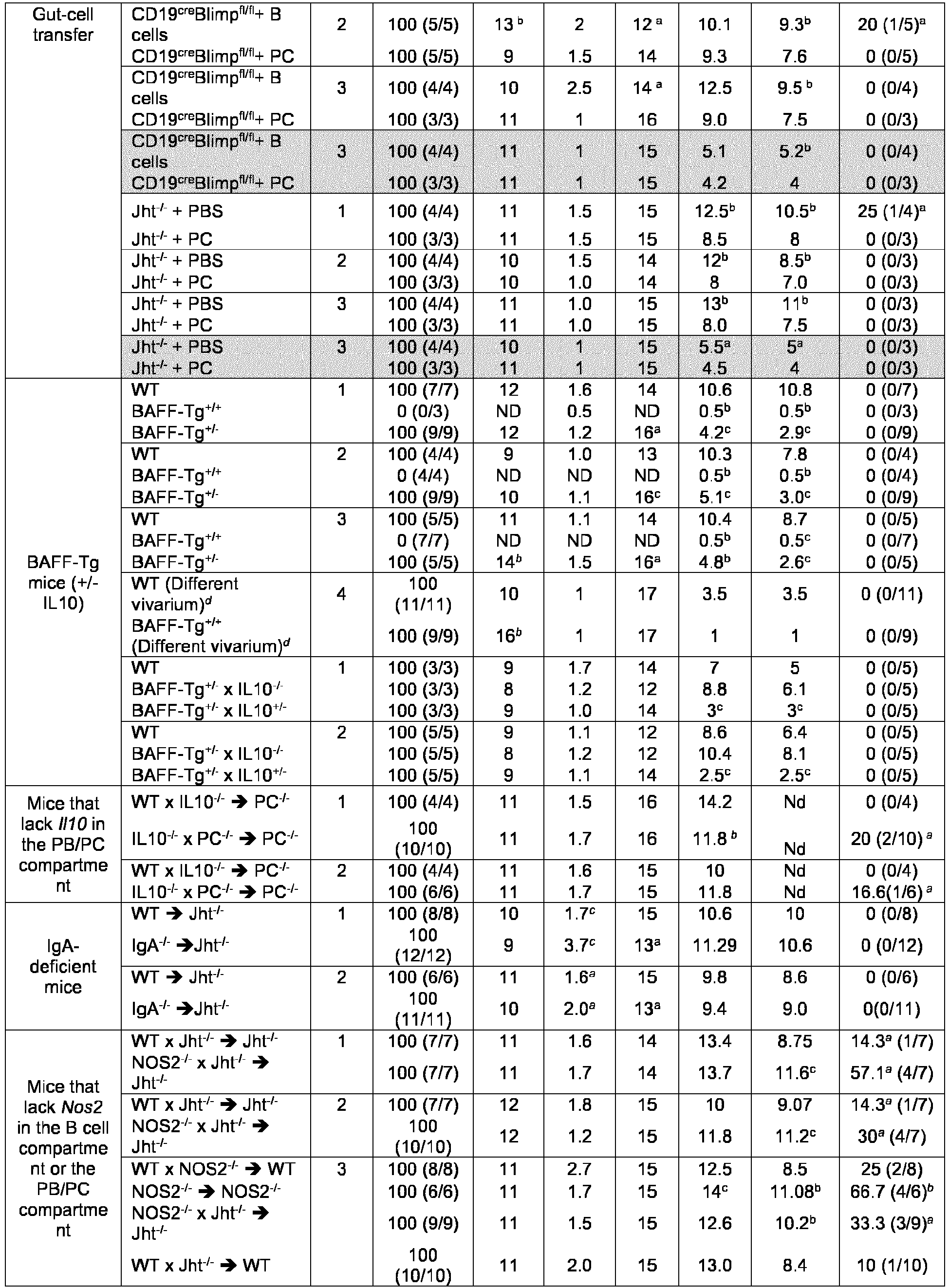

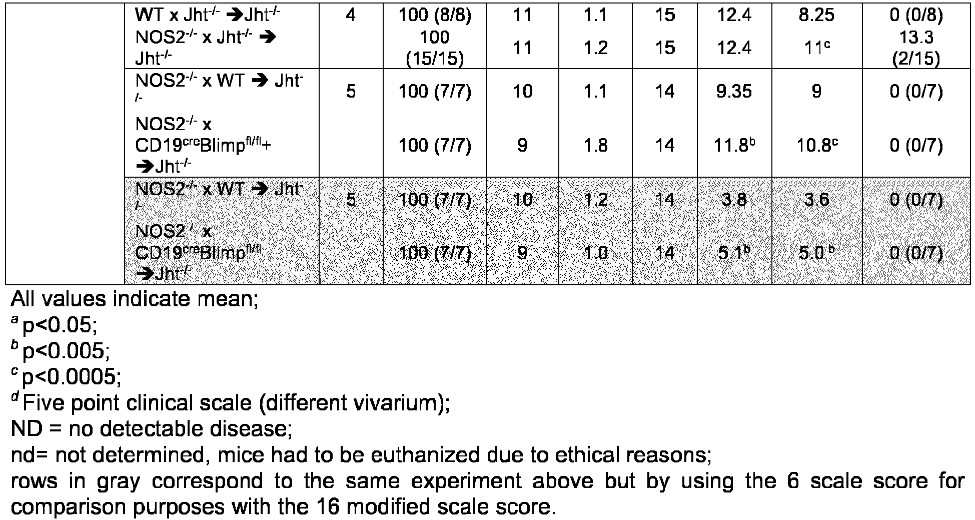

In summary, while CD138^{low}Ki67^{low}YFP⁺B220^{int/-} PB/PC are not observed in the CNS during the steady state, PB/PC that produce IgG and IgA accumulate in the Br and Sc during EAE, and the absence of PB/PC results in increased EAE severity.

### EXAMPLE 4: PB/PC are diminished in the gut during EAE, and transfer of gut-derived Blimp⁺ cells to PB/PC-less mice attenuates disease

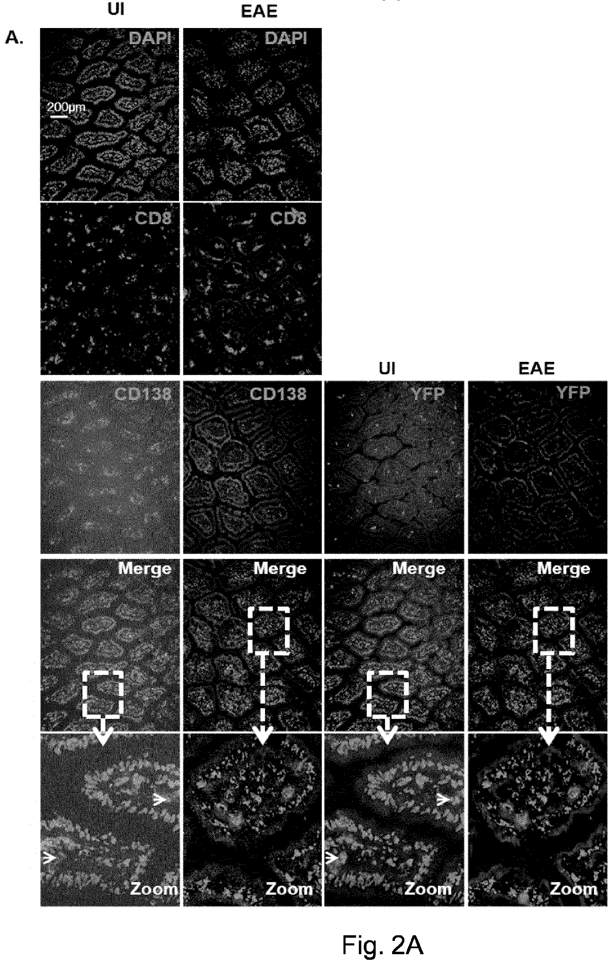

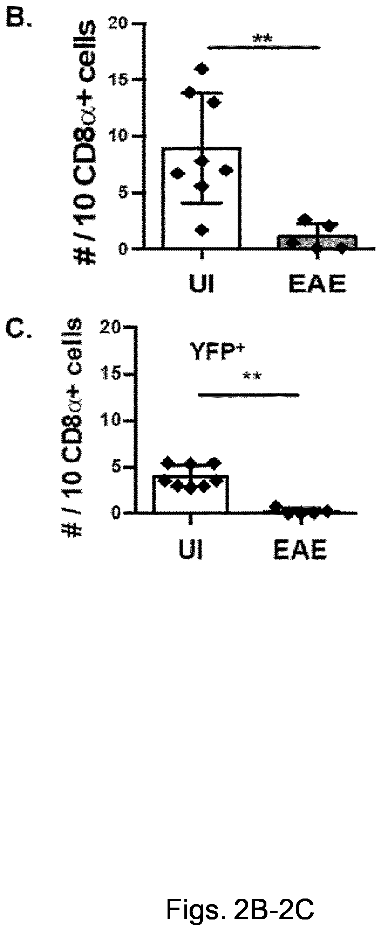

It has been shown that IgA⁺ PC exhibit immunosuppressive functions in both prostate and liver tumor microenvironments (Shalapour et al., 2015; Shalapour et al., 2017). Since IgA⁺ PB/PC were observed in the inflamed CNS and PB/PC-less mice exhibit exacerbated EAE, IgA⁺ PB/PC may be specifically responsible for suppressing neuroinflammation. The ensuing experiments focused on IgA⁺ PB/PC derived from the gut, since this is the largest reservoir of IgA-producing cells in the body. A significant decrease in the number of CD138⁺*Aicd*-YFP⁺ PB/PC in the SILP was observed during the chronic phase of EAE, indicating that the homeostasis or localization of gut-resident PB/PC is altered during EAE (Fig. 2a-c). Moreover, this reduction in YFP⁺ PB/PC in the SILP was accompanied by visible gaps between DAPI⁺ epithelial cells (Fig. 2a), indicating that the gut may be damaged during EAE.

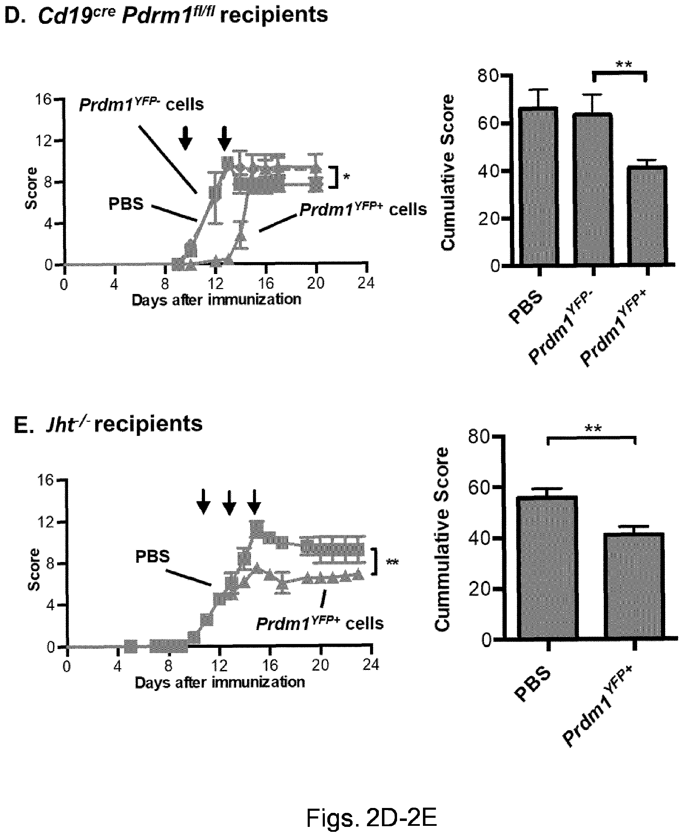

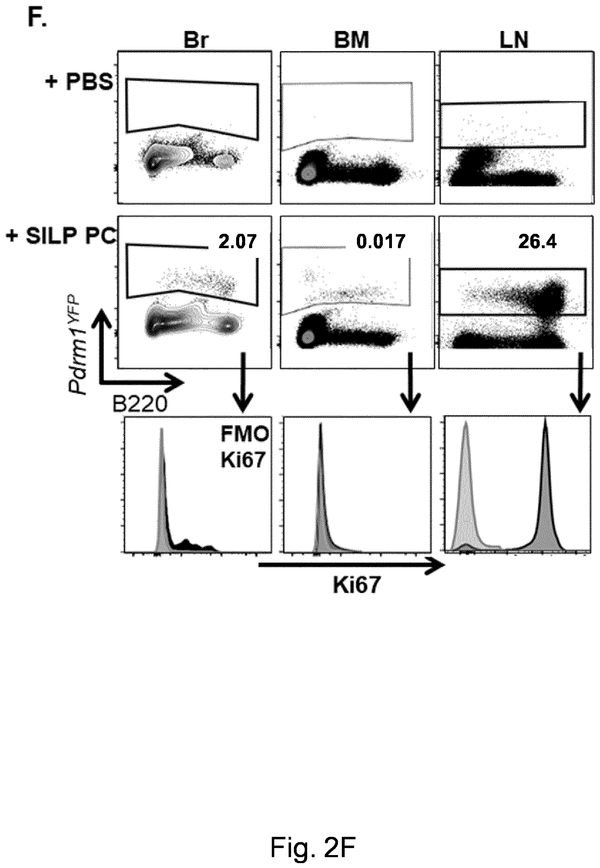

To test whether gut IgA⁺ PB/PC suppress neuroinflammation, adoptive transfers of SILP-derived B lineage cells into PB/PC-less EAE mice were performed. Post-sort analyses confirmed that the transferred PB/PC were IgA⁺, Ki67⁻ and B220⁻, whereas transferred B cells were B220⁺CD5⁻ (Fig. 8). Comparing with PBS treatment, a delay in disease onset and reduced EAE severity were observed when SILP-derived *Prdm1*-YFP⁺B220⁻ PC were transferred into PB/PC-less mice. However, SILP-derived *Prdm1*-YFP⁻B220⁺ B cells had no effect on EAE (Fig. 2d, Table II). Similar results were also observed upon transfer of *Prdm1*-YFP⁺B220⁻ cells into B-cell deficient (*Jht*^{*-*/*-*}) EAE mice (Fig. 2e). At the termination of the experiment (chronic phase of the disease) the tissues were examined for the presence of the adoptively transferred SILP-derived *Prdm1*-YFP⁺B220⁻ cells. *Prdm1*-YFP⁺ cells were detected in the Br, BM and the LN of recipient mice (Fig. 2f), confirming that the transferred cells could reach these tissues. In summary, the SILP contains PB/PC that, when adoptively transferred, can reach the CNS and are sufficient to reduce the clinical symptoms of EAE.

### EXAMPLE 5: IgA⁺ B cells from the SILP recirculate to distal tissues

Since a decrease was detected in IgA⁺ B cells in the gut concomitant with an increase in IgA-producing cells in the CNS during EAE, and because the transfer of gut-resident PB/PC attenuates the clinical presentation of EAE, the capacity of gut-resident IgA⁺ B cells to recirculate to other tissues was further assessed in a non-transfer scenario. To this end, an ELISPOT assay that enables monitoring of IgA ASC specific for a gut-encountered pathogen was devised. For this assay, Rotavirus (RV) was selected as the pathogen since it specifically infects mature enterocytes in the small intestine and provokes a robust local IgA response (Franco and Greenberg, 1999).

Using this approach, it was first tested whether RV-specific IgA ASC can be recovered in other mucosal tissues outside of the gut, such as the lungs. For example, in the context of Influenza infection, the gut becomes highly damaged in the absence of detectable flu virus in the gut, and T cells primed in the lung-draining LN migrate to the SILP of flu infected mice (Wang et al., 2014). The reverse (i.e. IgA ASC leaving the gut) has never been assessed.

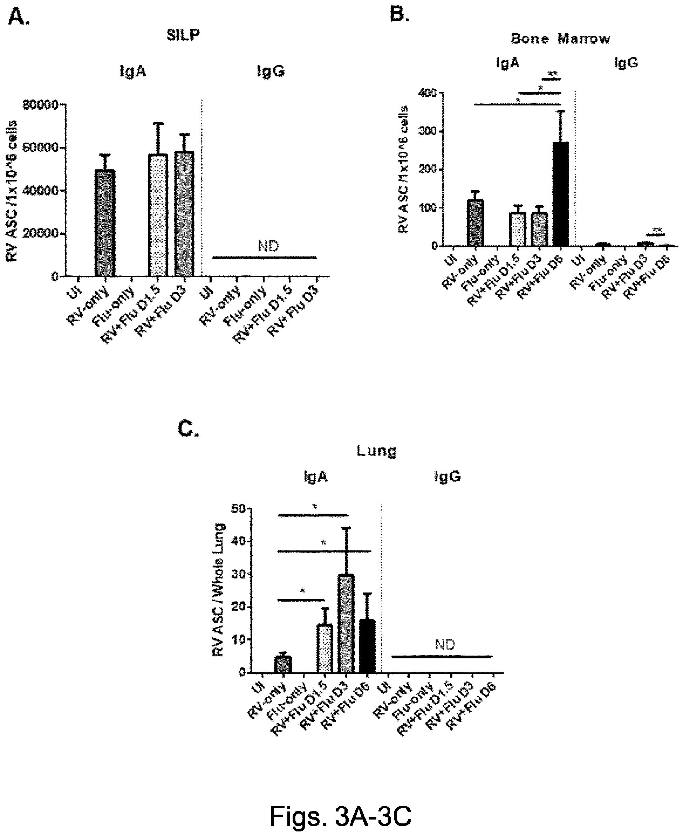

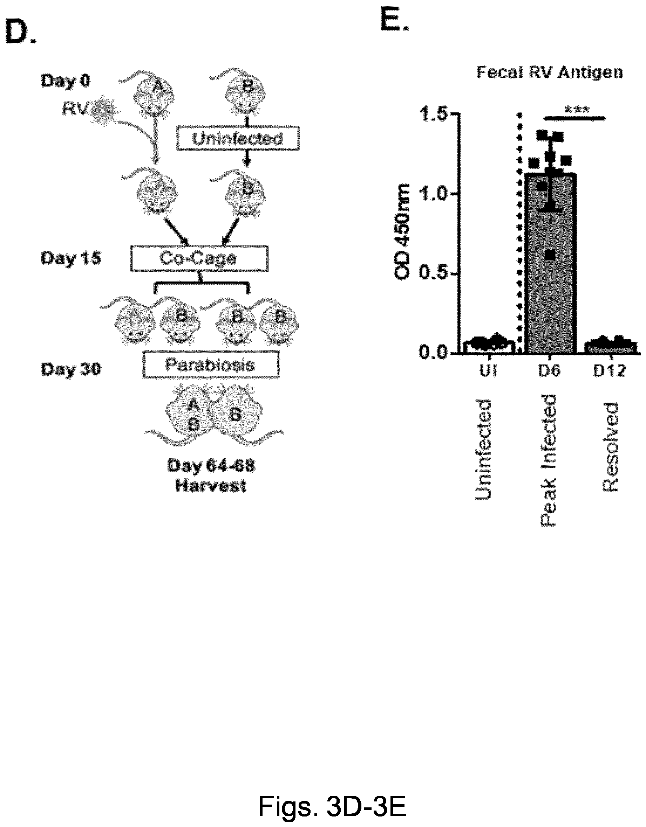

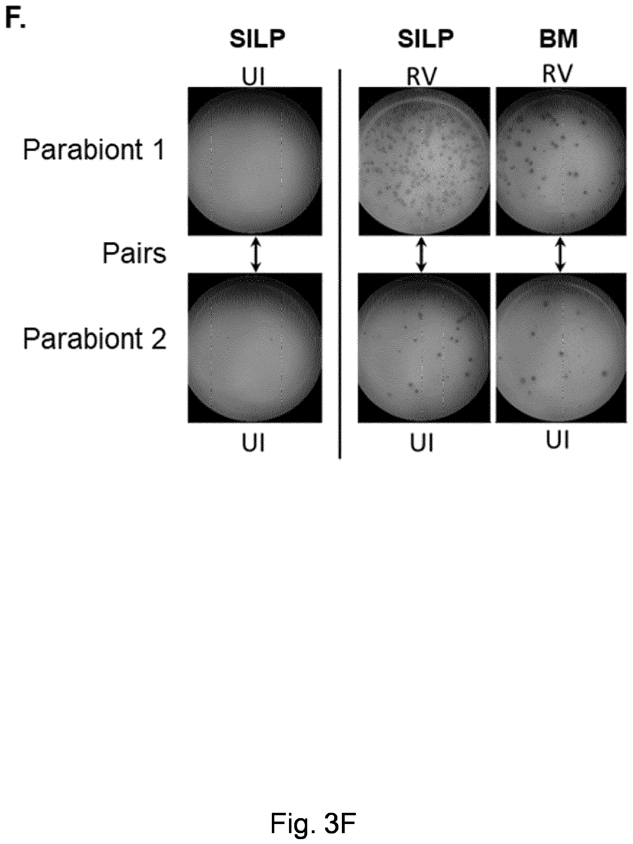

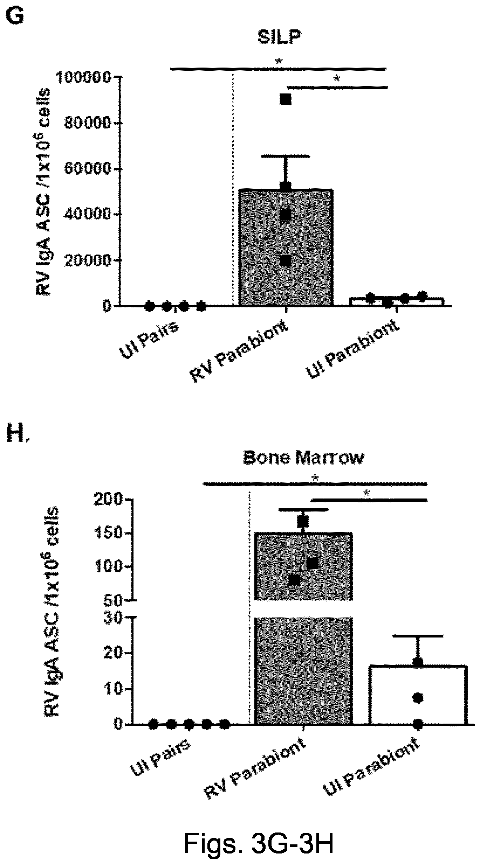

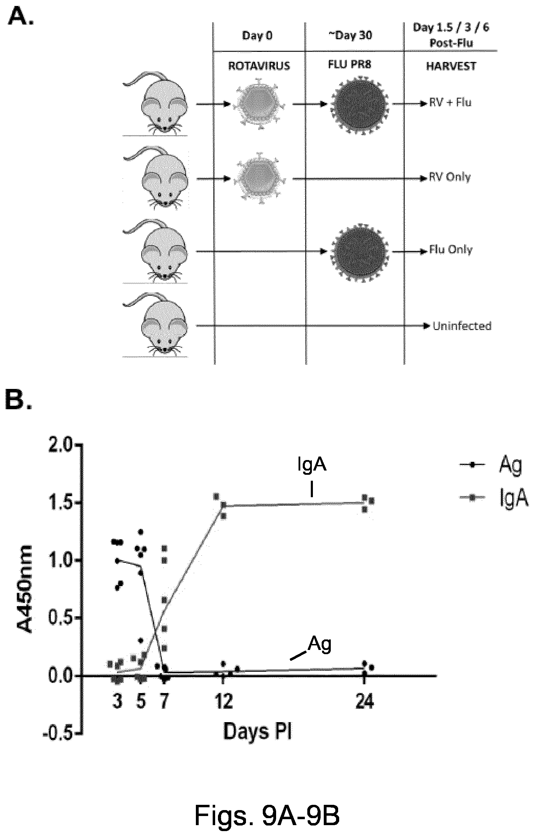

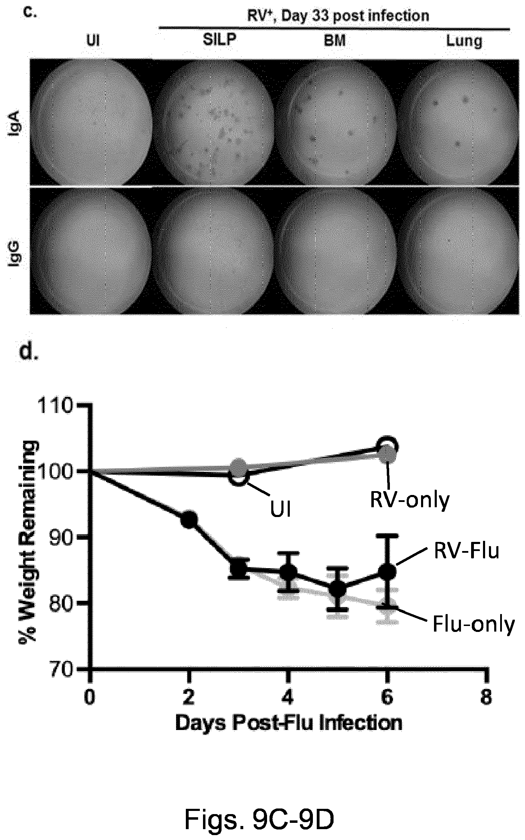

Since the lungs are also a "pit-stop" for immune cells entering the CNS (Steinert et al., 2015), it was tested whether they could also be a site that harbors gut-derived B cells. Accordingly, a dual infection approach was used whereby mice were first orally infected with RV (primary infection), followed 30 days later by intra-nasal infection with Influenza (secondary infection). Using this system, the question of whether ASC specific for orally administered RV could migrate to the lungs was tested. The following four groups of mice were evaluated: RV and Flu naïve (uninfected, "Ul"); primary infection alone ("RV-only"); secondary infection alone ("Flu-only") and primary plus secondary infection ("RV+Flu") - see schematic (Fig. 9a). It was found that RV was cleared from the gut by day 7 post-infection (p.i), and the RV-specific IgA response peaked at day 12 p.i. (Fig. 9b). RV-specific IgA ASC were found in both the SILP and the BM at 30 days p.i. (Fig. 9c). In "Ul" or "Flu-only" mice, no evidence of RV-specific ASC was found in any of the tissues examined (see Fig. 3).

To examine the migration patterns of RV-primed IgA ASC, the frequency of RV-specific IgA ASC in the SILP of "RV-only" and "RV+Flu" mice was first determined. Of note, no differences in weight loss were observed when comparing Flu infected mice that had or had not been previously infected 30 days earlier with RV (Fig. 9d). RV-specific IgA ASC were detected at high frequencies in the gut post-flu infection in the "RV-only" and "RV+Flu" mice (Fig. 3a). Due to the highly damaged state of the gut at the peak of influenza infection (D6 post-flu infection) only limited numbers of cells could be isolated from the gut at that time point, thus D6 for the gut was not included in the analysis. RV-specific IgG ASC were not detected in the gut at any time point, indicating that the RV response in the SILP is strongly polarized towards generating anti-RV ASC of the IgA isotype.

Next, the frequency of RV-specific IgA ASC in the BM of "RV-only" and "RV+Flu" mice was determined. The BM is a known reservoir for long-lived PC due to the presence of PC growth factors such as BAFF, IL-6 and APRIL (Chu and Berek, 2013). Indeed, a high frequency of RV-specific IgA ASC was observed in the BM of "RV-only" and "RV+Flu" mice. RV-specific IgG ASC were also observed in the BM, but these cells were present at low levels compared to RV-specific IgA ASC (Fig. 3b).

Lastly, the frequency of RV-specific IgA ASC in an extra-intestinal mucosal site (the lungs) of "RV-only" and "RV+Flu" mice was determined. IgA ASC were detected in the lungs of "RV+Flu" mice as early as D1.5 post-flu infection, and in the lungs from D1.5, D3 and D6 "RV+Flu" mice exhibited a significantly elevated frequency of RV-specific IgA ASC compared to lungs from "RV-only" mice (Fig. 3c). This shows that inflammation in the lungs may attract RV-specific IgA ASC to migrate from the gut to the lungs. The RV-specific IgA ASC that were recovered from the lungs of flu infected mice are unlikely to be B cells cross-reactive for both RV and Flu antigens since a low frequency of RV-specific IgA ASC was observed in the lungs of "RV-only" mice (Fig. 3c), arguing against the possibility of cross-reactivity and indicating that there may be low-level homeostatic movement of RV-specific IgA ASCs to the lungs in the steady state. This observation was confirmed with *an in vitro* Boyden chamber system whereby lamina propria *Prdm1-*YFP⁺B220⁻ PC harvested from *Prdm1*^{yfp} mice were found to migrate across lung endothelial cells *in vitro* regardless of their activation status (Fig. 9e).

In summary, these results demonstrate that IgA ASC previously generated in response to an intestinal pathogen 30 days prior can be rapidly recruited to the lungs.

### EXAMPLE 6: RV-specific IgA ASC can access the circulatory system and populate non-mucosal peripheral tissues, including the CNS

Since a low frequency of RV-specific IgA⁺ ASC was noted in the lungs at steady-state in "RV-only" mice, it was next determined if IgA⁺ B cells could access the circulation in resting mice. To test this hypothesis, a parabiosis technique was used whereby mice previously infected with RV (Group A) are surgically attached to uninfected mice (Group B - Fig. 3d). Since Group A was allowed to clear the infection prior to surgery as confirmed by ELISA (Fig. 3e), this effectively separated RV priming events in one mouse from migration events in a second mouse. At approximately 35 days post-surgery, SILP and BM were collected for RV-specific ASC detection by ELISPOT (Fig. 3f). Analysis of paired animals revealed that RV-specific IgA ASC were present in the gut and BM of both the RV infected and RV-naïve parabionts (Fig. 3g-h). Although there were fewer RV-specific IgA ASC in the gut and BM of Group B mice compared to their Group A partners, these data nevertheless demonstrate that RV-specific IgA ASC can access the circulation in the steady state.

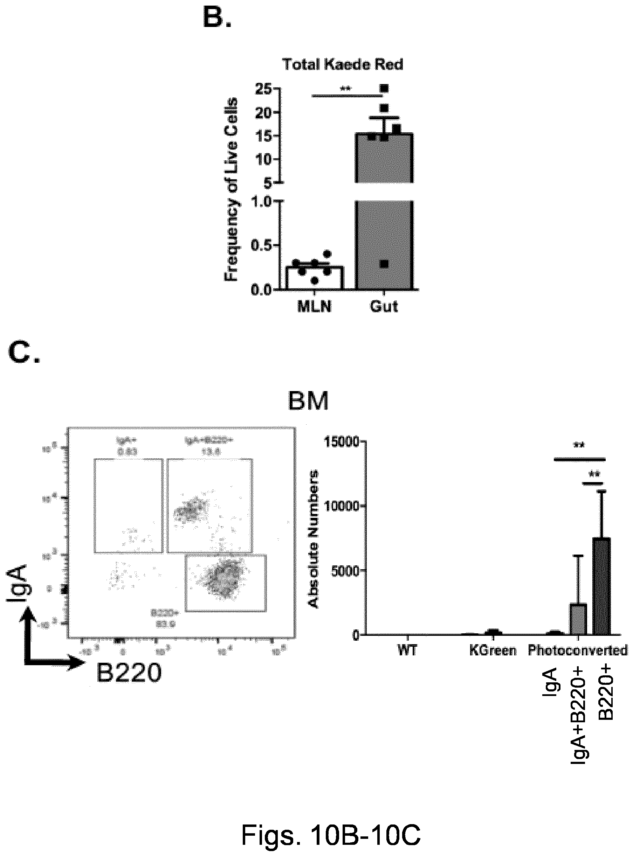

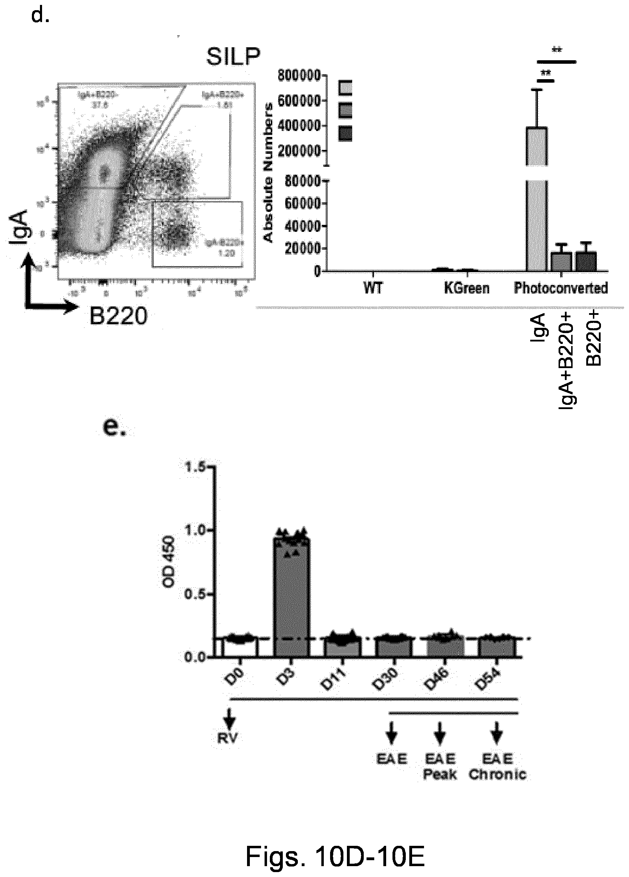

An alternative experimental approach of tissue-directed photo-conversion was used to confirm the above findings. Specifically, upon exposure to violet light, cells expressing a Kaede transgene are irreversibly converted from green to red. Using a transgenic mouse model in which all cell types express the Kaede fluorescent protein (Tomura et al., 2008), the length of the small intestine was photoconverted by surgically exposing it to violet light. Three days following surgery, flow cytometry was used to measure the number of Kaede-Red⁺ cells at the site of photoconversion (SILP) as well as in the BM, an extra-intestinal tissue (see representative gating strategy in Fig. 10a). While significant photoconversion of SILP-resident cells was observed (18% Kaede-Red⁺), Kaede-Red⁺ cells were undetectable in controls (Kaede mice subjected to sham surgery and non-transgenic WT mice). Moreover, photoconversion of mesenteric LN cells was minimal (0.2%, see Fig. 10b) demonstrating that photoconversion was specific to the small intestine. Next, the number of Kaede-Red⁺ cells in the BM (Fig. 10c) and the SILP (Fig. 10d) was determined. Of the Kaede-Red⁺ cells in the SILP, IgA⁺B220⁺, IgA⁺B220⁻, and IgA⁻B220⁺ cells were observed. Among these populations, the majority were IgA⁺B220⁻ and thus presumably PB/PC. Importantly, IgA⁻B220⁺ and IgA⁺B220⁺ Kaede-Red⁺ B cells were also observed in the BM, demonstrating that IgA⁺ B cells photoconverted in the gut can be recovered from the BM in the steady state.

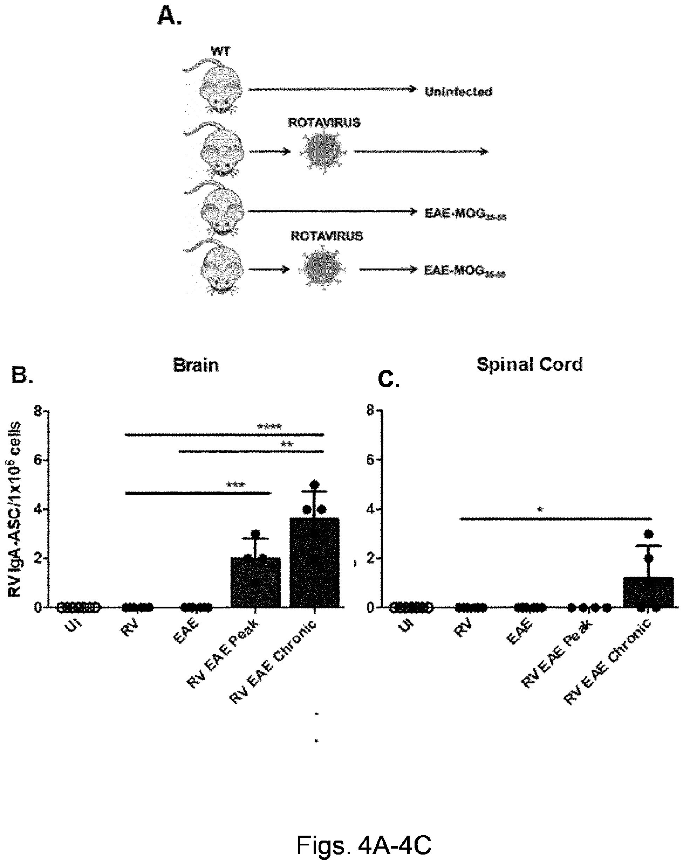

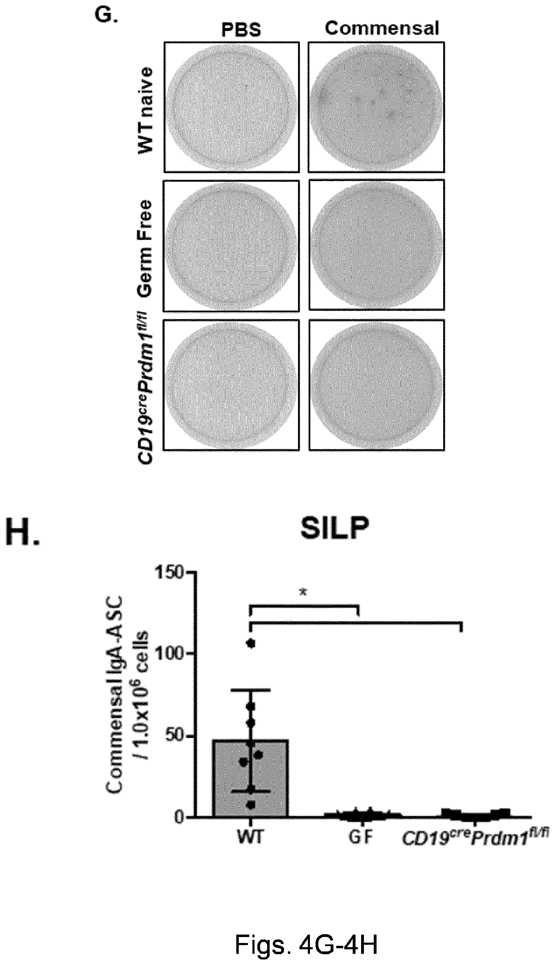

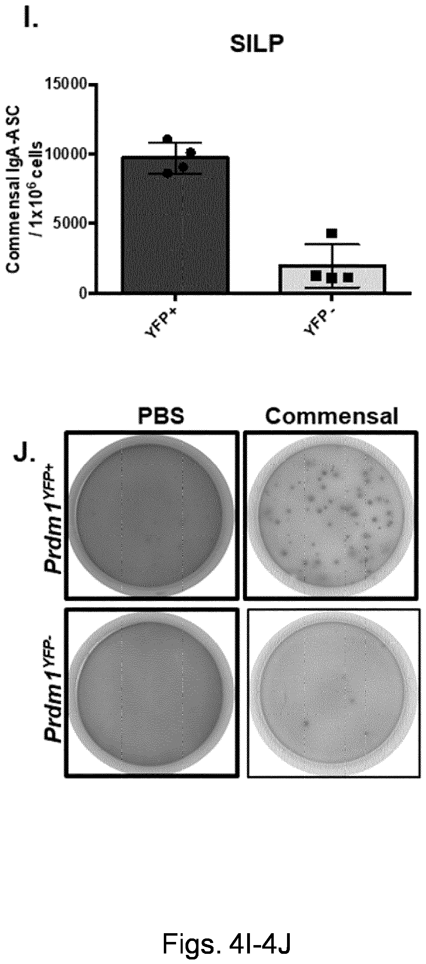

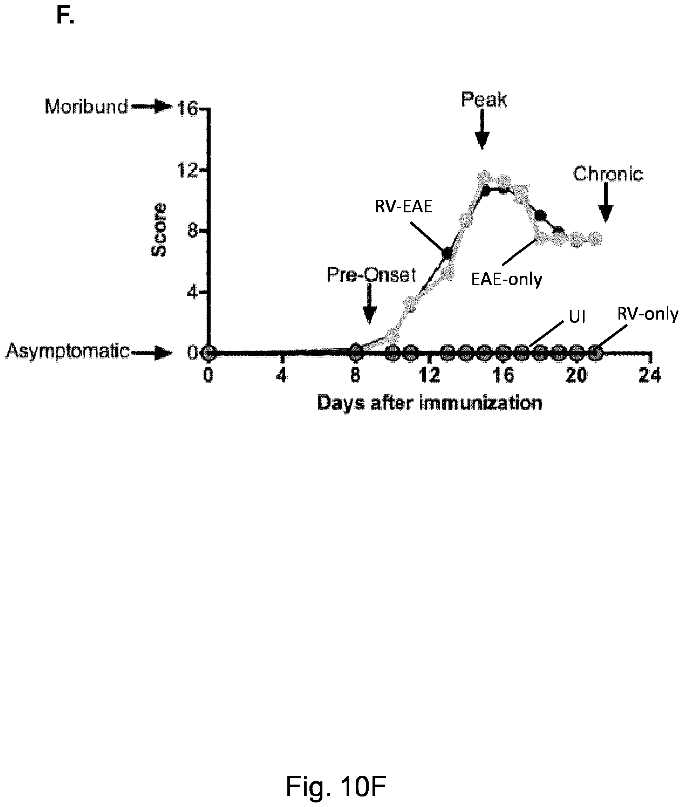

Lastly, to relate these findings back to EAE, it was determined whether B cells primed in the GALT could be recovered in the CNS. The Kaede photo-conversion approach could not be used because the dual effects of abdominal surgery and EAE induction would have been prohibitively stressful on the mice. As an alternative approach, the CNS was examined for presence of RV-specific IgA ASC using a similar sequential challenge model where RV-infected mice underwent MOG₃₅₋₅₅-induced EAE (see schematic - Fig. 4a). The clearance of RV prior to EAE (Fig. 10e) was validated. It was also confirmed that *a priori* infection with RV does not impact the severity of EAE (Fig. 10f).

Using this approach, it was found that RV-specific IgA ASC were absent from the CNS in the steady state (Fig. 4b). However, during peak and chronic EAE, RV-specific IgA ASC were observed in the Br (Fig. 4b). RV-specific IgA ASC were also observed in the Sc at the chronic phase of the disease (Fig. 4c). This observation was confirmed *in vitro* with a Boyden chamber system. Specifically, although lamina propria *Prdm1*-YFP⁺B220⁻ PC harvested from *Prdm1*^{yfp} mice were not found to migrate across resting brain endothelial cells, *Prdm1*-YFP⁺B220⁻ PC migrated across brain endothelial cells that had been previously activated with a combination of TNFα and IFNy (Fig. 9e).

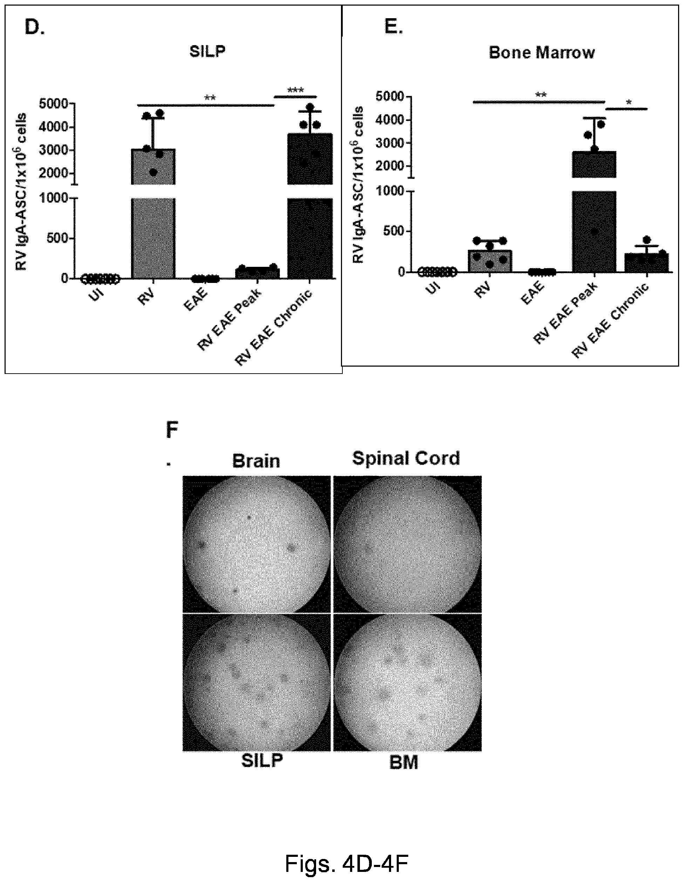

In the SILP, while frequencies of RV-specific IgA ASC were detectable in all RV infected mice regardless of EAE status, a significant reduction in RV-specific IgA ASC was observed at the peak of disease in "RV+EAE" mice (Fig. 4d). In the BM, IgA ASC were observed at the peak stage of EAE, but this was followed by a significant drop in RV-specific IgA ASC at the chronic phase of EAE in "RV+EAE" mice (Fig. 4e). Representative images of RV-ELISPOTs derived from "RV+EAE" mice at the chronic stage of EAE are shown in Fig. 4f.

Taken together, gut-derived RV-specific IgA⁺ B cells can access the circulatory system and home to the BM in the steady state as well as to the CNS during EAE.

### EXAMPLE 7: Commensal-reactive IgA ASC populate the CNS during EAE

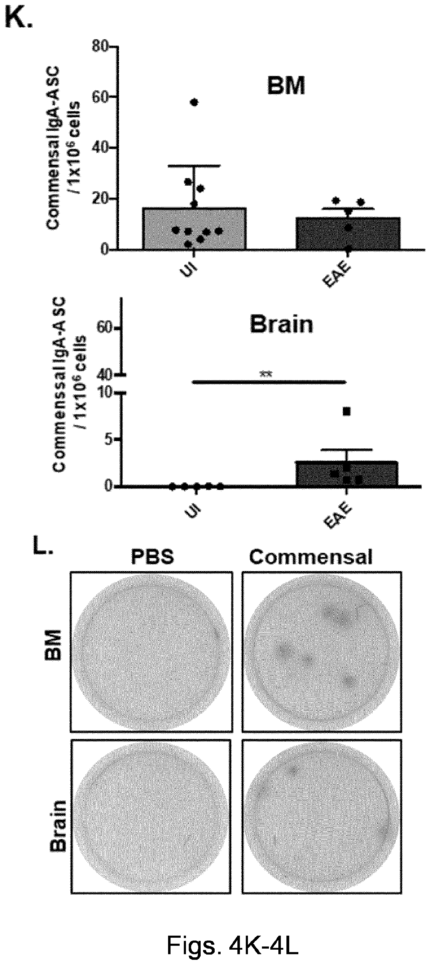

The RV-specific IgA response is largely (but not exclusively) T cell-dependent (Franco and Greenberg, 1997). To ascertain if the findings are generalizable to gut-intrinsic commensal microbes, responses to which are largely T-independent (Bunker et al., 2015), a "commensal ELISPOT" assay was developed by coating plates with heat-killed autologous fecal matter. Single cell suspensions from different tissues were subsequently added to the plates and commensal-reactive IgA were then visualized with anti-lgA detection. Application of single cell suspensions from the SILP of WT mice to commensal-coated plates (but not PBS-coated plates) resulted in the detection of commensal-reactive IgA ASC, as expected. However, if single cell suspensions were derived from the SILP of germ-free mice or from PB/PC-less *Cd19*^{cre}*Prdm1*^{fl/fl} mice, no spots were observed, confirming specificity of the assay (Fig. 4g-h). Moreover, pre-sorting *Prdm1-*YFP⁺B220⁻ cells from the SILP of *Prdm1*^{yfp} reporter mice resulted in a significant enrichment of commensal-reactive IgA ASC in the ELISPOT assay, whereas minimal IgA ASC were observed in in ELISPOT wells with *Prdm1-*YFP⁻B220⁺ cells, indicating that the majority of commensal-reactive IgA ASC detected in this assay are PB/PC (Fig. 4i-j). Next it was determined if commensal-reactive IgA ASC can be detected in the BM and the CNS like RV-specific IgA-producing cells. Indeed, commensal-reactive IgA+ PB/PC were detected in the BM. In the Br, commensal-specific IgA ASC were also detected during the chronic phase of EAE, but not at steady state (Fig. 4k-l). In summary, commensal-reactive IgA PB/PC can be found in extra-intestinal sites such as the BM and the inflamed Br.

### EXAMPLE 8: A commensal microbe that induces an IgA response attenuates EAE

Given that EAE is associated with the presence of gut-derived IgA+ PB/PC in the CNS, and that transfer of gut-derived IgA+ PB/PC attenuates EAE, commensal microbes that increase systemic levels of IgA+ PB/PC should reduce the severity of EAE. To test this, an already established microbiota was supplemented with a singular commensal microbe, *Tritrichomonas musculis* (*T.mu*)*,* that has been shown to elevate systemic IgA levels (Chudnovskiy et al., 2016). *T.mu* naive (*T.mu-*) and *T.mu* colonized (*T.mu+*) cohorts of mice were generated, and EAE was induced by immunization with MOG₃₅₋₅₅ three weeks after colonization. *T.mu*+ mice harboured on average 140 x 10⁶ +/- 44.9 x 10⁶ trophozoites per caecum.

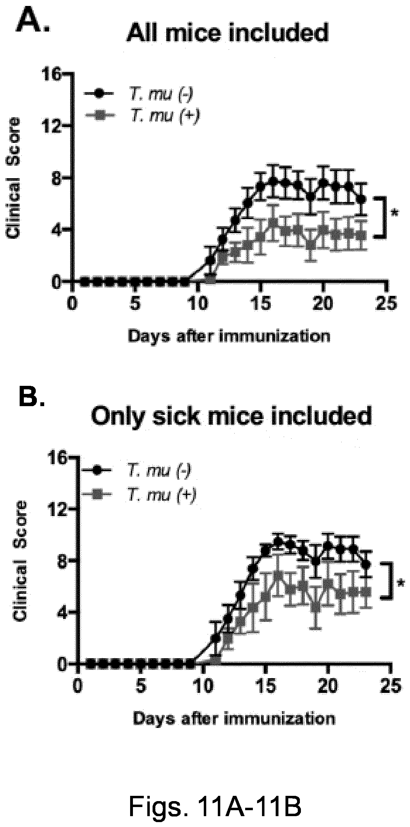

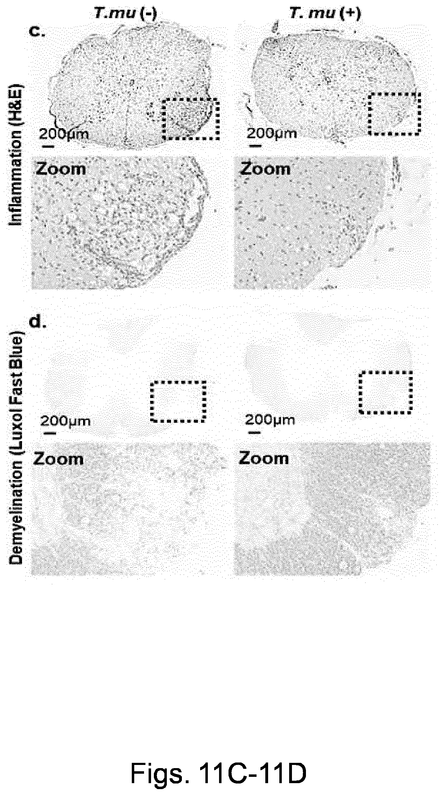

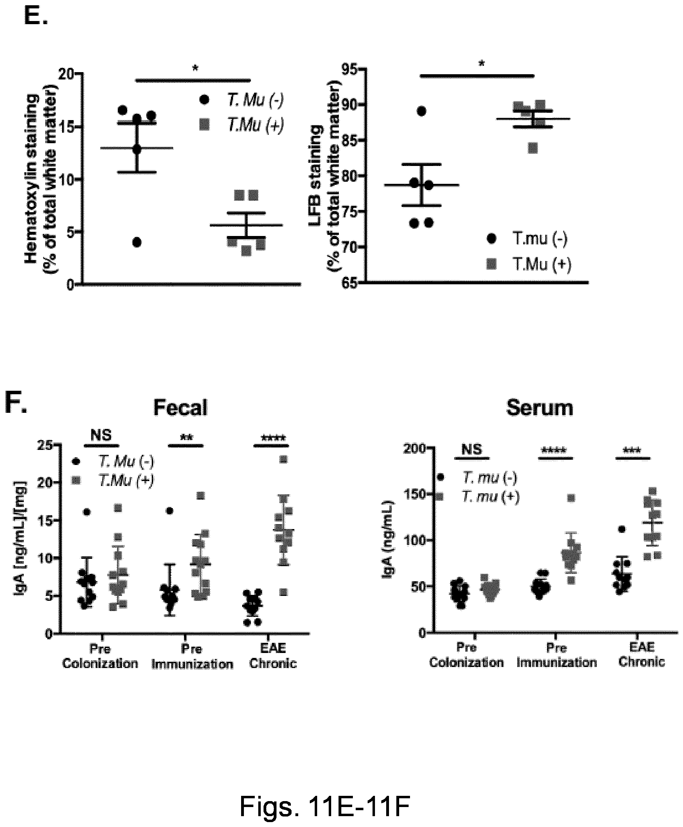

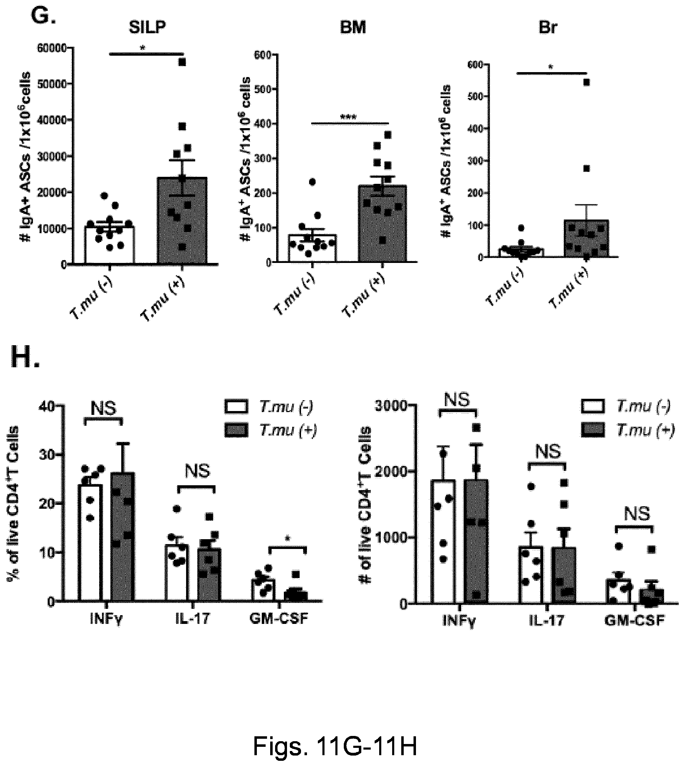

Compared to *T.mu-* mice, *T.mu*+ colonized mice exhibited less severe EAE with lower incidence of disease (Fig. 11a-b), as well as reduced inflammation and demyelination in the spinal cord (Fig. 11c-e). Reduced EAE correlated with elevated serum and fecal IgA (Fig. 11f) as well as an increase in the frequency of IgA ASC in the gut, bone marrow and brain (Fig. 11g). Although the frequency of CNS-resident Th17 and Th1 cells was unaltered by colonization of T.mu, the frequency of GM-CSF producing CD4+ T cells in the CNS was significantly reduced (Fig. 11h). Thus, although *T.mu* has been shown to elevate Th1 and Th17 responses in the gut (Chudnovskiy et al., 2016), colonization with *T.mu* protects against severe EAE concomitant with an elevation of IgA ASC in the brain.

### EXAMPLE 9: Assessment of PC-derived factors involved in EAE suppression

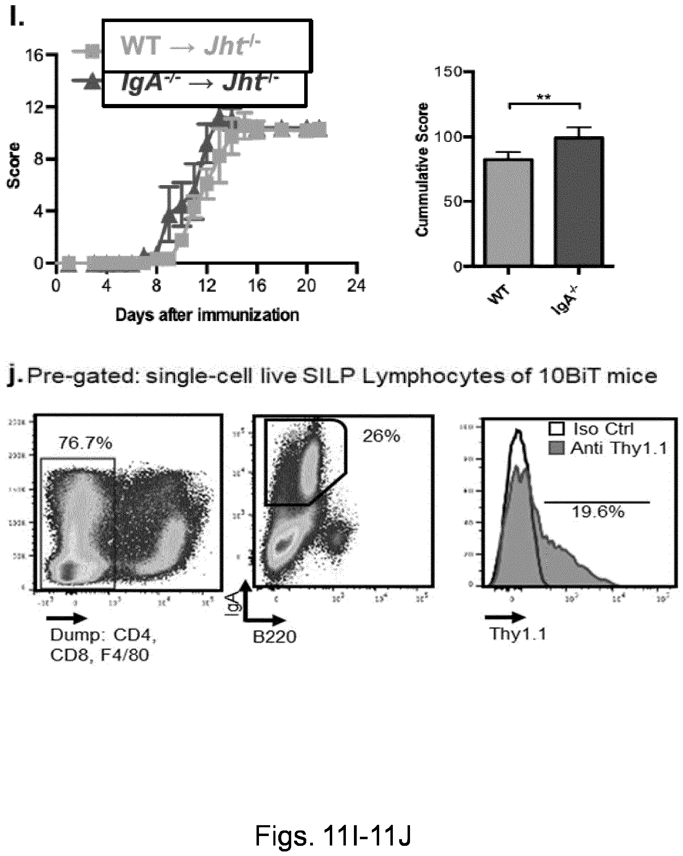

The accumulation of RV-specific and commensal-reactive IgA ASC in the brain implies that IgA⁺ PB/PC can migrate to an inflamed tissue irrespective of their B cell receptor. Moreover, since colonization of mice with *T.mu* increases the numbers of IgA ASC in the CNS concomitant with a decrease in EAE, IgA antibodies may play a role in dampening disease. However, it was found that irradiated B cell deficient *Jht*^{*-*/*-*}mice reconstituted with IgA^{-/-} BM exhibited roughly similar EAE severity as co-housed *Jhf*^{-/-}mice that received WT BM, although a slight acceleration in onset in the IgA^{-/-} recipient group resulted in a modest increase in cumulative score (Fig. 11i). These experiments indicate that IgA itself likely does not play a primary role in dictating the severity of EAE.

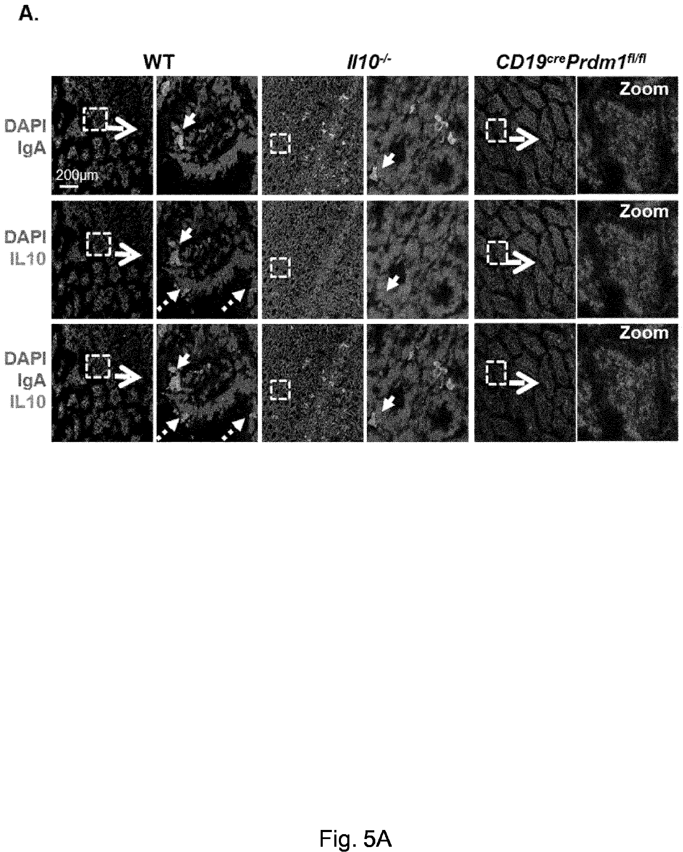

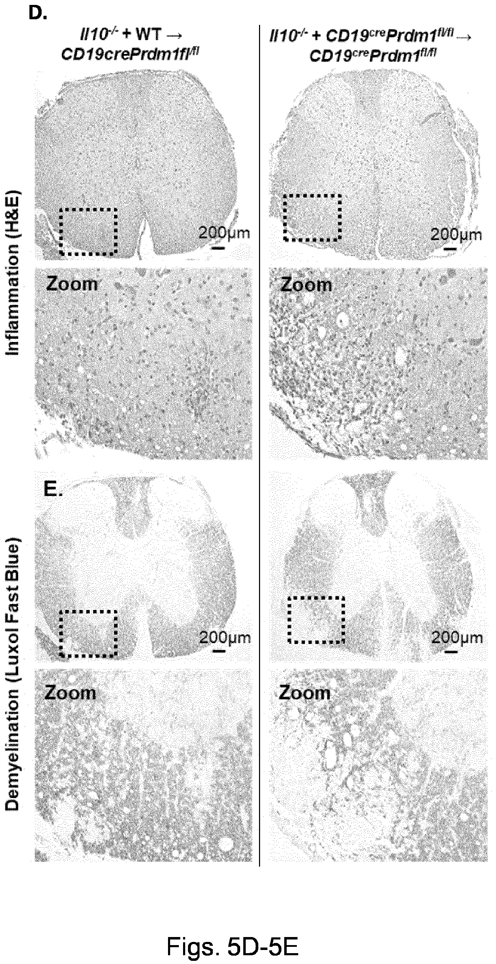

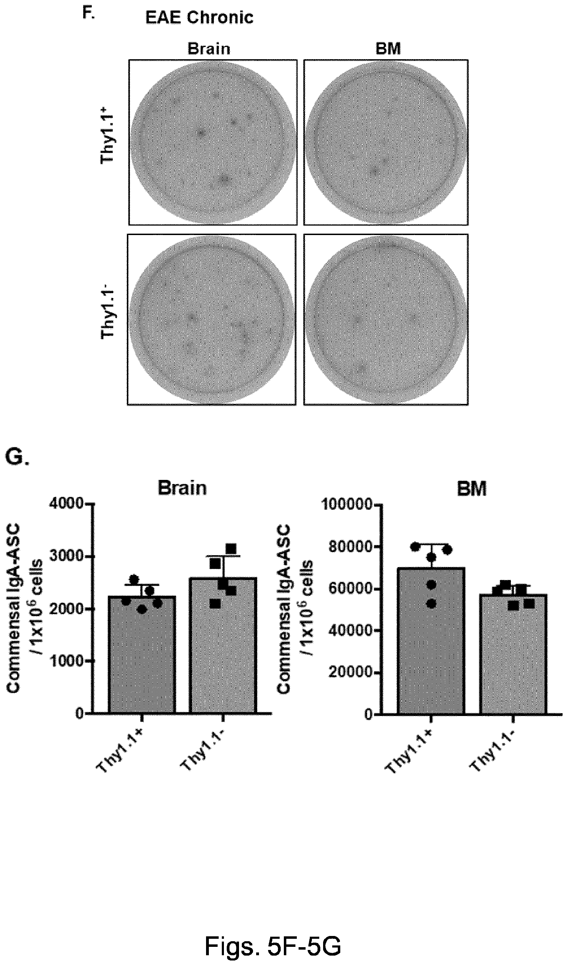

In attenuating EAE, PB/PC may directly or indirectly suppress ongoing inflammation in the CNS. IL10 has a critical role in attenuating EAE (Matsumoto et al., 2014; Shen et al., 2014), and accordingly, an experiment was carried out to test whether gut-resident IgA⁺ cells were a significant source of IL10. It was found that IgA⁺ B cells in the SILP showed evidence of IL10 immunofluorescence staining that was not present in IL10^{-/-} mice. Moreover, IL10 staining was greatly reduced in PB/PC-less mice, demonstrating that the majority of IL10 in the SILP at steady-state is derived from IgA⁺ B cells (Fig. 5a).

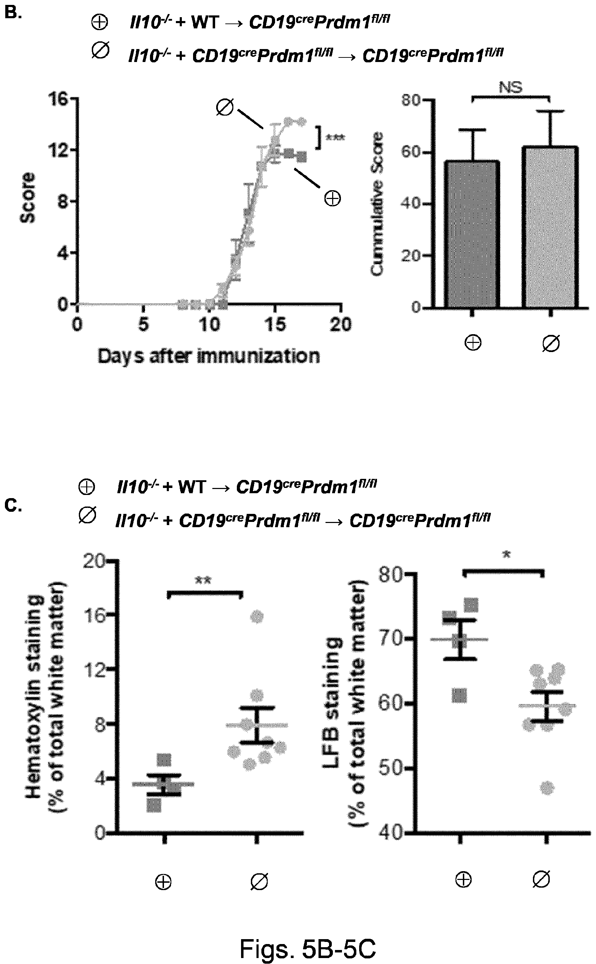

Next, it was assessed whether PB/PC-intrinsic IL10 was required for suppression of EAE. This was done by reconstituting irradiated *Cd19*^{cre}*Prdm1*^{fl/fl} recipient mice with an 80/20 mixture of *Cd19*^{cre}*Prdm1*^{fl/fl} + *Il10*^{-/-} BM in order to generate mice whereby PB/PC cannot produce IL10 but other hematopoietic cells retain this capacity. Compared with control chimeras, mixed chimeric mice that harbour IL10^{-/-} PB/PC exhibited exacerbated EAE (Fig. 5b) with some mice requiring humane euthanization compared to control mixed BM chimeric mice (Table II). Due to the severity of disease, the experiment could not be carried out through to the chronic stage of EAE, and consequently, cumulative scores were not significantly different. The clinical findings were confirmed by quantifying immune cell infiltration and demyelination in the spinal cord. Mixed chimeras with PB/PC that are unable to produce IL10 exhibited significantly increased immune infiltration (Fig. 5c-d) and demyelination (Fig. 5c, e).

In order to analyze cells that are actively transcribing *Il10*, a reporter mouse was used that expresses a transgene consisting of the coding sequence of Thy1.1 inserted into a mouse *Il10* gene contained in a bacterial artificial chromosome (10BiT mice). Flow cytometric analysis of *Il10* mRNA expression as reported by surface Thy1.1 protein showed that IgA⁺B220⁻ cells in the SILP express IL10, confirming the immunofluorescence findings (Fig. 11j). Next, all cells expressing *Il10* were isolated by sorting Thy1.1⁺ versus Thy1.1⁻ cells from the Br and the BM during the chronic phase of EAE. Sorted cells were then plated in commensal ELISPOT plates and developed with anti-lgA. It was found that approximately 50% of commensal-reactive IgA ASC in the BM and the Br were derived from the Thy1.1+ fraction (Fig. 5f-g). Note the frequency of spots reported in the brain in 10BiT mice were higher than in the brain in WT mice in Fig. 4l because in the case of the 10BiT mice, a pre-sort step was integrated (Thy1.1+ or Thy1.1-fractions were derived from Dump- cells).

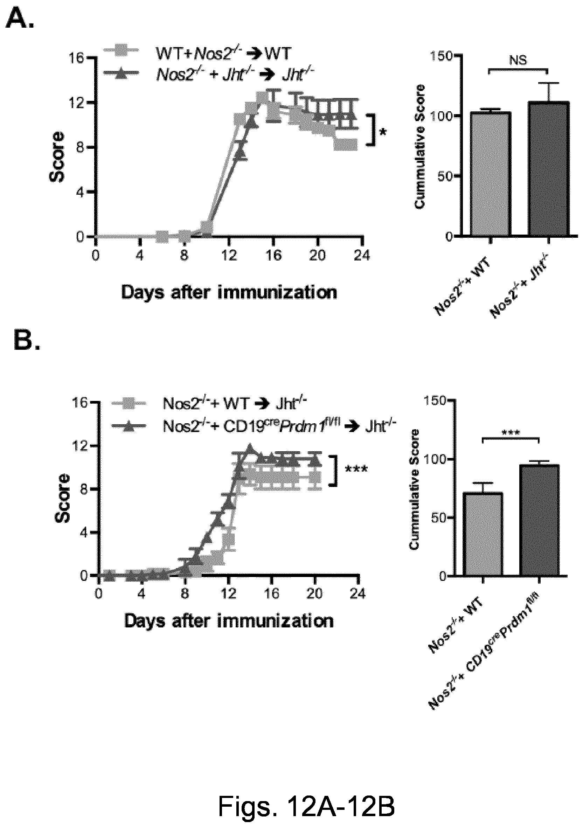

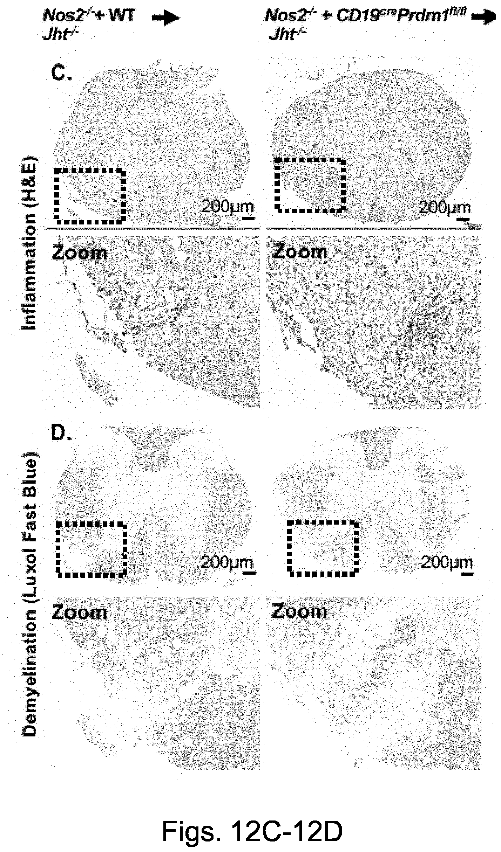

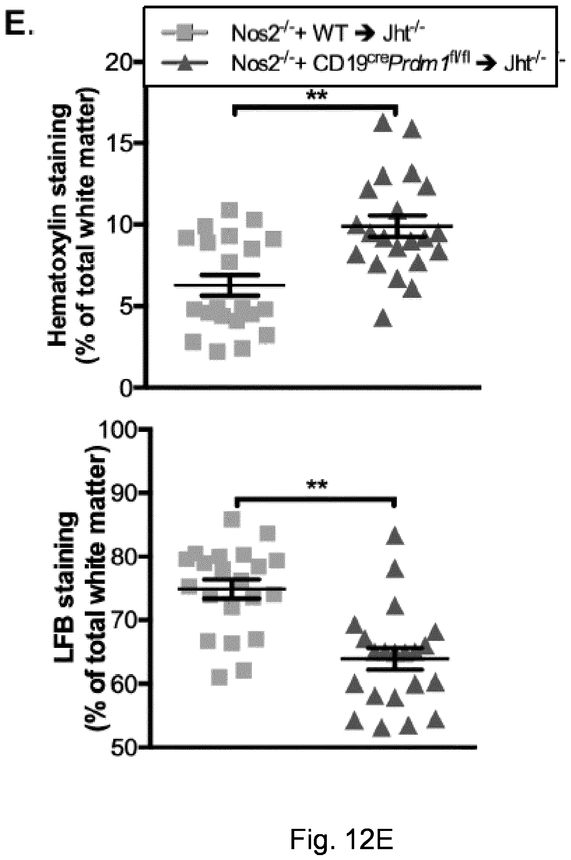

PB/PC express other cytokines and immunomodulators beyond IL10, including IL35 and iNOS. This indicates that PB/PC may employ a variety of mechanisms to attenuate EAE. Since the absence of iNOS results in exacerbated EAE (Fenyk-Melody et al., 1998), the possibility of a PB/PC-intrinsic role for iNOS in suppressing EAE was assessed. To test this, mixed BM chimeras were constructed in a similar manner as for *Il10*^{-/-} mice. Compared to control chimeric mice, deletion of *Nos2* in B cells (Fig. 12a) or specifically in PB/PC (Fig. 12b), resulted in modestly exacerbated EAE and higher mortality (phenocopying *Nos2*^{-/-} → *Nos2*^{*-*/*-*} BM chimeras, see Table II), although exacerbation of EAE in mice lacking PB/PC-intrinsic *Nos2* expression was not as dramatic as what was observed in the absence of *Il10.* The exacerbated EAE in mice lacking PB/PC-intrinsic *Nos2* expression was accompanied by enhanced spinal cord inflammation and demyelination (Fig. 12c-e). Collectively, the data show that PB/PC-intrinsic IL10 and iNOS play primary and secondary roles respectively in suppressing EAE.

In summary, while IgA itself is not sufficient to suppress EAE, intestinal IgA⁺ PC express IL10 and the expression of IL10 by PB/PC (and less so iNOS) is required to reduce the symptoms of EAE. Moreover, commensal-reactive IgA⁺ ASC actively expressing *Il10* can be recovered from the BM and the Br during EAE.

### EXAMPLE 10: BAFF-transgenic mice are highly resistant to the effector phase of EAE

Mice that overexpress the B cell survival factor BAFF (*Baff*-*Tg* mice) have a massively expanded pool of IgA⁺ PC in the SILP accompanied by commensal-reactive IgA in the serum (McCarthy et al., 2011). Since it was found that SILP-derived *Prdm1*-YFP⁺B220⁻ cells could dampen EAE, the following experiments were performed to determine whether BAFF-Tg mice exhibit an altered response to EAE.

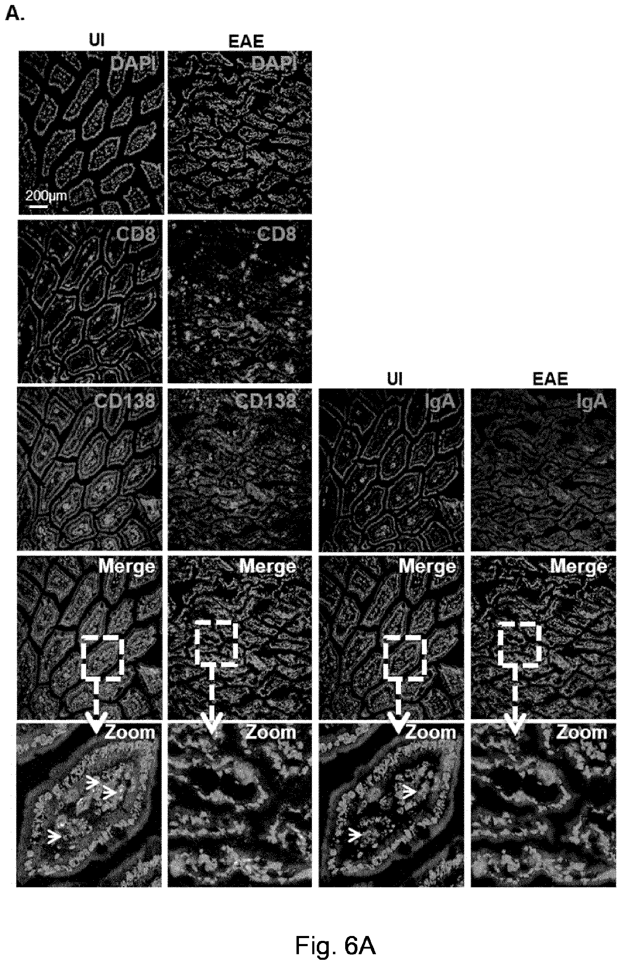

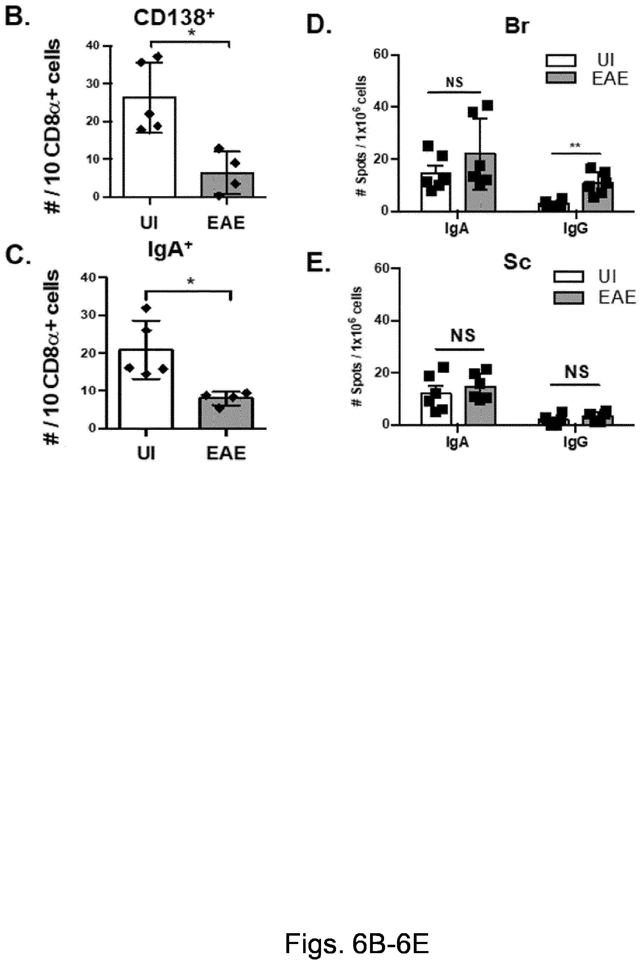

First, it was examined whether gut-resident IgA⁺CD138⁺ cells were modulated during neuroinflammation, and consistent with the observations in WT mice, a dramatic decrease in these cells was observed during the chronic stage of EAE, accompanied by a disruption in DAPI⁺ epithelial cells (Fig. 6a-c). The CNS was also evaluated for the presence of IgA ASC by ELISPOT. Unlike WT mice where IgA and IgG ASC were virtually undetectable in the CNS of unimmunized mice, BAFF-Tg mice exhibited a baseline level of IgA ASC in the Br and SC at steady state that was not further elevated during EAE (Fig. 6d-e). Indeed, the frequency of IgA ASC in the resting Br was nearly the same as what was observed in WT mice during the chronic phase of EAE. In contrast, like WT mice, IgG ASC were only detected in the CNS of BAFF-Tg mice during EAE, but not in the steady state.

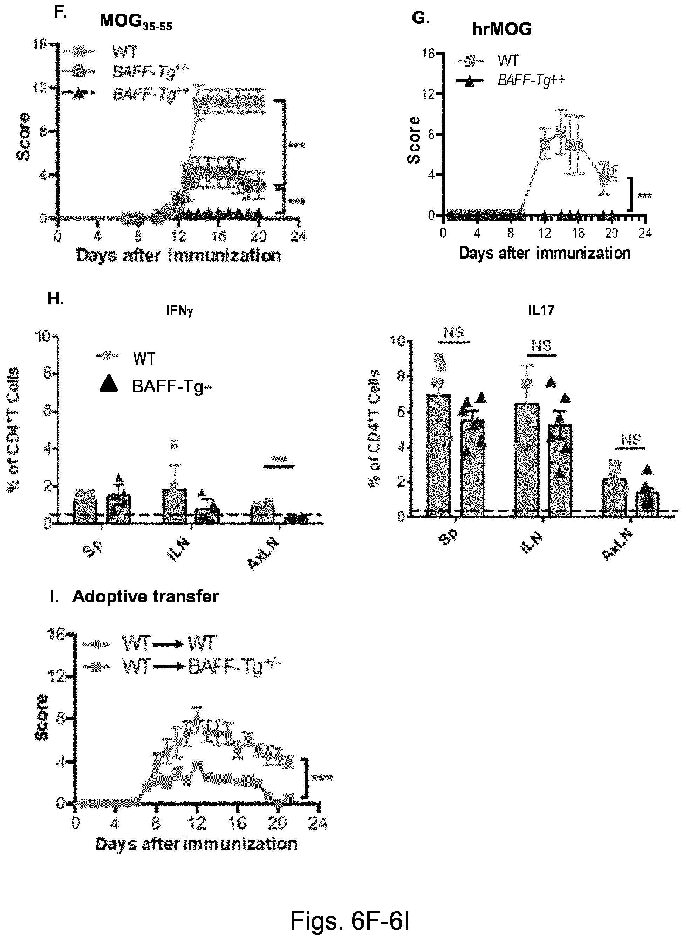

Next, it was determined whether BAFF-Tg^{+/+} mice, which have a massive surplus of **IgA⁺** PB/PC (McCarthy et al., 2011), would be resistant to EAE. Compared to littermate controls, BAFF-Tg^{+/+} mice immunized with MOG₃₅₋₅₅ in fact did not develop clinical symptoms of disease compared to co-caged WT littermates, and this was also observed in mice with only one copy of the BAFF transgene (BAFF-Tg^{+/-}) where only mild disease was observed (Fig. 6f). These results were also confirmed in a different vivarium (Table II). To assess whether BAFF-mediated resistance to MOG₃₅₋₅₅ EAE was generalizable to other EAE models, BAFF-Tg^{+/+} mice were immunized with full-length recombinant human MOG (rhMOG), a protein that induces pathogenic anti-MOG auto-antibodies that promote CNS injury (Bansal et al., 2013; Marta et al., 2005). BAFF-Tg^{+/+} mice were likewise resistant to rhMOG-induced disease (Fig. 6g).

To determine whether over-expression of BAFF and a surplus of IgA⁺ PB/PC in these mice affects the priming versus the effector stage of EAE, the production of IFNγ by CD4⁺ T cells in the draining axillary lymph nodes, inguinal lymph nodes and spleen of MOG₃₅₋₅₅ immunized BAFF-Tg^{+/+} mice was examined during the pre-onset period of the disease (day 7-9). CD4⁺IFNγ⁺, and CD4⁺IL17⁺ T cells were detected above background in all 3 of these organs and at similar levels in both WT and BAFF-Tg^{+/+} mice, with a reduction in CD4⁺IFNγ⁺ T cells only being observed in the axillary lymph nodes of BAFF-Tg^{+/+} mice (Fig. 6h). Similar results were also observed for CD4⁺TNFα⁺ cells where no significant differences in these cells were detected in the spleen or draining LN in WT versus BAFF-Tg^{+/+} mice. Thus, there is no overt defect in priming of T cells in the LN or spleen of BAFF-Tg mice in response to immunization with MOG₃₅₋₅₅ peptide. Similar results were found upon immunization with rhMOG.

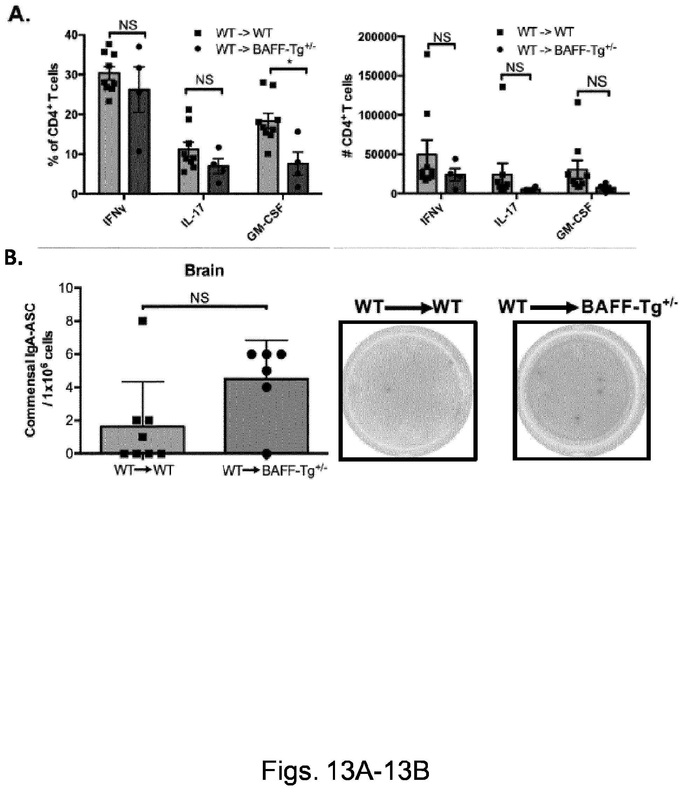

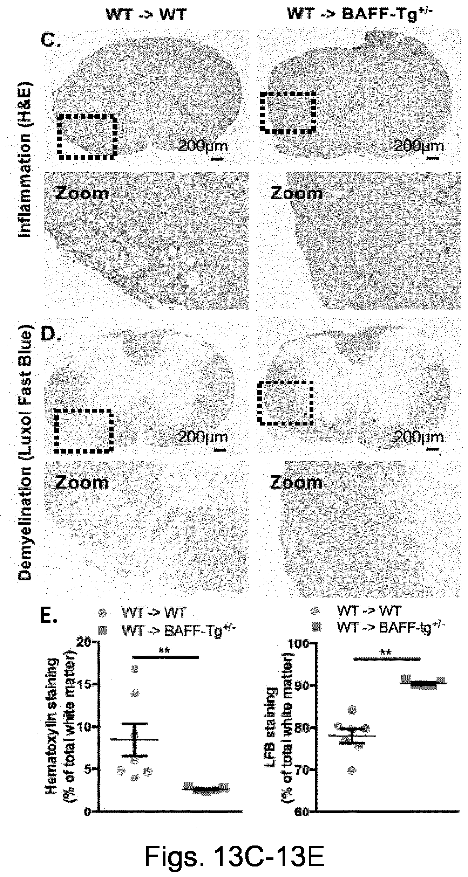

In contrast to the relatively normal priming response to MOG₃₅₋₅₅ peptide, defects were observed in the effector phase of the disease. Specifically, while adoptive transfer of pre-primed T cells from WT donor mice immunized with MOG₃₅₋₅₅ peptide resulted in EAE when T cells were transferred into WT recipients, transfer of the same T cells into BAFF-Tg^{+/-} recipients resulted in significantly attenuated EAE similar to what is observed in actively immunized BAFF-Tg^{+/-} mice (Fig. 6i). This was accompanied by a non-significant trend of reduced frequency and numbers of IFNγ⁺CD4⁺ and IL17⁺CD4⁺T cells, and a significant reduction in the frequency of GM-CSF⁺ CD4⁺ T cells. A trend towards increased frequency of commensal-reactive IgA ASC (measured by ELISPOT) in the CNS was also noted (Fig. 13a-b), as well as a significant reduction in inflammation and demyelination in the spinal cord (Fig. 13c-e).

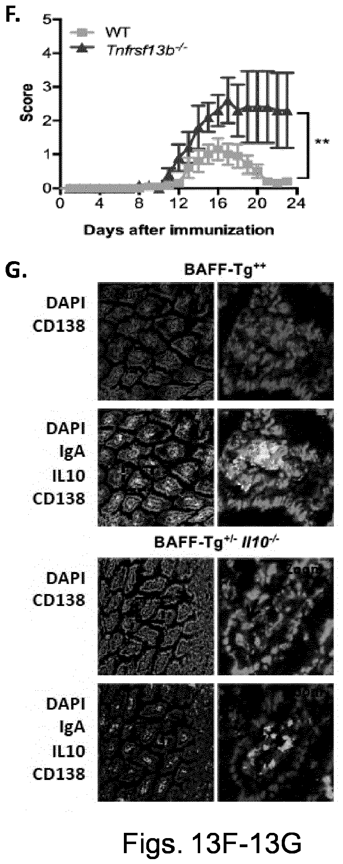

Lastly, it was determined whether TACI (TNFRSF13b), a receptor for BAFF and APRIL that is highly expressed by PB/PC (Pracht et al., 2017) and important for the generation of IgA-producing cells (He et al., 2010), provides protective signals during EAE. To test this, *Tnfrsf13b*^{*-*/*-*}mice were immunized with MOG₃₅₋₅₅peptide and assessed for clinical parameters of EAE. Similar to PB/PC-less mice, *Tnfrsf13b*^{*-*/*-*} mice also show evidence of exacerbated EAE compared to WT controls (Fig. 13f).

In summary, BAFF-Tg mice, which have an overabundance of IgA-producing cells in the gut and harbor IgA-producing cells in the CNS during the steady state, are highly resistant to both MOG₃₅₋₅₅ and rhMOG-induced EAE, possibly due to TACI-derived signals. Moreover, whereas the priming response induced by MOG immunization is relatively normal, the effector phase of EAE disease is attenuated in BAFF-Tg mice compared to WT mice, as evidenced by diminished GM-CSF production in the CNS.

### EXAMPLE 11: Gut-derived IgA PB/PC promote resistance to EAE in BAFF-Tg mice via IL10

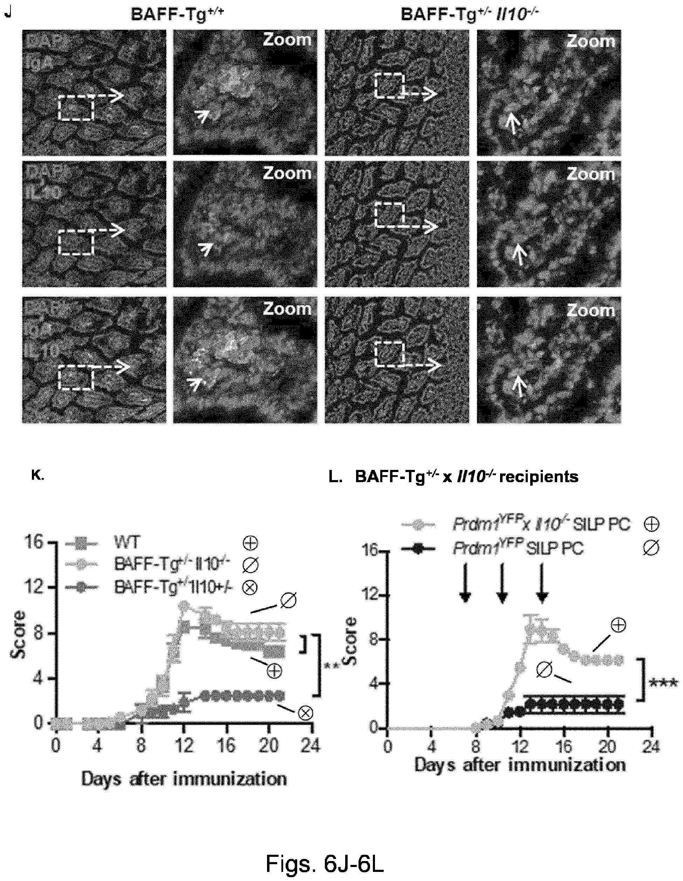

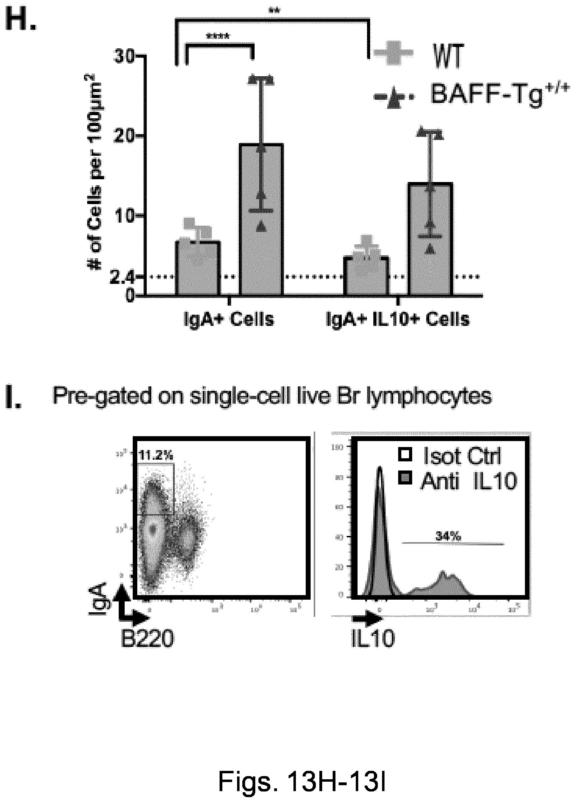

Next, experiments were carried out in order to understand the mechanism of EAE resistance in BAFF-Tg mice. Given that PB/PC are a necessary source of IL10 for the attenuation of EAE and that IgA⁺ cells in the SILP can also express IL10, IL10 may be involved in EAE resistance in BAFF-Tg mice. Indeed, BAFF-Tg^{+/+} mice showed evidence of IL10 expression by SILP-resident IgA⁺ cells (using BAFF-Tg^{+/-} x *Il10*^{-/-} mice as a staining control - Fig. 6j and Fig. 13g), and compared to WT mice, IgA⁺IL10⁺ cells were even more abundant in the BAFF-Tg^{+/+} gut (Fig. 13h). Also like WT mice, there is evidence of IL10 expression by a portion of IgA⁺B220⁺ PB/PC in the brain of BAFF-Tg^{+/+} mice during EAE (Fig. 13i). Therefore, BAFF-Tg mice harbour a large population of IgA+ cells in the gut and in the EAE brain that express IL10.

To test whether IL10 is required for EAE resistance in BAFF-Tg mice, BAFF-Tg^{+/-}IL10^{-/-}, BAFF-Tg^{+/-}IL10^{+/-} and WT littermates were generated. BAFF-Tg^{+/-}IL10^{-/-} mice developed EAE disease similar to what was observed in WT littermate controls (Fig. 6k, Table II), indicating that IL10 is necessary for BAFF-driven resistance to EAE. To ascertain if IgA-producing SILP-derived PB/PC were a relevant source of IL10 in BAFF-Tg mice, *Prdm1*-YFP⁺ PC were transferred from the SILP of *Prdm-1*^{yfp} mice or *Prdm1*-YFP⁺x *Il10*^{*-*/*-*} mice into BAFF-Tg^{+/-}*Il10*^{-/-} recipient mice. It was found that only IL10-sufficient *Prdm1*-YFP⁺ cells from the SILP of *Il10*^{+/+} mice, but not *Il10*^{*-*/*-*} mice could confer disease relief in BAFF-Tg^{+/-}*Il10*^{-/-} recipient mice (Fig. 6l), demonstrating that intestinal PB/PC provide an essential source of IL10 that is sufficient to suppress EAE.

In summary, BAFF-Tg, but not BAFF-Tg^{+/-}*Il10*^{*-*/*-*} mice, are highly resistant to EAE, and BAFF-Tg mice harbour a significant number of **IgA⁺** cells in the SILP that express IL10. Moreover, SILP-derived PB/PC are a sufficient source of IL10 for the attenuation of EAE clinical symptoms in BAFF-Tg^{+/-}*Il10*^{*-*/*-*} mice.

### EXAMPLE 12: BAFF-Fc treatment suppresses EAE

B cells express three different receptors for BAFF: TNFRSF13b (TACI), TNFRSF13c (BAFF-R), and TNFRSF17 (BCMA). BAFF-Fc can bind to these receptors and stimulate their activation.

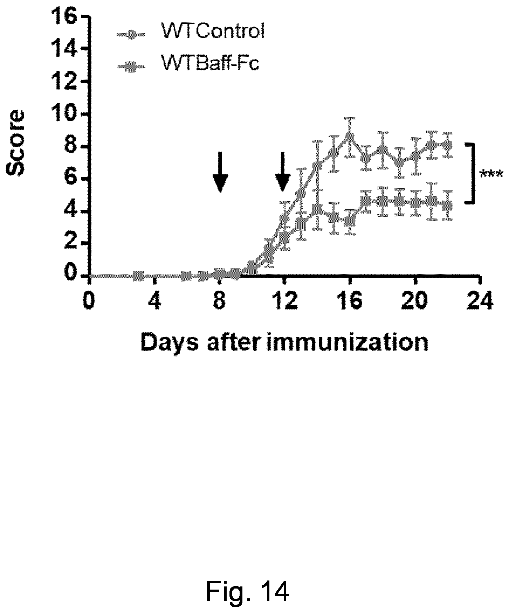

The BAFF-Fc fusion protein was administered by intraperitoneal injection to WT C57BL6 mice at a dosage of 100 µg per mouse on day 8 and day 12 after immunization with MOG₃₅₋₅₅. The mice were monitored for symptoms of EAE using the modified 16-point scale clinical scoring system. The mice treated with BAFF-Fc showed significantly lower EAE clinical scores in comparison with control mice treated with PBS (Fig. 14). In summary, treatment with BAFF-Fc leads to the suppression of EAE.

### EXAMPLE 13: BAFF-Fc improves bone marrow PB/PC survival in vitro.

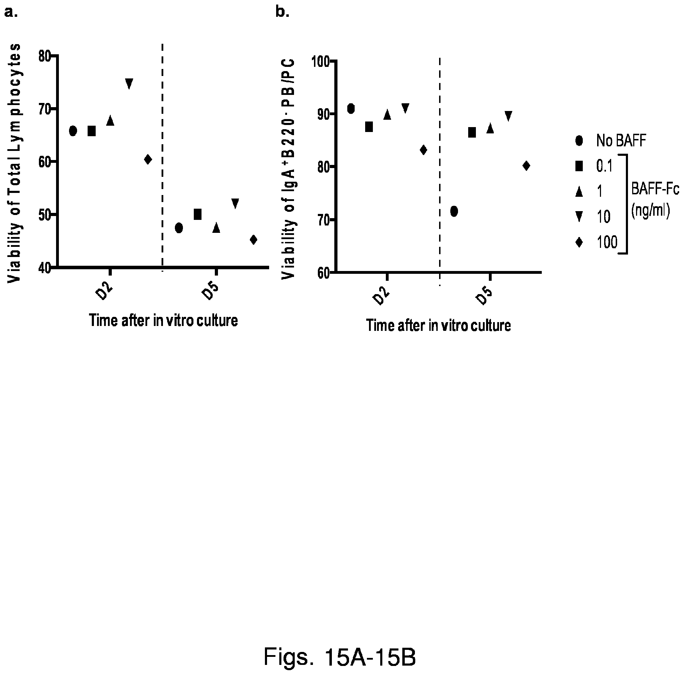

Since it was found that BAFF-Tg mice were resistant to the development of EAE, and EAE mice treated with BAFF-Fc developed milder disease compared to control treated mice, maintaining IgA+ PB/PC survival could be a mechanism of BAFF-Fc treatment. The ability of the BAFF-Fc compound to maintain IgA+B220- PB/PC survival by using an in vitro culture system was evaluated. Total Bone Marrow Lymphocytes from Prdm-1 YFP mice were cultured in vitro with different concentrations of the BAFF-Fc compound (0,1, 1, 10 or 100 ng/ml); the viability of IgA+B220- PB/PC was determined by Flow cytometry (FACS). The viability of total lymphocytes was evaluated by FACS using Aqua viability dye after D2 and D5 of culture, using different concentrations of BAFF-Fc. The viability of IgA+PB/PC specifically, was evaluated by FACS using Aqua viability dye after D2 and D5 of culture, using different concentrations of BAFF-Fc. As little as 1ng/ml of BAFF-Fc improved the survival of IgA+B220- PB/PC (Fig. 15b). There is also a mild effect of BAFF-Fc at improving survival of total lymphocytes (Fig. 15a). Therefore, BAFF-Fc can be used to promote the survival of commensal-reactive IgA ASC in the blood.

### EXAMPLE 14: Commensal-IgA ASC can be detected in different tissues in BAFF-Tg mice under steady state.

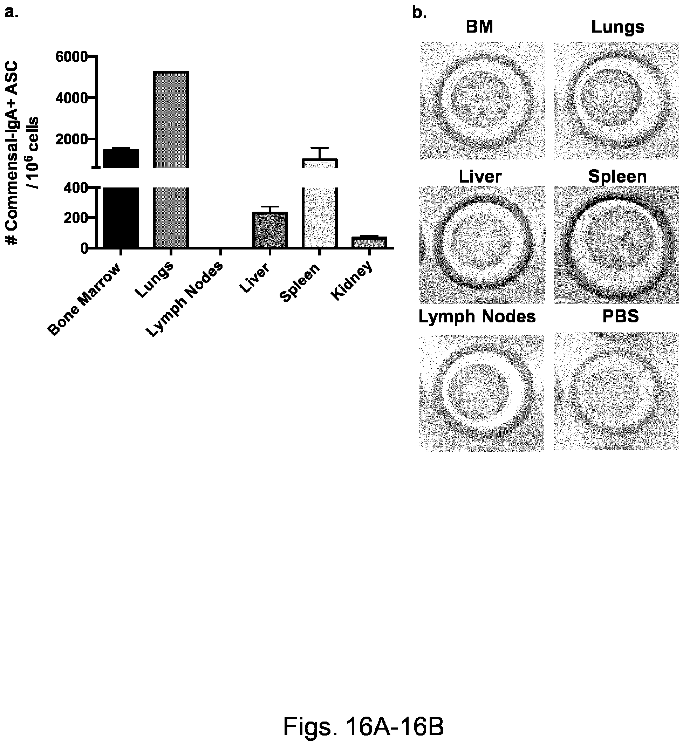

Since commensal-reactive IgA ASC was able to be detected in the brain and bone marrow of naïve BAFF-Tg mice, it was next determined if high levels of BAFF may lead to the accumulation of commensal-reactive IgA ASC in other tissues. Single cell suspensions from the bone marrow, lungs, peripheral lymph nodes, liver, spleen and kidney from BAFF-Tg mice were subjected to the commensal ELSIPOT assay previously described in Example 7. The frequency of commensal-reactive IgA ASC per million cells derived from different tissues in BAFF-Tg mice was determined and representative images of commensal-reactive IgA ASC in different tissues from BAFF-Tg mice were obtained. Commensal-reactive IgA ASC accumulate in different tissues in the presence of excess BAFF, even under naïve conditions (Figs. 16a and b). This indicates that administration of a drug therapy that augments the number of commensal-reactive IgA ASC, such as BAFF-Fc, will result in colonization of many different peripheral tissues with commensal-reactive IgA ASC. Therefore, BAFF-Fc or an alternative therapeutic that boosts the number of commensal-reactive IgA ASC can be used to treat other autoimmune diseases that target tissues other than the inflamed brain.

### EXAMPLE 15: Detection of Commensal-IgA ASC in peripheral blood of healthy volunteers.

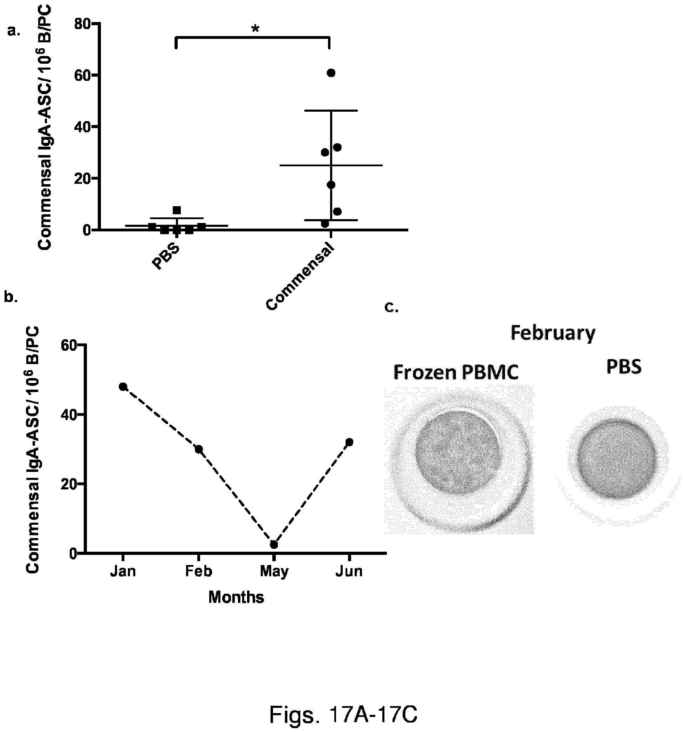

To validate the hypothesis that commensal-reactive IgA ASC access the periphery in human, a Commensal ELISPOT assay using human peripheral blood mononuclear cells (PBMC) was designed. Using this assay, it was then examined whether commensal-reactive IgA ASC could be detected in the blood of healthy human subjects. Lymphocytes were isolated from peripheral blood of healthy volunteers and cryopreserved. Subsequently, after thawing, B cells and PC were enriched using a negative selection kit with magnetic beads. The ELISPOT assay was performed by coating an ELISPOT plate with a defined human microbial Ecosystem (MET-1) or a heat-inactivated human fecal sample. Thawed PBMC were incubated in the plate overnight. A variable number of commensal-reactive IgA ASC were detected in the peripheral blood of healthy volunteers (Fig 17a). In addition, a dynamic number of commensal-reactive IgA ASC were detected in PBMC from the same volunteer over time (Fig. 17b). Representative images of the commensal-lgA ASC obtained by ELISPOT using thawed PBMC from healthy volunteers after a B/PC enrichment with negative selection beads were also obtained (Fig. 17c). A variable number of circulating commensal-lgA ASC were detected in the peripheral blood of healthy volunteers with a dynamic range over time. This indicates that gut-derived commensal-reactive IgA ASC can routinely access the blood of humans, as we observed in mice.

### EXAMPLE 16: BAFF-Fc improves the recovery of commensal-reactive and regulatory IgA+ PB/PC derived from healthy volunteer PBMC.

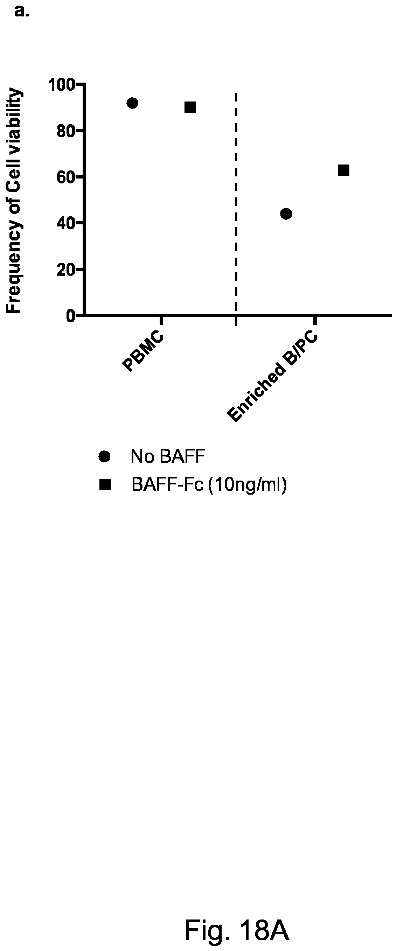

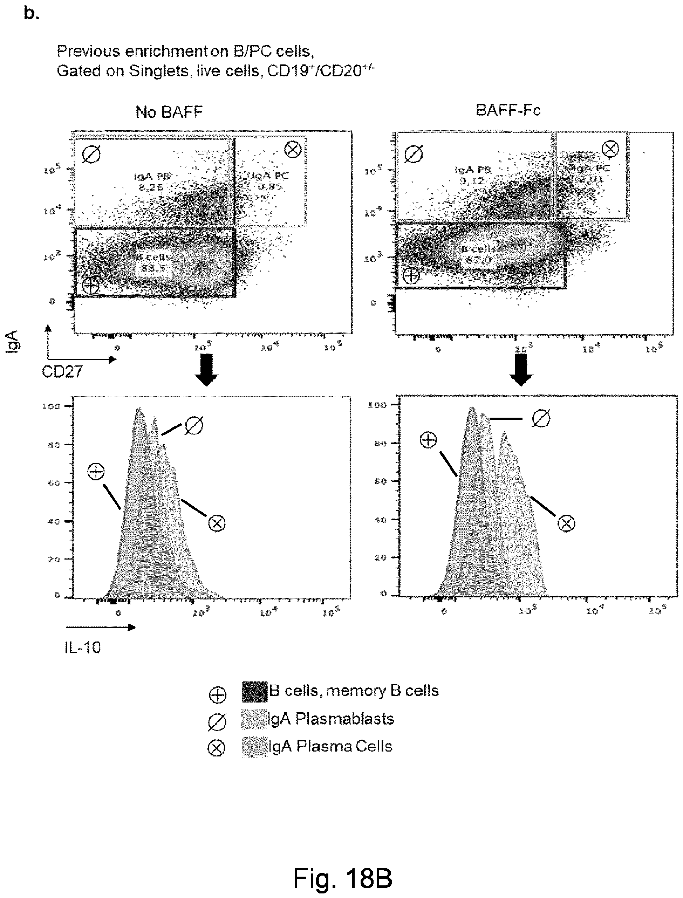

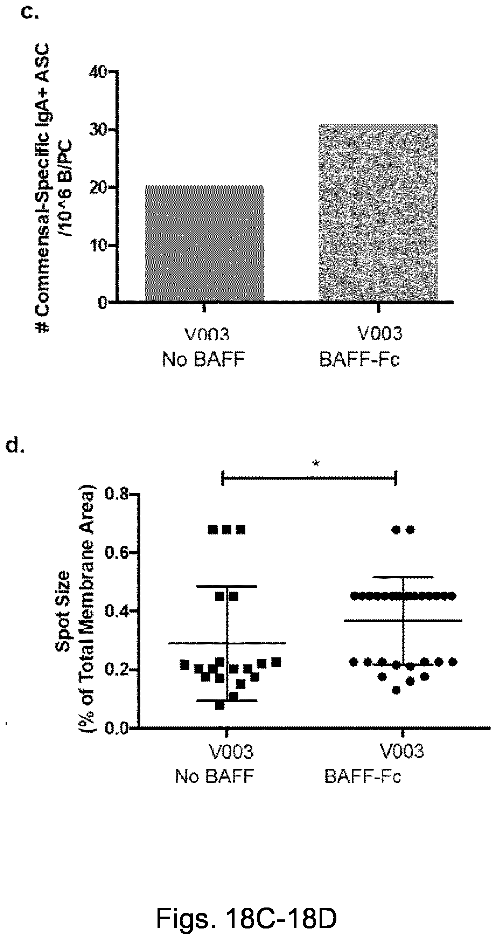

To determine if BAFF-Fc may be improving the survival of human commensal-reactive IgA ASC and more specifically, immunoregulatory IgA+ PB/PC (iPC), the ability of the addition of BAFF-Fc to enhance the survival of these cells in vitro was determined. PBMC were isolated from healthy volunteers. B/PC were enriched using a negative selection Magnetic Beads kit. Total PBMC as well as B/PB enriched cells were incubated with BAFF-Fc (10ng/ml) for 3 days. Flow cytometry was subsequently performed to determine viability and IL10 production derived from IgA+ PB/PC. In some cases, the enriched B/PC were subjected to the commensal ELISPOT in the absence or presence of BAFF-Fc. The frequency of cell viability in total PBMC or in B/PC ONLY was evaluated by FACS using Aqua viability marker after D3 of culture, and BAFF-FC enhanced the frequency of cell viability in B/PC (Fig. 18a). Representative FACS dot plot images showed an enrichment of IL-10 producing IgA PB/PC after in vitro culture with BAFF-Fc (Fig. 18b). The addition of BAFF-Fc during the Elispot assay increased the number of commensal-reactive IgA ASC (Fig. 18c). BAFF-Fc also increased the size of commensal-reactive IgA ASC spots in the ELISPOT assay, suggesting an indirect increase in ASC survival and IgA production. In conclusion, BAFF-Fc enhances the survival of IL10-producing IgA PB/PC and more specifically, the survival and IgA production derived from commensal-reactive IgA ASC in vitro.

## Claims

1. A composition comprising a B-cell Activating Factor (BAFF) polypeptide for use in treatment of multiple sclerosis in a subject.

2. The composition for use of claim 1, wherein the composition reduces inflammation caused by multiple sclerosis in a subject.

3. The composition for use of claim 2, wherein the inflammation is reduced in the central nervous system of the subject.

4. The composition for use of any one of claims 2-3, wherein the inflammation is neuroinflammation.

5. The composition for use of any one of claims 1-4, wherein the composition further comprises an agent that promotes survival and/or migration of gut-derived commensal-reactive B cells to the central nervous system of a subject, wherein the agent that promotes survival and/or migration of gut-derived commensal-reactive B cells:
- is a cytokine or a chemokine and/or
- is IL-10 and/or iNOS.

6. The composition for use of claim 5, wherein:
- the gut-derived commensal-reactive B cells are IgA+ plasmablasts and/or plasma cells; and/or
- the gut-derived commensal-reactive B cells express IL-10 and/or iNOS.

7. The composition for use of any one of claims 5-6, wherein the composition enriches gut-derived commensal-reactive IgA+ plasmablasts and/or plasma cells in the central nervous system of a subject that has multiple sclerosis.

8. The composition for use of any one of claims 5-7, wherein the composition promotes survival of gut-derived commensal-reactive IgA+ plasmablasts and/or plasma cells in a subject that has multiple sclerosis so as to reduce inflammation in a tissue in the subject.

9. The composition for use of claim 7 or 8, wherein the gut-derived commensal-reactive IgA+ plasmablasts and/or plasma cells express IL-10 and/or iNOS.

10. The composition for use of any one of claims 1-4, wherein the composition further comprises a gut commensal that increases IgA levels,, wherein the gut commensal is a commensal microbe, preferably wherein the gut commensal comprises *Tritrichomonas musculis* or a gut microbial community that has been modified by carriage of *Tritrichomonas musculis.*

11. The composition for use of claim 10, wherein the gut commensal comprises an effective amount of a microbe or community of microbes for oral or rectal administration.

12. The composition for use of any one of claims 1-11, wherein the multiple sclerosis is relapsing-remitting multiple sclerosis, primary progressive multiple sclerosis or secondary progressive multiple sclerosis.

## Patentansprüche

1. Zusammensetzung, umfassend ein BAFF(B-cell Activating Factor)-Polypeptid zur Verwendung bei der Behandlung von multipler Sklerose bei einem Individuum.

2. Zusammensetzung zur Verwendung nach Anspruch 1, wobei die Zusammensetzung durch multiple Sklerose verursachte Entzündung bei einem Individuum verringert.

3. Zusammensetzung zur Verwendung nach Anspruch 2, wobei die Entzündung im Zentralnervensystem des Individuums verringert wird.

4. Zusammensetzung zur Verwendung nach einem der Ansprüche 2-3, wobei es sich bei der Entzündung um Neuroinflammation handelt.

5. Zusammensetzung zur Verwendung nach einem der Ansprüche 1-4, wobei die Zusammensetzung ferner ein Mittel umfasst, das Überleben mit aus dem Darm stammenden Kommensalen reagierender B-Zellen und/oder deren Migration zum Zentralnervensystem eines Individuums fördert, wobei es sich bei dem Mittel, das Überleben und/oder Migration mit aus dem Darm stammenden Kommensalen reagierender B-Zellen fördert, um:
- ein Cytokin oder ein Chemokin und/oder
- IL-10 und/oder iNOS handelt.

6. Zusammensetzung zur Verwendung nach Anspruch 5, wobei:
- es sich bei den mit aus dem Darm stammenden Kommensalen reagierenden B-Zellen um IgA+-Plasmablasten und/oder -Plasmazellen handelt; und/oder
- die mit aus dem Darm stammenden Kommensalen reagierenden B-Zellen IL-10 und/oder iNOS exprimieren.

7. Zusammensetzung zur Verwendung nach einem der Ansprüche 5-6, wobei die Zusammensetzung mit aus dem Darm stammenden Kommensalen reagierende IgA+-Plasmablasten und/oder -Plasmazellen im Zentralnervensystem eines Individuums, das an multipler Sklerose leidet, anreichert.

8. Zusammensetzung zur Verwendung nach einem der Ansprüche 5-7, wobei die Zusammensetzung das Überleben von mit aus dem Darm stammenden Kommensalen reagierenden IgA+-Plasmablasten und/oder -Plasmazellen in einem Individuum, das an multipler Sklerose leidet, fördert, so dass Entzündung in einem Gewebe im Individuum verringert wird.

9. Zusammensetzung zur Verwendung nach Anspruch 7 oder 8, wobei die mit aus dem Darm stammenden Kommensalen reagierenden IgA+-Plasmablasten und/oder -Plasmazellen IL-10 und/oder iNOS exprimieren.

10. Zusammensetzung zur Verwendung nach einem der Ansprüche 1-4, wobei die Zusammensetzung ferner einen Darmkommensalen umfasst, durch den IgA-Spiegel erhöht werden, wobei es sich bei dem Darmkommensalen um eine kommensale Mikrobe handelt, vorzugsweise wobei der Darmkommensale *Tritrichomonas musculis* oder eine Darmmikrobengemeinschaft, die durch Mitnahme von *Tritrichomonas musculis* modifiziert wurde, umfasst.

11. Zusammensetzung zur Verwendung nach Anspruch 10, wobei der Darmkommensale eine wirksame Menge einer Mikrobe oder Gemeinschaft von Mikroben zur oralen oder rektalen Verabreichung umfasst.

12. Zusammensetzung zur Verwendung nach einem der Ansprüche 1-11, wobei es sich bei der multiplen Sklerose um schubförmig remittierende multiple Sklerose, primär progrediente multiple Sklerose oder sekundär progrediente multiple Sklerose handelt.

## Revendications

1. Composition comprenant un polypeptide facteur d'activation des lymphocytes B (BAFF) pour une utilisation dans le traitement de la sclérose en plaques chez un sujet.

2. Composition pour une utilisation selon la revendication 1, la composition réduisant l'inflammation causée par la sclérose en plaques chez un sujet.

3. Composition pour une utilisation selon la revendication 2, dans laquelle l'inflammation est réduite dans le système nerveux central du sujet.

4. Composition pour une utilisation selon l'une quelconque des revendications 2-3, dans laquelle l'inflammation est une neuroinflammation.

5. Composition pour une utilisation selon l'une quelconque des revendications 1-4, la composition comprenant en outre un agent qui favorise la survie et/ou la migration de lymphocytes B réactifs de manière commensale dérivés de l'intestin vers le système nerveux central d'un sujet, dans laquelle l'agent qui favorise la survie et/ou la migration de lymphocytes B réactifs de manière commensale dérivés de l'intestin :
- est une cytokine ou une chimiokine et/ou
- est IL-10 et/ou iNOS.

6. Composition pour une utilisation selon la revendication 5, dans laquelle :
- les lymphocytes B réactifs de manière commensale dérivés de l'intestin sont des plasmablastes IgA+ et/ou des cellules plasmatiques ; et/ou
- les lymphocytes B réactifs de manière commensale dérivés de l'intestin expriment IL-10 et/ou iNOS.

7. Composition pour une utilisation selon l'une quelconque des revendications 5-6, la composition enrichissant des plasmablastes IgA+ et/ou des cellules plasmatiques réactifs de manière commensale dérivés de l'intestin dans le système nerveux central d'un sujet atteint de sclérose en plaques.

8. Composition pour une utilisation selon l'une quelconque des revendications 5-7, la composition favorisant la survie de plasmablastes IgA+ et/ou de cellules plasmatiques réactifs de manière commensale dérivés de l'intestin chez un sujet atteint de sclérose en plaques afin de réduire l'inflammation dans un tissu du sujet.

9. Composition pour une utilisation selon la revendication 7 ou 8, dans laquelle les plasmablastes IgA+ et/ou les cellules plasmatiques réactifs de manière commensale dérivés de l'intestin expriment IL-10 et/ou iNOS.

10. Composition pour une utilisation selon l'une quelconque des revendications 1-4, la composition comprenant en outre un commensal intestinal qui augmente les niveaux d'IgA, dans laquelle le commensal intestinal est un microbe commensal, de préférence dans laquelle le commensal intestinal comprend *Tritrichomonas musculis* ou une communauté microbienne intestinale qui a été modifiée par le transport de *Tritrichomonas musculis.*

11. Composition pour une utilisation selon la revendication 10, dans laquelle le commensal intestinal comprend une quantité efficace d'un microbe ou d'une communauté de microbes pour une administration orale ou rectale.

12. Composition pour une utilisation selon l'une quelconque des revendications 1-11, dans laquelle la sclérose en plaques est une sclérose en plaques récidivante-rémittente, une sclérose en plaques progressive primaire ou une sclérose en plaques progressive secondaire.
